# EUROPEAN PATENT SPECIFICATION

(11) **EP 1 078 060 B1**
(45) Date of publication and mention of the grant of the patent: **10.01.2007**
(21) Application number: 99923574.0
(22) Date of filing: 11.05.1999
(51) Int. Cl.: C12N 15/13, C07K 16/18, C07K 16/46, C12N 5/20, C07K 14/705, C12N 15/86, C12N 5/10, C07K 19/00, C12N 15/62, C12N 5/06, G01N 33/53, A61K 39/00, A61K 39/395, A61K 31/70, A61K 35/14, A01K 67/027, G01N 33/577, G01N 33/68, C12Q 1/68, A61K 48/00

(54) **ANTIBODIES TO DENDRITIC CELLS AND HUMAN DENDRITIC CELL POPULATIONS AND USES THEREOF**
ANTIKÖRPER GEGEN DENDRITISCHE ZELLEN UND MENSCHLICHE DENDRITISCHE ZELLPOPULATIONEN UND DEREN VERWENDUNGEN
ANTICORPS DES CELLULES DENDRITIQUES ET POPULATIONS DE CELLULES DENDRITIQUES HUMAINES ET UTILISATION DE CEUX-CI

(30) Priority: 11.05.1998 EP 98108534
(43) Date of publication of application: 28.02.2001
(73) Proprietor: Rieber, E.P., Prof. Dr., 01309 Dresden (DE)
(72) Inventor: Rieber, E.P., Prof. Dr., 01309 Dresden (DE)
(74) Representative: Vossius & Partner
(86) International application number: PCT/EP1999/003218
(87) International publication number: WO 1999/058678

(56) References cited:
- EP-A- 0 404 097
- WO-A-93/04187
- WO-A-95/12409
- WO-A-97/04802
- WO-A-98/15579
- SNAPP, KAREN R. ET AL: "A novel P- selectin glycoprotein ligand-1 monoclonal antibody recognizes an epitope within the tyrosine sulfate motif of human PSGL-1 and blocks recognition of both P- and L- selectin" BLOOD (1998), 91(1), 154-164 , XP002123922
- HSU F J ET AL: "Antigen-pulsed dendritic cells are effective in inducing immune responses in patients with B-cell lymphoma (Meeting abstract)." BLOOD, (1994). VOL. 84, NO. 10, SUPPL. 1, PP. 520A. ISSN: 0903-1936., XP000857234 Div. Of Oncology, Stanford Univ. Medical Center, Stanford, CA.
- SCHAKEL K ET AL: "A novel dendritic cell population in human blood: one-step immunomagnetic isolation by a specific mAb ( M - DC8 ) and in vitro priming of cytotoxic T lymphocytes." EUROPEAN JOURNAL OF IMMUNOLOGY, (1998 DEC) 28 (12) 4084-93. , XP000857207
- TUTING, T T.AL.: "Autologous human monocyte-derived dendritic cells genetically modified to express melanoma antigens elicit primary cytotoxic T cell responses in vitro: enhancement by cotransfection of genes encoding the Th1-biasing cytokines IL-12 and IFN-alpha." JOURNAL OF IMMUNOLOGY, vol. 160, no. 3, 1 February 1998 (1998-02-01), pages 1139-1147, BALTIMORE, US
- O'DOHERTY U; ET AL: "HUMAN BLOOD CONTAINS TWO SUBSETS OF DENDRITIC CELLS, ONE IMMUNOLOGICALLY MATURE AND THE OTHER IMMATURE" IMMUNOLOGY, vol. 82, 1994, pages 487-493, XP000610064 OXFORD, GB
- THOMAS R; LIPSKY P E: "HUMAN PERIPHERAL BLOOD DENDRITIC CELL SUBSETS ISOLATION AND CHARACTERIZATION OF PRECURSOR AND MATURE ANTIGEN-PRESENTING CELLS" JOURNAL OF IMMUNOLOGY, vol. 153, no. 9, 1 November 1994 (1994-11-01), pages 4016-4028, XP000608282 BALTIMORE, US

### SEQUENCE LISTING

(1) GENERAL INFORMATION:
   (i) APPLICANT:
      (A) NAME: Micromet GmbH
      (B) STREET: Am Klopferspitz 19
      (C) CITY: Martinsried
      (D) STATE: none
      (E) COUNTRY: Germany
      (F) POSTAL CODE (ZIP): 82152
   (ii) TITLE OF INVENTION: Novel antibodies and human dendritic cell population and uses thereof
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
(2) INFORMATION FOR SEQ ID NO: 1:
   (i) SEQUENCE CHARACTERISTICS:
      (A) LENGTH: 336 base pairs
      (B) TYPE: nucleic acid
      (C) STRANDEDNESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:1..336
   (xi) SEQUENCE DESCRIPTION: SEQ ID NO: 1:
   CAG GTC CAA CTG CAG CAG TCA GGG GCT GAG CTT GTG AAG CCT GGG GCT       48
   Gln Val Gln Leu Gln Gln Ser Gly Ala Glu Leu Val Lys Pro Gly Ala
    1               5                  10                  15
   TCA GTG AAG CTG TCC TGC AAG GCT TCT GGC TAC ACC CTC ACC AGC TAC       96
   Ser Val Lys Leu Ser Cys Lys Ala Ser Gly Tyr Thr Leu Thr Ser Tyr
               20                  25                  30
   TGG TTG CAC TGG GTG AAG CAG TGG CCT GGA CGA GGC CTT GAG TGG ATT      144
   Trp Leu His Trp Val Lys Gln Trp Pro Gly Arg Gly Leu Glu Trp Ile
           35                  40                  45
   GGA AGG ATT GAT CCC AAT AGT GGT GGT ACT AAG TAC GAT GAG AAG TTC      192
   Gly Arg Ile Asp Pro Asn Ser Gly Gly Thr Lys Tyr Asp Glu Lys Phe
       50                  55                  60
   AAG AGC AAG GCC ACA CTG ACT GTA GAC AAA CCC TCC AGC ACA GCC TAC      240
   Lys Ser Lys Ala Thr Leu Thr Val Asp Lys Pro Ser Ser Thr Ala Tyr
   65                  70                  75                      80
   ATG CAG CTC AGC AGC CTG ACA TCT GAG GAC TCT GCG GTC TAT TAT TGT      288
   Met Gln Leu Ser Ser Leu Thr Ser Glu Asp Ser Ala Val Tyr Tyr Cys
                   85                  90                  95
   GCA AGA TGG GAC TAC TGG GGC CAA GGG ACC ACG GTC ACC GTC TCC TCA      336
   Ala Arg Trp Asp Tyr Trp Gly Gln Gly Thr Thr Val Thr Val Ser Ser
               100                 105                 110
(2) INFORMATION FOR SEQ ID NO: 2:
   (i) SEQUENCE CHARACTERISTICS:
      (A) LENGTH: 112 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Gln Val Gln Leu Gln Gln Ser Gly Ala Glu Leu Val Lys Pro Gly Ala
    1               5                  10                  15
   Ser Val Lys Leu Ser Cys Lys Ala Ser Gly Tyr Thr Leu Thr Ser Tyr
               20                  25                  30
   Trp Leu His Trp Val Lys Gln Trp Pro Gly Arg Gly Leu Glu Trp Ile
           35                  40                  45
   Gly Arg Ile Asp Pro Asn Ser Gly Gly Thr Lys Tyr Asp Glu Lys Phe
       50                  55                  60
   Lys Ser Lys Ala Thr Leu Thr Val Asp Lys Pro Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Trp Asp Tyr Trp Gly Gln Gly Thr Thr Val Thr Val Ser Ser
               100                 105                 110
(2) INFORMATION FOR SEQ ID NO: 3:
   (i) SEQUENCE CHARACTERISTICS:
      (A) LENGTH: 324 base pairs
      (B) TYPE: nucleic acid
      (C) STRANDEONESS: double
      (D) TOPOLOGY: linear
   (ii) MOLECULE TYPE: cDNA
   (iii) HYPOTHETICAL: NO
   (iv) ANTI-SENSE: NO
   (ix) FEATURE:
      (A) NAME/KEY: CDS
      (B) LOCATION:1..324
   (xi) SEQUENCE DESCRIPTION: SEQ ID NO: 3:
   GAC ATT CAG CTG ACC CAG TCT CCA GCA ATC ATG TCT GCA TCT CCA GGG 48
   Asp Ile Gln Leu Thr Gln Ser Pro Ala Ile Met Ser Ala Ser Pro Gly
   115 120 125
   GAA AAG GTC ACC ATG ACC TGC AGG GCC AGC TCA AGT GTT AGT TCC AGT 96
   Glu Lys Val Thr Met Thr Cys Arg Ala Ser Ser Ser Val Ser Ser Ser
   130 135 140
   TAC TTG CAC TGG TAC CAG CAG AAG TCA GGT GCC TCC CCC AAA CTC TGG 144
   Tyr Leu His Trp Tyr Gln Gln Lys Ser Gly Ala Ser Pro Lys Leu Trp
   145 150 155 160
   ATT TAT AGC ACA TCC AAC TTG GCT TCT GGA GTC CCT GCT CGC TTC AGT 192
   Ile Tyr Ser Thr Ser Asn Leu Ala Ser Gly Val Pro Ala Arg Phe Ser
   165 170 175
   GGC AGT GGG TCT GGG ACC TCT TAC TCT CTC ACA ATC AGC AGT GTG GAG 240
   Gly Ser Gly Ser Gly Thr Ser Tyr Ser Leu Thr Ile Ser Ser Val Glu
   180 185 190
   GCT GAA GAT GCT GCC ACT TAT TAC TGC CAG CAG TAC AGT GGT TAC CCG 288
   Ala Glu Asp Ala Ala Thr Tyr Tyr Cys Gln Gln Tyr Ser Gly Tyr Pro
   195 200 205
   TAC ACG TTC GGA GGG GGG ACC AAG CTG GAG ATC AAA 324
   Tyr Thr Phe Gly Gly Gly Thr Lys Leu Glu Ile Lys
   210 215 220
(2) INFORMATION FOR SEQ ID NO: 4:
   (i) SEQUENCE CHARACTERISTICS:
      (A) LENGTH: 108 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Asp Ile Gln Leu Thr Gln Ser Pro Ala Ile Met Ser Ala Ser Pro Gly
   1 5 10 15
   Glu Lys Val Thr Met Thr Cys Arg Ala Ser Ser Ser Val Ser Ser Ser
   20 25 30
   Tyr Leu His Trp Tyr Gln Gln Lys Ser Gly Ala Ser Pro Lys Leu Trp
           35                  40                  45
   Ile Tyr Ser Thr Ser Asn Leu Ala Ser Gly Val Pro Ala Arg Phe Ser
       50                  55                  60
   Gly Ser Gly Ser Gly Thr Ser Tyr Ser Leu Thr Ile Ser Ser Val Glu
   65                  70                  75                  80
   Ala Glu Asp Ala Ala Thr Tyr Tyr Cys Gln Gln Tyr Ser Gly Tyr Pro
                   85                  90                  95
   Tyr Thr Phe Gly Gly Gly Thr Lys Leu Glu Ile Lys
               100                 105

### SEQUENCE LISTING as ANNEX

<110> MICROMET GMBH
<120> Novel antibodies and human dendritic cell population and uses thereof
<130> B 3357 PCT
<140>
   <141>
<160> 4
<170> PatentIn Ver. 2.1
<210> 1
   <211> 336
   <212> DNA
   <213> Homo sapiens
<400> 1
   caggtccaac tgcagcagtc aggggctgag cttgtgaagc ctggggcttc agtgaagctg 60
   tcctgcaagg cttctggcta caccctcacc agctactggt tgcactgggt gaagcagtgg 120
   cctggacgag gccttgagtg gattggaagg attgatccca atagtggtgg tactaagtac 180
   gatgagaagt tcaagagcaa ggccacactg actgtagaca aaccctccag cacagcctac 240
   atgcagctca gcagcctgac atctgaggac tctgcggtct attattgtgc aagatgggac 300
   tactggggcc aagggaccac ggtcaccgtc tcctca                           336
<210> 2
   <211> 112
   <212> PRT
   <213> Homo sapiens
<400> 2
   Gln Val Gln Leu Gln Gln Ser Gly Ala Glu Leu Val Lys Pro Gly Ala
   1               5                   10                  15
   Ser Val Lys Leu Ser Cys Lys Ala Ser Gly Tyr Thr Leu Thr Ser Tyr
               20                  25                  30
   Trp Leu His Trp Val Lys Gln Trp Pro Gly Arg Gly Leu Glu Trp Ile
           35                  40                  45
   Gly Arg Ile Asp Pro Asn Ser Gly Gly Thr Lys Tyr Asp Glu Lys Phe
       50                  55                  60
   Lys Ser Lys Ala Thr Leu Thr Val Asp Lys Pro Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Trp Asp Tyr Trp Gly Gln Gly Thr Thr Val Thr Val Ser Ser
               100                 105                 110
<210> 3
   <211> 324
   <212> DNA
   <213> Homo sapiens
<400> 3
   gacattcagc tgacccagtc tccagcaatc atgtctgcat ctccagggga aaaggtcacc 60
   atgacctgca gggccagctc aagtgttagt tccagttact tgcactggta ccagcagaag 120
   tcaggtgcct cccccaaact ctggatttat agcacatcca acttggcttc tggagtccct 180
   gctcgcttca gtggcagtgg gtctgggacc tcttactctc tcacaatcag cagtgtggag 240
   gctgaagatg ctgccactta ttactgccag cagtacagtg gttacccgta cacgttcgga 300
   ggggggacca agctggagat caaa 324
<210> 4
   <211> 108
   <212> PRT
   <213> Homo sapiens
<400> 4
   Asp Ile Gln Leu Thr Gln Ser Pro Ala Ile Met Ser Ala Ser Pro Gly
   1 5 10 15
   Glu Lys Val Thr Met Thr Cys Arg Ala Ser Ser Ser Val Ser Ser Ser
   20 25 30
   Tyr Leu His Trp Tyr Gln Gln Lys Ser Gly Ala Ser Pro Lys Leu Trp
   35 40 45
   Ile Tyr Ser Thr Ser Asn Leu Ala Ser Gly Val Pro Ala Arg Phe Ser
   50 55 60
   Gly Ser Gly Ser Gly Thr Ser Tyr Ser Leu Thr Ile Ser Ser Val Glu
   65 70 75 80
   Ala Glu Asp Ala Ala Thr Tyr Tyr Cys Gln Gln Tyr Ser Gly Tyr Pro
   85 90 95
   Tyr Thr Phe Gly Gly Gly Thr Lys Leu Glu Ile Lys
   100 105

## Description

The present invention relates to novel antibodies specifically as deposited under DSM ACC 2241, DSM ACC 2399 or DSM ACC 2398 recognizing a distinct population of human dendritic cells (DCs) and methods of isolating said DCs using said antibodies. The present invention further relates to antigens and epitopes recognized by the above-described antibodies as well as to polynucleotides encoding said antibodies. Furthermore, the present invention relates to vectors comprising said polynucleotides as well as to host cells transformed therewith and their use in the production of said antibodies. The present invention additionally relates to polypeptides comprising a domain of the binding site of the aforementioned antibodies, or an antigen or epitope described above and at least one further, preferably functional domain and to polynucleotides encoding such polypeptides. Furthermore, the present invention relates to vectors comprising said polynucleotides, host cells transfected with said polynucleotides or vectors and their use for the preparation of the above-described polypeptides. The present invention also involves a method for isolating or identifying DCs as defined above and relates to DCs obtainable by said method and/or characterized by recognition of the above-described antibody, and/or containing the aforementioned antigen or epitope. The present invention also involves a method for preparing or identifying T cells in a certain status as well as methods for identifying compounds which interfere with T cell mediated activation of immune responses. In addition the present invention relates to kits and compositions, preferably pharmaceutical and diagnostic compositions, comprising any of the aforedescribed antibodies, antigens, epitopes, polypeptides, polynucleotides, vectors, dendritc cells or T cells or compounds obtainable by the aforementioned method. A further object of the present invention are vaccines comprising antigens exposed to dendritic cells, antigen expressing DCs or comprising an antigen or epitope mentioned before. Furthermore, the above-described dendritic cells are subject for immunopotentiating compositions.

Moreover, the present invention relates to the use of T cells obtainable by the above-described method, the aforementioned DCs, antibodies, polynucleotides and vectors for the preparation of pharmaceutical compositions for adoptive immunotherapy, preferably against cancer and infectious diseases and and their use for the preparation of vaccines and immunotherapeutics or for the identification of new antigenic targets for immunotherapy.

Several documents are cited throughout the text of this specification. Each of the documents cited herein (including any manufacturer's specifications, instructions, etc.) are hereby incorporated by reference; however, there is no admission that any document cited is indeed prior art of the present invention.

Immune responses to foreign antigens are mediated by three different interacting cell types, so called T cells, B cells and antigen-presenting cells (APC). Thymus-derived lymphocytes (T cells) can be divided into two functional and phenotypic distinct subsets. Helper T cells respond to antigen stimulation by producing and secreting lymphokines which activate various other cell types in the immune system. Cytotoxic T lymphocytes (CTL) are specialized for directly killing antigen-positive target cells, e. g. virus-infected cells. B cells recognize antigens by antibodies, either acting as cell surface receptors or as secreted proteins, which bind directly to antigens on a solid surface or in solution. By way of contrast T cells only recognize antigens that have been processed or degraded into small fragments and presented on a solid phase such as the surface of APC. Additionally, antigenic fragments must be presented to T cells in association with major histocompatibility complex (MHC)-encoded class I or class II molecules. CD4+ T cells recognize antigens presented by MHC class II products, while CD8+ T cells recognize antigens in the context of MHC class I proteins. The presentation of antigens to T cells is carried out by specialized cell populations referred to as APC. Being involved in early events of an immune response APC are critical to the initiation of both T and B cell responses. Typically, APC include macrophages/monocytes, B cells, and bone marrow-derived dendritic cells (DC). The most important APC are dendritic cells (Sprent et al., 1987, Adv Immunol 41: 39-133). These cells are specialized for internalizing of exogenous antigens, for the processing of antigens into small peptide fragments, and for the presentation of these fragments at the cell surface, in association with MHC molecules so that an appropiate T cell can recognize the peptide-MHC complex and be activated (Goldberg and Rock, 1992, Nature 357: 375-379). Since APC express both MHC-coded class I and class II proteins, they can present antigenic fragments to both CD4+ and CD8+ T cells for the initiation of an immune response. Besides presentation of antigens to T cells with antigen-specific receptors, APC provide all additional signals for T cell activation. Such signals involve a variety of cell surface adhesion and costimulatory molecules as well as cytokines or growth .. factors. Factors necessary for the activation of naive or unprimed T cells may be different from those required for the re-activation of previously primed memory T cells. In contrast to DC, the antigen presenting capacity of monocytes and B cells seem to be limited to the re-activation of previously sensitized T cells because they are not capable of directly activating functionally naive or unprimed T cells. The term "dendritic cells" refers to a diverse group of morphologically similar cells present in various lymphoid and non-lymphoid tissues (Steinmann, 1991, Ann Rev immunol 9: 271-296). These cells include DC of lymphoid organs such as lymph nodes and spleen, Langerhans cells of the epidermis, veiled cells in the afferent lymphatic vessels and DC in the blood circulation. Phenotypically human DC are characterized by a high density of MHC class II antigens, the presence of a wide range of adhesion molecules and the absence or low expression of a range of lineage specific cell surface antigens characteristic for T, B, monocyte and natural killer cells (CD3, CD14, CD19, CD20, CD56). Despite this phenotypic characterization, identification and purification of DC remains difficult as the majority of these antigens are expressed by other cell types. Most published reports have utilized DC isolated from the mouse spleen, which show that DC are unique APC in that they are capable of activating naive T cells in primary antigen-specific response (Inaba et al., 1987, J Exp Med 166: 182-194; Hengel et al., 1987 J Immunol 139: 4196-4202; Kast et al., 1988, J Immunol 140: 3186-3193; Romani et al., 1989, J Exp Med 169: 1169-1178; Macatonia et al., 1989, J Exp Med 169: 1255-1264: Inaba et al., 1990, J Exp Med 172: 631-640). Therefore, it is possible that human DC are also capable of such potent antigen presentation function.

The most suitable way to enrich DC from human tissue is their isolation from human peripheral blood because of its easy accessibility. Several studies have described the isolation of human DC from the peripheral blood (Young and Steinmann, 1990, J Exp Med 171: 1315-1332; Freudenthal and Steinman, 1990, Proc Natl Acad Sd USA 87: 7698-7702; Macatonia et al., 1989, Immunol. 67: 285-289; Markowicz and Engleman, 1990, J Clin Invest 85: 955-961). Current protocols for the isolation of fresh blood DC consist of two different components: First, enrichment of DC by consecutive depletion of other cell populations with the help of lineage-specific monoclonal antibodies directed to cell surface antigens not expressed by DC. Examples of such antibodies include anti-CD3, anti-CD4 and anti-CD8 specific for T cells; anti-CD19 and anti-CD20 specific for B cells; anti-CD14 specific for monocytes; and anti-CD56 specific for natural killer cells. And second, positive selection of DC from the lineage markers negative cell fraction by using cell surface markers differentially expressed by DC as MHC class II antigen (O'Doherty et al., 1994, Immunol 82: 487-493; Thomas et al., 1993, J Immunol 150: 821-834; O'Doherty et al., 1993, J Exp Med 178: 1067-1076). Upon binding to antibodies, cells can be removed by adsorption to a solid surface coated with secondary antibodies directed to the constant region of the primary antibodies. Alternatively antibodies conjugated with biotin can be removed by an avidin or streptavidin-coated surface. If antibodies are conjugated to magnetic beads, antibody bound cells can be separated in an magnetic field (Harlow and Lane, 1988, "Antibody", Cold Spring Harbor Laboratory). In addition to antibody selection density gradient centrifugation and red blood cell rosetting techniques are applied. This procedures are time-consuming and yield only limited numbers of purified DC. Moreover the isolated DC fraction often is heterogeneous due to other contaminating cell populations. Besides gradient compounds and their osmotic effects may induce phenotypic and functional changes of cells (McLellan et al., 1995, Eur L Immunol 25: 2064-2068; Kabel et al., 1989, Immunbiology 179: 341-395).

Another widely used technique to obtain DC is based on the differentiation of bone marrow or blood derived CD34+ cells or of CD14+ monocytes, induced by a combined in vitro treatment with GM-CSF and TNF-α or IL-4 which has to be maintained for at least several days (Caux et al., 1992, Nature 360: 258-261; Bemhard et al., 1995, Cancer Res 55: 1099-1104); Sallusto et al., 1994, J Exp Med 179: 1109-1118; Romani et al., 1994, J Exp Med 180: 83-93). The use of this procedures is restricted to phenotypic and functional changes which may occur during cultivation of the cells. Moreover treatment with GM-CSF and IL-4 may enhance the nonspecific stimulation of T cells (Dillon et al., 1997, Scand J Immunol 46: 1-9).

Direct isolation of DC from peripheral blood is hampered by their low frequency in the circulation and by the lack of selective markers. No human DC specific lineage marker has been identified. The few markers reported so far to be specific for DC are not suitable for isolation of blood DC. The CD83 molecule described by Zhou et al. (Zhou et al., 1995, J lmmunol 154: 3821-3835) is regarded as an activation marker preferentially induced during in vitro culture. The mAb CMRF-44 recognizes an early activation antigen that is not expressed on freshly isolated blood DC but is induced during the physical isolation procedures usually used to purify DC (Hock et al., 1994, Immunology 83: 573-581). The p55 antigen, an actin bundling protein, is confined to the cytoplasm (O'Doherty et al., 1993, J Exp Med 178: 1067-1076). So there remains a need for monoclonal antibodies directed to antigens selectively expressed by human DC that may be used to facilitate their direct isolation from peripheral blood. Previous attempts to isolate antibodies specific for human DC have been largely unsuccessful, yielding only antibodies that bind common antigens of both DC and other leukocytes.

WO 98/15579 describes an antibody, CMRF-56 which reacts with dendritic cells but also with other PBMCs, in particular also a subpopulation of CD19⁺ lymphocytes.

Tuting (1998), J. of Immunology, 160, 1139-1147, discloses genetically modified human monocyte-derived dendritic cells which express melanoma antigens in vitro.

O'Doherty (1994), Immunology, 82, 487-493, described that the human blood contains two subsets of dendritic cells, in particular immature dendritic cells.

Thomas (1994), J. of Immunology, 153, 4016-4028, also described human peripheral blood dendritic cells and characterized subsets of precursor and mature antigen-presented cells.

Thus, the technical problem of the present invention is to provide means and methods for the modulation of immune responses.

The solution to this technical problem is achieved by providing the embodiments characterized in the claims.

Accordingly, the invention relates to an antibody which
(i) reacts with an epitope on dendritic cells (DCs) displaying features of immature and/or mature DCs from peripheral blood mononuclear cells (PBMCs) but
(ii) does not react with other PBMCs, wherein said antibody is produced by hybridoma cell line DSM ACC 2241, by hybridoma cell DSM ACC 2399 or by hybridoma cell line DSM ACC 2398.

Usually, dendritic cells comprise a heterogeneous population of cells with different phenotypes and functional properties they all stand out by their marked capacity to present antigen and to prime naive T lymphocytes¹. Conventional strategies such as depletion of lineage marker positive cells and subsequent isolation of MHC class II-expressing cells have led to the definition of two major DC populations which largely represent mature and immature DC^{2,3}. More recently, several reports attempted to divide DC according to various categories into lymphoid or myeloic dendritic cells (Wu et al., J. Exp. Med. 184 (1996), 903-911; Galy et al., Immunity 3 (1995), 459-473), or marginal and interdigitating cells depending on the microanatomical location in the murine spleen (Leenen et al., J. Immunol 160 (1998), 2166-2173). The antibody of the present invention specifically reacts with a hitherto unidentified population of DC that comprise DCs displaying properties of immature DCs which are HLA-DR^{low}, CD33^{dim}, CD45RA^{high}, CD11c⁻ and CD64⁻ and DCs resembling substantially mature DCs with the surface marker characteristics HLA-DR^{high}, CD33^{high}, CD45RA^{low}, CD11c⁺ and CD64⁺. The DCs recognized by the antibody of the invention, may preferably be defined as being a maturational stage between immature and mature DCs and by their capability to react with the novel antibody of the invention designated mAb M-DC8. Accordingly, the DCs of the present invention will also be referred to as M-DC8⁺ or M-DC8⁺ cells. The above cited novel mAb M-DC8 was used to specifically select Jurkat T cell lymphoma subclones which express the M-DC8 antigen (see appended example 10). Said Jurkat subclone was employed to generate further novel antibodies of the invention. These antibodies comprise the mAbs D-DC8.1 and D-DC8.2. DCs recognized by these antibodies will also be referred to as M-CD8⁺ or M-DC8⁺ cells.

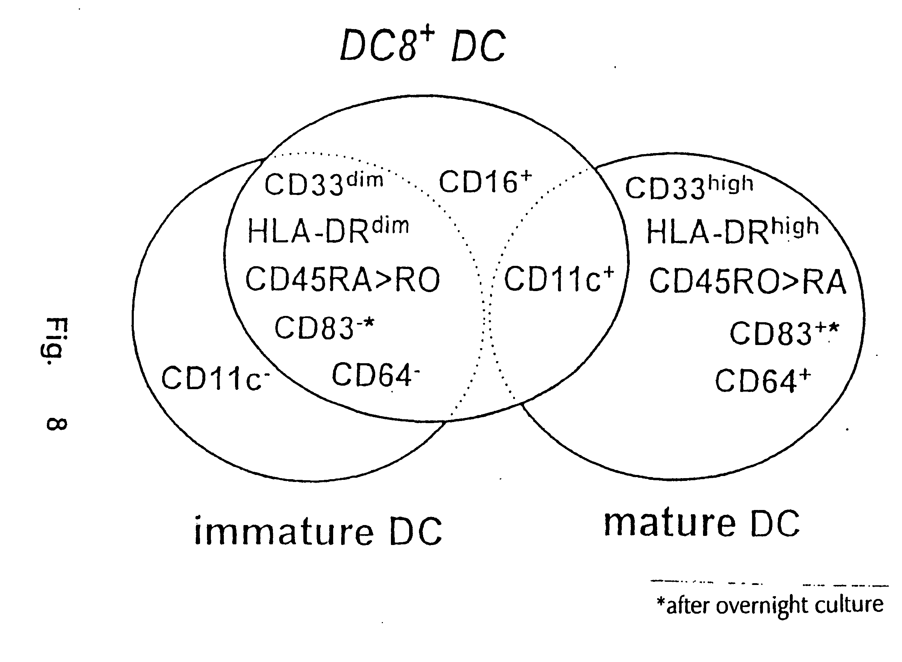

In context with the present invention, the term "DC population representing a maturational stage between immature and mature DCs" refers to a population of human blood DC that overlaps with two major subsets of DC that have previously been defined by several investigators^{2,3,20} as schematically depicted in Figure 8. Cells of the first subset are HLA-DR^{low}, CD33dim, CD45RA^{high}, CD11c- and CD64- and represent immature DC, whereas the second subset contains mature DC with the surface marker characteristics HLA-DR^{high}, CD33^{high}, CD45RA^{low}, CD11c⁺ and CD64⁺. These cells, in addition, express CD83 after overnight culture²⁰. The main distinction between both established DC populations and M-bC8⁺ cells is the expression of M-DC8 and CD16 and the absence of the intracytoplasmic p55 antigen. So, it is tempting to speculate that M-DC8⁺ cells represent an intermediate developmental stage.

The term "peripheral blood mononuclear cells (PBMCs)" within the meaning of the present invention refers to nucleated blood cells isolated by centrifugation of freshly drawn venous blood over a Ficoll^{R}-gradient of 1.077 g/ml density.

The term "does not react with other PBMCs", as used herein means that the antibody of the invention does not react or only negligible cross-reacts with PBMCs (like B-lymphocytes, T-lymphocytes and CD14^{high+} monocytes) as defined above except the M-DC8⁺ cells. Preferably, the cross-reactivity of the antibody of the invention is less than 10%, more preferably less than 5% and particularly preferred less than 3% or even less than 2% or 1%.

The present invention is based on the observation that a novel monoclonal antibody (mAb M-DC8) obtained by immunizing mice with human mononuclear blood cells highly enriched for DC (obtained as described in appended example I) specifically reacted with leukocytes comprising 1-2% of PBMC expressing HLA-DR and lacking common cell lineage-specific markers. The characteristic light scatter profile of M-DC8⁺ cells indicated a cell population of restricted size and granularity located between lymphocytes and monocytes. Within 48h of cell. culture isolated M-DC8⁺ cells acquired the typical morphology of mature DC characterized by undulating cytoplasmatic protrusions¹. In contrast to previous reports describing DC as poorly phagocytic cells³ freshly isolated M-DC8⁺ cells avidly ingested latex particles as well as antibody coated erythrocytes. While immature DC take up large quantities of antigens via endocytosis and macropinocytosis⁴, the findings on M-DC8⁺ cells obtained in accordance with the invention point to phagocytosis as an additional important way of antigen uptake.

Quite recently, Fanger et al. described the expression of the Fc receptors CD64 and CD32 on DC and their functional involvement in phagocytosis²⁰. In contrast, M-DC8⁺ cells lack CD64 but, instead, express CD16 together with CD32. Thus, phagocytosis of opsonized erythrocytes by M-DC8⁺ cells might be mediated through both CD16 and CD32.

in accordance with the present invention, it has surprisingly been found that besides their marked phagocytic activity fresh M-DC8⁺ cells exhibited an outstanding capacity to activate T cells as evidenced by the efficient alloantigen- and TT-dependent T cell proliferation. Furthermore, M-DC8⁺ cells not only stimulated T cells in an autologous mixed leukocyte reaction but also induced a primary T cell response against KLH, activities that all are considered typical for DC¹. In contrast to previous reports claiming that naive CD45RA⁺ T cells need to be enriched In order to obtain a primary T cell response^{21,22}, M-DC8⁺ cells could prime unselected T cells against KLH without prior enrichment. In addition, M-DC8⁺ cells very efficiently stimulated MHC class I restricted T cell responses. They activated a cytotoxic CD8⁺ T cell clone after sensitization with the specific antigen peptide as effectively as monocytes, but, In contrast to the latter, they also induced the differentiation of purified CD8⁺ T cells into alloantigen-specific cytotoxic effector cells in the total absence of CD4⁺ T helper cells, an activity claimed to be characteristic for DC^{17,18}. In addition, after being loaded with a melanoma-associated tyrosinase peptide M-DC8+ cells stimulated T cells from melanoma patients as well as from normal donors to develop specific cytotoxic activity against melanoma cells in a HLA-restricted fashion.

M-DC8⁺ cells revealed a unique pattern of cell surface molecules clearly distinguishing them from CD14⁺ monocytes. Freshly isolated M-DC8⁺ cells expressed lower surface levels of HLA-DR, CD33, CD45RO and CD11b whereas the density of CD86, CD40 and CD4 was similar to that found on monocytes. On the other hand, expression of CD45RA, CD11a, CD11c and, particularly, of CD16 was distinctly higher than on monocytes.

M-DC8⁺ cells differ from the majority of DC that are generated in vitro from CD34⁺ precursor cells or CD14⁺ monocytes in the presence of GM-CSF and IL-4¹⁴. M-DC8⁺ cells share a number of surface markers and functional characteristics with DC obtained by cytokine-driven differentiation in vitro. The M-DC8 antigen, however, was not detected on those in vitro generated DC with the exception of a small subset comprising 2-5% of DC that had been produced from CD34⁺ precursors through stimulation with GM-CSF and TNF-α. The origin of M-DC8⁺ cells as well as the signals inducing the expression of the M-DC8 marker remain to be elucidated.

FcγRIII (CD16) is an additional surface molecule whose expression was found to be different on M-DC8⁺ cells and on the heretofore defined DC. So far, CD16 expression by DC has been described only on Langerhans cells in mice, where it was supposed to enhance Internalization of immune complexes and presentation of antigens²³. In humans, CD16 expression was mainly confined to granulocytes, NK cells, macrophages and a subpopulation of monocytes²⁴ while DC were generally thought to be negative. In contrast, DC freshly isolated with the M-DC8 antibody expressed CD16 at higher concentration. This obvious discrepancy may be due to the short in vitro half life of CD16 on DC since It disappeared within a short period of in vitro culture. In general, Fey receptors seem to be downregulated very rapidly by prolonged handling of cells such as Ficoll density gradient centrifugation²⁵.

In a preferred embodiment of the invention, said DCs are HLA-DR⁺ and said PBMCs are depleted of T- and B-cells and monocytes. Preferably, said DCs are CD64⁻, CD33⁺, CD45RA⁺, CD11c⁺ and p55⁻ and mostly CD16⁺ and/or of restricted size and granularity located between lymphocytes and monocytes.

The antibody of the invention can be an antibody or immunoglobulin chain encoded by the herein disclosed polynucleotide as discussed below or the antibody prepared by the method as disclosed below, wherein said antibody of said immunoglobulin chain is capable of recognizing dendritic cells (DCs) from peripheral blood mononuclear cells (PBMCs) and wherein said antibody or immunoglobulin chain may be a monoclonal antibody, chimeric antibody, humanized antibody, bispecific antibody, synthetic antibody, antibody fragment such as Fab, Fv or scFv fragments etc., or a chemically modified derivative of any of these. Monoclonal antibodies can be prepared, for example, by the techniques as originally described in Köhler and

Milstein, Nature 258 (1975), 495, and Galfré, Meth. Enzymol. 73 (1981), 3, which comprise the fusion of mouse myeloma cells to spleen cells derived from immunized mammals with modifications developed in the art. Monoclonal antibodies can, inter alia, be obtained by immunizing mice, for example BALB/c mice with human mononuclear blood cells obtainable as described in appended example 1.

The antibodies can be monoclonal antibodies or synthetic antibodies as well as fragments of antibodies, such as Fab, Fv or scFv fragments etc. Furthermore, antibodies or fragments thereof to the aforementioned DCs can be obtained by using methods which are described, e.g., in Harlow arid Lane "Antibodies, A Laboratory Manual", CSH Press, Cold Spring Harbor, 1988. These antibodies can be used, for example, for the immunoprecipitation, immunolocalization or purification of the DCs of the invention as well as for the monitoring of the presence of such DCs and for the identification of compounds interacting with the DCs according to the invention. For example, surface plasmon resonance as employed in the BlAcore system can be used to increase the efficiency of phage display of antibodies which bind to an epitope recognized by an antibody of the invention (Schier, Human Antibodies Hybridomas 7 (1998), 97-105; Malmborg, J. Immunol. Methods 183 (1995), 7-13). The production of chimeric antibodies is described, for example, in WO89/09622. Methods for the production of humanized antibodies are described in, e.g., EP-A1 0 239 400 and WO90/07861. Furthermore, human antibodies in general have become accessible since the availability of transgenic mice expressing human antibodies also called xenogenic antibodies (Brüggemann. Immunol. Today 17 (1996), 391-397) and of the combinatorial antibody library and phage display technology allowing the In vitro combination of variable regions of immunoglobulin heavy and light chains (V_{H} and V_{L}) and the in vitro selection of their antigen binding specificity (Winter, Annu. Rev. Immunol. 12 (1994), 433-455). By using the phage display method, rare events like one specific binding entity out of 10⁷ to 10⁹ different V_{L}/V_{H}- or V_{H}/V_{L}-pairs can easily be isolated; this is especially true when the repertoire of variable regions has been enriched for specific binding entities by using B-lymphocytes from immunized hosts as a source for repertoire cloning. In addition, approaches using semisynthetic or fully synthetic V_{H}- and/or V_{L}- immunoglobulin chain repertoires have been developed. For example, almost the complete repertoire of unrearranged human V-gene-segments has been cloned from genomic DNA and used for in vitro recombination of functional variable region genes, resembling V-J- or V-D-J-recombination in vivo (Hoogenboom, J. Mol. Biol. 227 (1992), 381-388; Nissim, EMBO J. 13 (1994) 692-698; Griffiths, EMBO J. 13 (1994), 3245-3260). Hence, all these derivatives of the antibody described above and in the appended examples are within the scope of the present invention as long the antibody recognizes at least one epitope of an antigen specific for the DCs as defined above that is preferably the antigen recognized by the M-DC8, the D-DC8.1 and/or the D-DC8.2 antibody. As discussed above, the antibody of the invention may exist in a variety of forms besides complete antibodies; including, for example, Fv, Fab and F(ab)₂, as well as in single chains; see e.g. WO88/09344.

The antibodies of the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook, Molecular Cloning A Laboratory Manual, Cold Spring Harbor Laboratory (1989) N.Y.

In a preferred embodiment of the invention said bispecific antibody of the invention recognizes an epitope specific for a tumor cell, a virus-infected cell, a T cell, a tumor-associated protein, a microbial protein, an allergen, an autoantigen or a cytokine.

The DCs may be recognized by the antibody produced by hybridoma cell line DSM ACC 2241, preferably said antibody is the antibody M-DC8 (DC8) that is produced by hybridoma cell line DSM ACC 2241. Said hybridoma cell has been deposited in the culture collection Deutsche Sammlung von Mikroorganismen und Zellkulturen GmbH (DSMZ) in Braunschweig, Germany on October 26, 1995, in accordance with the Budapest Treaty. As described above and in the appended examples, the novel mAb M-DC8 provides a highly selective marker for a unique population of human DC which account for 0,5-1% of blood leukocytes and which can directly be isolated from blood using a one step immunomagnetic procedure. M-DC8⁺ cells characteristically express FcγRIII (CD16), they are highly phagocytic and display an outstanding capacity to present antigen to T cells as documented by autologous mixed leukocyte reaction, activation of T cells against primary antigens, induction of differentiation of purified CD8⁺ T cells into alloantigen-specific cytotoxic effector cells and induction of differentiation of T cells from melanoma patients and from normal blood donors into melanoma-specific cytotoxic cells. The mAb M-DC8 thus is a valuable tool to determine circulating DC for diagnostic purposes and to prepare DC for ex vivo and in vivo antigen-specific T cell-priming. Furthermore, the antibody of the invention is an antibody, which recognizes, detects and/or reacts with an epitope on said dendritic cells (DCs) and is produced by the hybridoma cell lines DSM ACC 2399 or DSM ACC 2398. These hybridoma cells have been deposited in the culture collection DSMZ in Braunschweig, Germany on May 5, 1999, in accordance with the Budapest Treaty. As described in the appended examples, these novel mAbs D-DC8.1 and D-DC8.2 provide for further antibodies which recognize the specific M-DC8⁺ cells described herein above. Like M-DC8, the mAbs D-DC8.1 and D-DC8.2 can be used, inter alia, for the immunoisolation, immunolocalization and/or purification of dendritic cells of the invention. Furthermore, they are useful for the detection and identification of compounds which are interacting or capable of interacting with the DCs according to the invention.

In another embodiment the present invention relates to a human B cell line which is capable of producing the antibody of the invention in a humanized form, e.g., by transduction of the cDNA coding for the variable heavy and light chain domains of the M-DC8, or of the D-DC8.1 or of the D-DC8.2 antibody linked to the constant domains of an immunoglobulin into a human B cell line. Said cDNA is obtainable by methods known to the person skilled in the art and are described, inter alia, in Sambrook, loc. cit. and Ausubel "Current Protocols in Molecular Biology", Green Publishing Associates and Wiley Interscience, N.Y. (1989). The cloning of said cDNA for expression (or sequencing) may follow standard protocols as described, e.g., In Orlandi, PNAS 86 (1989), 3833-3837 or as illustrated in appended example 9, documenting the cloning of the variable regions of mAb M-DC8.

Furthermore, the present invention relates to a continuous, stable antibody-producing cell line which is capable of producing an antibody of the invention. Said cell line can be a hybridoma cell line, preferably the hybridoma cell line having the deposit number DSM ACC 2241, the deposit number DSM ACC 2399 or the deposit number DSM ACC 2398.

In a still further embodiment, the present invention relates to an antigen or an epitope thereof which is recognized by an antibody of the invention. Said antigen or epitope may be glycosylated, unglycosylated or partially deglycosylated. As discussed herein and explained in the examples, the DCs of the present invention feature novel antigens, recognized by the aforedescribed antibodies, such as M-DC8. Preliminary biochemical data indicate that the M-DC8 antigen is a protein, more specifically, a carbohydrate moiety of proteins. This antigen comprises therefore a carbohydrate structure that may be present on glycolipids as well as on, inter alia, membrane proteins. A major advantage of this new marker is its capability to serve as a handle for rapid isolation of >97% pure DC from blood within a very short period of time. This rapid method of DC isolation may greatly facilitate the use of DC for various ex vivo attempts to immunize against viral or tumor antigens. For the identification and isolation of antigen and epitopes of the invention, e.g., cDNA libraries can be screened by injecting various cDNAs into oocytes, allowing sufficient time for expression of the cDNA gene products to occur, and testing for the presence of the desired cDNA expression product, for example, by using the antibody of the invention.

Alternatively, a cDNA expression library in E. coli can be screened indirectly for peptides having at least one epitope of the invention using antibodies of the invention (Chang and Gottlieb, *J. Neurosci.,* 8:2123, 1988). After having revealed the structure of such antigens the rational design of binding partners and/or domains may be possible. For example, folding simulations and computer redesign of structural motifs can be performed using appropriate computer programs (Olszewski, Proteins 25 (1996), 286-299; Hoffman, Comput. Appl. Biosci. 11 (1995), 675-679). Furthermore, computers can be used for the conformational and energetic analysis of detailed protein models (Monge, J. Mol. Biol. 247 (1995), 995-1012; Renouf, Adv. Exp. Med. Biol. 376 (1995), 37-45).

In another embodiment the present invention relates to a polynucleotide encoding at least a variable region of an immunoglobulin chain of any of the before described antibodies of the invention as produced by the hybridoma cell line DSM ACC 2241, DSM ACC 2399 or DSM ACC 2398. Polynucleotides encoding said regions are obtainable by methods which are well known in the art and comprise, inter alia, cloning techniques as described in Orlandi, PNAS 86 (1989), 3833-3837 or Sambrook, loc. cit. One form of immunoglobulin constitutes the basic structural unit of an antibody. This form is a tetramer and consists of two identical pairs of immunoglobulin chains, each pair having one light and one heavy chain. In each pair, the light and heavy chain variable regions or domains are together responsible for binding to an antigen, and the constant regions are responsible for the antibody effector functions. In addition to antibodies, immunoglobulins may exist in a variety of other forms (including less than full-length that retain the desired activities), including, for example, Fv, Fab, and F(ab')2, as well as single chain antibodies (e.g., Huston, Proc. Nat. Acad. Sci. USA 85(1988,5879-5883 and Bird, Science 242(1988), 423-426); see also supra. An immunoglobulin light or heavy chain variable domain consists of a "framework" region interrupted by three hypervariable regions, also called CDR's. The extent of the framework region and CDR's have been precisely defined; see, e.g., "Sequences of Proteins of Immunological Interest," Kabat, U.S. Department of Health and Human Services (1990). The sequences of the framework regions of different light or heavy chains are relatively conserved within a species. The framework region of an antibody, that is the combined framework regions of the constituent light and heavy chains, serves to position and align the CDR's. The CDR's are primarily responsible for binding to an epitope of an antigen. Chimeric antibodies are antibodies whose light and heavy chain genes have been constructed, typically by genetic engineering, from immunoglobulin variable and constant region genes belonging to different species. For example, the variable segments of the genes from a mouse monoclonal antibody may be joined to human constant segments.

Thus, the antibodies of the present invention can be produced by expressing recombinant DNA segments encoding the heavy and light immunoglobulin chain(s) of the antibody invention either alone or in combination. Said polynucleotide may be, e.g., DNA, cDNA, RNA or synthetically produced DNA or RNA or a recombinantly produced chimeric nucleic acid molecule comprising any of those polynucleotides either alone or in combination. Preferably said polynucleotide is part of a vector. Such vectors may comprise further genes such as marker genes which allow for the selection of said vector in a suitable host cell and under suitable conditions. Preferably, the polynucleotide of the invention is operatively linked to expression control sequences allowing expression in prokaryotic or eukaryotic cells. Expression of said polynucleotide comprises transcription of the polynucleotide into a translatable mRNA. Regulatory elements ensuring expression in eukaryotic cells, preferably mammalian cells, are well known to those skilled in the art. They usually comprise regulatory sequences ensuring Initiation of transcription and optionally poly-A signals ensuring termination of transcription and stabilization of the transcript. Additional regulatory elements may include transcriptional as well as translational enhancers, and/or naturally-associated or heterologous promoter regions. In this respect, the person skilled in the art will readily appreciate that the polynucleotides encoding at least the variable domain of the light and/or heavy chain may encode the variable domains of both immunoglobulin chains or only one. Likewise, said polynucleotides may be under the control of the same promoter or may be separately controlled for expression. Possible regulatory elements permitting expression in prokaryotic host cells comprise, e.g., the P_{L}, *lac, trp* or *tac* promoter in *E. coli,* and examples for regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron In mammalian and other animal cells. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pCDM8, pRc/CMV, pcDNA1, pcDNA3 (In-vitrogene), pSPORT1 (GIBCO BRL).

Preferably, the expression control sequences will be eukaryotic promoter systems in vectors capable of transforming or transfecting eukaryotic host cells, but control sequences for prokaryotic hosts may also be used. Once the vector has been incorporated into the appropriate host, the host is maintained under conditions suitable for high level expression of the nucleotide sequences, and, as desired, the collection and purification of the immunoglobulin light chains, heavy chains, light/heavy chain dimers or intact antibodies, binding fragments or other immunoglobulin forms may follow; see, Beychok, Cells of Immunoglobulin Synthesis, Academic Press, N.Y., (1979).

As described above the polynucleotide of the invention can be used alone or as part of a vector to express the antibody of the invention in cells, for, e.g., gene therapy or diagnostics of diseases related to malignant transformed DCs or DC related leukemias. The polynucleotides or vectors containing the DA sequence(s) encoding any one of the above described antibodies is introduced Into the cells which in turn produce the antibody of interest Gene therapy, which is based on introducing therapeutic genes into cells by *ex-vivo* or *in-vivo* techniques is one of the most important applications of gene transfer. Suitable vectors, methods or gene-delivery systems for *in-vitro* or *in-vivo* gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996). 714-716; WO94/29469; WO 97/00957, Onodua, Blood 91 (1998), 30-36; Verzeletti, Hum. Gene Ther. 9 (1998), 2244-2251; Verma, Nature 389 (1997), 239-242; US 5,580,859; US 5,589,466; US 4,394,448 or Schaper, Current Opinion in Biotechnology 7 (1996), 635-640, and references cited therein. The polynucleotides and vectors of the invention may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell. This embodiment is particularly suited for bispecific antibodies of the invention, e.g., with one specificity against a tumor antigen which would facilitate the internalization, processing and presentation of tumor cells or parts thereof such as soluble tumor antigens and the like.

Furthermore, the present invention relates to vectors, particularly plasmids, cosmids, viruses and bacteriophages used conventionally in genetic engineering that comprise a polynucleotide encoding a variable domain of a chain of an antibody of the invention; optionally in combination with a polynucleotide of the invention that encodes the variable domain of the other chain of the antibody of the invention. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the polynucleotides or vector of the invention into targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1989). Alternatively, the polynucleotides and vectors of the invention can be reconstituted into liposomes for delivery to target cells. The vectors containing the polynucleotides of the invention (e.g., the heavy and/or light variable domain(s) of the immunoglobulin chains encoding sequences and expression control sequences) can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra. Once expressed, the whole antibodies, their dimers, individual light and heavy chains, or other immunoglobulin forms of the present invention, can be purified according to standard procedures of the art, including ammonium sulfate precipitation, affinity columns, column chromatography, gel electrophoresis and the like; see, Scopes, "Protein Purification", Springer-Verlag, N.Y. (1982). Substantially pure immunoglobulins of at least about 90 to 95% homogeneity are preferred, and 98 to 99% or more homogeneity most preferred, for pharmaceutical uses. Once purified, partially or to homogeneity as desired, the polypeptides may then be used therapeutically (including extracorporeally) or in developing and performing assay procedures.

The present invention furthermore relates to host cells transformed with a polynucleotide or vector of the invention. Said host cell may be a prokaryotic or eukaryotic cell. The polynucleotide or vector of the invention which is present in the host cell may either be integrated into the genome of the host cell or it may be maintained extrachromosomally.

The host cell can be any prokaryotic or eukaryotic cell. The term "prokaryotic" is meant to include all bacteria which can be transformed or transfected with a DNA or RNA molecules for the expression of an antibody of the invention or the corresponding immunoglobulin chains. Prokaryotic hosts may include gram negative as well as gram positive bacteria such as, for example, *E. coli, S. typhimurium, Serratia marcescens* and *Bacillus subtilis.* The term "eukaryotic" is meant to include but not being limited to insect, fungal, plant, animal or human cells. Preferred fungal cells are, for example, those of the genus Saccharomyces, in particular those of the species S. cerevisiae. A polynucleotide coding for an antibody of the invention can be used to transform or transfect the host using any of the techniques commonly known to those of ordinary skill in the art. Especially preferred is the use of a plasmid or a virus containing the coding sequence of the antibody recognizing the DCs of the invention for purposes of eukaryotic or prokaryotic transformation or transfection, respectively. Methods for preparing fused, operably linked genes and expressing them in, e.g., mammalian cells and bacteria are well-known in the art (Sambrook, et al., Molecular Cloning: A Laboratory Manual, Cold Spring Harbor Laboratory, Cold Spring Harbor, NY, 1989). The genetic constructs and methods described therein can be utilized for expression of the antibody of the invention in eukaryotic or prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of providing phenotypic selection of the transformed cells. The transformed hosts can be grown in fermentors and cultured according to techniques known in the art to achieve optimal cell growth. The antibody or its corresponding immunoglobulin chain(s) of the invention can then be isolated from the growth medium, cellular lysates, or cellular membrane fractions. The isolation and purification of the, e.g., microbially expressed antibodies or immunoglobulin chains of the invention may be by any conventional means such as, for example, preparative chromatographic separations and immunological separations such as those involving the use of monoclonal or polyclonal antibodies directed, e.g., against the constant region of the antibody of the invention.

Thus, in a further embodiment the invention relates to a method for preparing an antibody or a functional fragment thereof, said antibody or said functiaonl fragment being capable of recognizing dendritic cells (DCs) of a DC population of immature and mature DCs from peripheral blood mononuclear cells (PBMCs), said method comprising
(a) culturing the cell of the invention and
(b) isolating said antibody, functional fragment or immunoglobulin chain thereof from the cells or the culture medium.

The present invention also involves a method for producing cells capable of expressing an antibody of the invention or its corresponding immunoglobulin chain(s) comprising genetically engineering cells with the polynucleotide or with the vector of the invention. Preferably, the immunoglobulin chain(s) thus expressed are displayed on the cell surface of the transfected cell. This embodiment as well as some others mentioned herein may be adapted for phage display techniques such as described above. The cells obtainable by the method of the invention can be used, for example, to test the interaction of the antibody of the invention with its antigen. The cells obtainable by the above-described method may also be used for the screening methods referred to herein below. Furthermore, transgenic animals, preferably mammals, comprising a polynucleotide, vector or cells of the invention may be used for the large scale production of the antibody of the invention.

Furthermore, the invention relates to an antibody of the invention or a fragment or a derivative thereof or immunoglobulin chain encoded by a polynucleotide according to the invention or obtainable by the above-described method or from cells produced by the method described above. The antibodies of the present invention will typically find use individually in treating substantially any disease susceptible to monocional antibody-based therapy. In particular, the immunoglobulins can be used for passive immunization or the removal of unwanted cells or antigens, such as by complement mediated lysis, all without substantial immune reactions (e.g., anaphylactic shock) associated with many prior antibodies. For an antibody of the invention, typical disease states suitable for treatment include graft versus host disease and transplant rejection in patients undergoing an organ transplant, such as heart, lungs, kidneys, liver, etc. Other diseases include autoimmune diseases, such as Type I diabetes, multiple sclerosis, rheumatoid arthritis, systemic lupus erythematosus, and myasthenia gravis. Derivatives of the antibody of the present invention can be prepared by methods known to the person skilled in the art, inter alia, by peptidomimetics. Methods for the generation and use of peptidomimetic combinatorial libraries are described, for example in Ostresh, Methods in Enzymology 267 (1996), 220-234 and Domer, Bioorg. Med. Chem. 4 (1996), 709-715. Furthermore, the three-dimensional and/or crystallographic structure of antigens like the M-DC8 antigen can be used for the design of peptidomimetic antibody derivatives (Rose, Biochemistry 35 (1996), 12933-12944; Rutenber, Bioorg. Med. Chem. 4 (1996), 1545-1558).

In this context it is also understood that the antibodies according to the invention may be further modified by conventional methods known in the art. By providing the antibodies according to the present invention it is also possible to determine the portions relevant for their binding activity. This may allow the construction of chimeric proteins comprising an amino acid sequence derived from an antibody of the invention which is crucial for binding activity and other functional amino acid sequences e.g. nuclear localization signals, transactivating domains, DNA-binding domains, hormone-binding domains, protein tags (GST, GFP, h-myc peptide, Flag, HA peptide) which may be derived from heterologous proteins.

The antibodies of the invention can be used therapeutically in patients having an autoimmune response, e.g., to proteins or glycoproteins of hormone producing cells such as islet (β) cells of the pancreas, of cells of the thyroid gland, of glial cells in the central nervous system including myelin sheaths, of synovial cells or synovial tissue, of cells or tissue of the uvea or of cells or tissue of the blood vessel wall. Such therapy can be accomplished by, for example, the administration of antibodies, antigens or epitopes of the invention. Such administration can utilize unlabeled as well as labeled antibodies or antigens. It is, for example, envisaged when unlabeled the antigen or epitope is utilized advantageously, it would be in a form wherein, for example, the antigens are in fragments which are too small to stimulate an immune response, but large enough to bind, or block, the continuance of the autoimmune response. For example, the antigen of the invention could be digested enzymatically into epitope-sized peptides (typically 5-12 amino acids in length) and thereby bind to Fab binding portions present in the body fluids, or on the surface of DCs, of the patient with autoimmune disease.

Alternatively, the antibodies of the invention could be administered linked to a therapeutic agent. These agents can be coupled either directly or indirectly to the antibodies of the invention. One example of indirect coupling is by use of a spacer moiety. These spacer moieties, In tum, can be either insoluble or soluble (Diener, *et al., Science,* 231:148, 1988) and can be selected to enable drug release from the antigen at the target site. Examples of therapeutic agents which can be coupled to the antibodies of the invention for immunotherapy are drugs, radioisotopes, lectins, and toxins. The drugs with which can be conjugated to the antibodies, antigens and epitopes of the invention include compounds which are classically referred to as drugs such as mitomycin C, daunorubicin, and vinblastine.

In using radioisotopically conjugated antibodies of the invention for, e.g., immunotherapy, certain isotopes may be more preferable than others depending on such factors as leukocyte distribution as well as stability and emission. Depending on the autoimmune response, some emitters may be preferable to others. In general, α and β particle-emitting radioisotopes are preferred In immunotherapy. Preferred are short range, high energy α emitters such as ²¹²Bi. Examples of radioisotopes which can be bound to the antibodies, antigens or epitopes of the invention for therapeutic purposes are ¹²⁵I, ¹³¹I, ⁹⁰Y, ⁶⁷Cu, ²¹²Bi, ²¹²At, ²¹¹Pb, ⁴⁷Sc, ¹⁰⁹Pd and ¹⁸⁸Re.

Lectins are proteins, usually isolated from plant material, which bind to specific sugar moieties. Many lectins are also able to agglutinate cells and stimulate lymphocytes. However, ricin is a toxic lectin which has been used immunotherapeutically. This is accomplished by binding the α-peptide chain of ricin, which is responsible for toxicity, to the antibody molecule to enable site specific delivery of the toxic effect.

Toxins are poisonous substances produced by plants, animals, or microorganisms that, in sufficient dose, are often lethal. Diphtheria toxin is a substance produced by *Corynebacterium diphtheria* which can be used therapeutically. This toxin consists of an a and β subunit which under proper conditions can be separated. The toxic A component can be bound to an antibody or antigen and used for site specific delivery to a DC or T-cell expressing a receptor for the antigen, respectively.

Other therapeutic agents such as described above which can be coupled to the antibody of the invention, as well as *ex vivo* and *in vivo* therapeutic protocols, are known, or can be easily ascertained, by those of ordinary skill in the art. Wherever appropriate the person skilled in the art may use a polynucleotide of the invention encoding any one of the above described antibodies, antigens or epitopes or the corresponding vectors instead of the proteinaeous material itself. Furthermore, dendritic cells and/or T cells which have been obtained according to the method of the invention and optionally modified can be used in accordance with the embodiments described above.

According to the foregoing, the present invention further relates to a polypeptide comprising
(a) a domain of a binding site of the antibody of the invention as produced by the hybridoma cell line DSM ACC 2241, DSM ACC 2399 or DSM ACC 2398; and
(b) at least one further domain, said domains being linked by covalent or non-covalent bonds.

Said binding site domain contained in a polypeptide of the invention comprises at least one complementarity determining region (CDR) of the antibody of the invention. The person skilled in the art knew that each variable domain (the heavy chain V_{H} and light chain V_{L}) of an antibody comprises three hypervariable regions, sometimes called complementarity determining regions or "CDRs" flanked by four relatively conserved framework regions or "FRs". The CDRs contained In the variable regions of the antibody of the invention can be determined, e.g., according to Kabat, "Sequences of Proteins of Immunological Interest" (U.S. Department of Health and Human Services, third edition, 1983, fourth edition, 1987, fifth edition 1990). The person skilled in the art will readily appreciate that the binding site domain of the antibody of the invention or antigen or epitope of the invention can be used for the construction of other polypeptides or antibodies of desired specificity and biological function. Thus, the present invention also relates to polypeptides and antibodies comprising a binding site domain or antigen or epitope of the invention. The person skilled In the art will readily appreciate that using the binding sites or CDRs described above antibodies can be constructed according to methods known in the art, e.g., as described in EP-A1 0 451 216, EP-A1 0 549 581 and WO 88/09344.

Multivalent polypeptides such as recombinant bifunctional antibody constructs play an increasingly important therapeutic and scientific role in particular in the medical field, for example, in the development of new treatment approaches for cancer and autoimmune diseases or as interesting tools for the analysis and modulation of cellular signal transduction pathways. For example, by cross-linking of the CD3-activation antigen on T cells with a tumor associated antigen on tumor cells, bispecific single-chain antibodies can bring both cells together so that the tumor cell is efficiently lysed during the cell-cell contact (Mack, Proc. Natl. Acad. Sci. U.S.A. 92 (1995) 7021-7025). Comparable approaches have been or are being developed for other target cells (e.g. virus-infected cells) and for the recruitment of other effector cell populations (e.g. NK-cells and mononuclear phagocytes). Using bifunctional fusion proteins that carry an antibody fragment as targeting mechanism, a large number of different receptors and ligands can be specifically bound to defined surface molecules on selected cell populations. It is particularly interesting that surface molecules on the same cell can be cross-linked by bi-specific antibodies in order to modulate cellular function or the state of activation or differentiation of such cells. A possible application of this type of approach may be the induction of anergy in auto-aggressive B- or T-lymphocytes that play a pathogenetic role In many autoimmune diseases. Regarding the broad scientific and therapeutic relevance, efficient and reproducible methods for producing recombinant polypeptides comprising functional antigen binding sites are of particular importance; such methods yield, for example, functionally active bispecific antibody constructs by expression in bacteria and in mammalian cells. Said recombinant bifunctional single-chain proteins usually are built up by different scFv-antibody fragments, each of which consists of one immunoglobulin variable heavy (V_{H}) and one variable light (V_{L})-antigen binding domain. Alternatively, they may comprise such an antibody fragment and one non-immunoglobulin part. All functional domains are located on a single polypeptide chain and joined together by flexible Glycin-Serin- or other appropriate peptide linkers. The bifunctional polypeptide chain can be produced as functional protein by transfecting mammalian or less preferentially other host cells with the corresponding DNA-sequence, that additionally may encode an optional protein-tag, preferentially a poly-histidine-tag, enabling easy purification of the recombinant protein for example by using a nickel-chelate-column. As has been demonstrated by way of example with a bispecific single-chain antibody functionally expressed in CHO-cells, scFv-antibody fragments can in principle bind to their antigen either as the N-terminal or the C-terminal part of a bifunctional single-chain construct, (Mack, Proc. Natl. Acad. Sci. U.S.A. 92(1995) 7021-7025).

In a preferred embodiment of the invention, said at least one further domain comprises a polypeptide selected from the group consisting of effector proteins having a conformation suitable for biological activity, amino acid sequences capable of sequestering an ion, and amino acid sequences capable of selective binding to a solid support or to a preselected antigen.

Preferably, said effector protein is an enzyme, toxin, receptor, binding site, biosynthetic antibody binding site, growth factor, cell-differentiation factor, lymphokine, cytokine, hormone, a remotely detectable moiety, anti-metabolite or antigen. Said antigen can be, e.g., tumor antigen, a viral antigen, a microbial antigen, an allergen, an auto-antigen, a virus, a microorganism, a polypeptide, a peptide or a plurality of tumor cells.

Furthermore, said sequence capable of sequestering an ion is preferably selected from calmodulin, methallothionein, a fragment thereof, or an amino acid sequence rich in at least one of glutamic acid, aspartic acid, lysine, and arginine.

In addition, said polypeptide sequence capable of selective binding to a solid support can be a positively or negatively charged amino acid sequence, a cysteine-containing amino acid sequence, avidin, streptavidin, or a fragment of Staphylococcus protein A.

The effector proteins and amino acid sequences described above may be present in a proform which itself is either active or not and which may be removed, when, e.g., entering a certain cellular environment.

In a most preferred embodiment of the invention, said receptor Is a costimulatory surface molecule important for T-cell activation or comprises an epitope binding site or a hormone binding site.

In a further most preferred embodiment of the invention, said costimulatory surface molecule is CD80 (B7-1) or CD86 (B7-2).

Advantageously, said domains including the V_{H} and/or V_{L} domains are connected by a flexible linker, preferably by a polypeptide linker disposed between said domains, wherein said polypeptide linker comprises plural, hydrophilic, peptide-bonded amino acids of a length sufficient to span the distance between the C-terminal end of one of said domains and the N-terminal end of the other of said domains when said polypeptide assumes a conformation suitable for binding when disposed in aqueous solution.

Polynucleotides which upon expression encode the above-described antigens, epitopes or polypeptides are also described herein. Said polynucleotides may be fused to suitable expression control sequences known in the art to ensure proper transcription and translation of the polypeptide. Furthermore, the polynucleotides may be comprised in a vector which further comprises a selectable marker; see also supra.

Also described is a cell containing the polynucleotide or vector described above, said cell may be a mammalian cell if therapeutic uses of the polypeptide are envisaged. Of course, yeast and bacterial cells may serve as well, in particular if the produced antigen, epitope or polypeptide is used as a diagnostic means; see also supra.

Also described herein is a process for the preparation of an antigen or polypeptide described above comprising cultivating a cell under conditions suitable for the expression of the antigen or polypeptide and isolating the antigen or polypeptide from the cell or the culture; see also supra.

The present invention allows the recombinant production of polypeptides comprising a binding site having affinity and specificity for an epitope and antigen of the DCs of the invention or comprising said antigen or epitope. As is evident from the foregoing, the invention provides a large family of polypeptides comprising such binding site domains or antigens or epitopes for any use in therapeutic and diagnostic approaches. It will be apparent to those skilled in the art that the binding site domains and antigens or epitopes can be further coupled to other moieties as described above for, e.g., drug targeting and imaging applications. Such coupling may be conducted chemically after expression of the polypeptides to site of attachment or the coupling product may be engineered into the polypeptide of the invention at the DNA level. The DNAs are then expressed in a suitable host system, and the expressed proteins are collected and renatured, if necessary. As described above, the binding site domain is preferably derived from the variable region of antibodies, preferably monoclonal antibodies of the invention. In this respect, hybridoma technology enables production of cell lines secreting antibody to essentially any desired substance that produces an immune response. RNA encoding the light and heavy chains of the immunoglobulin can then be obtained from the cytoplasm of the hybridoma. The 5' end portion of the mRNA can be used to prepare cDNA to be used in the method of the present invention. The DNA encoding the polypeptides of the invention can then be expressed in cells, preferably mammalian cells.

Depending on the host cell, renaturation techniques may be required to attain proper conformation. If necessary, point substitutions seeking to optimize binding may be made in the DNA using conventional cassette mutagenesis or other protein engineering methodology such as is disclosed herein. Preparation of the polypeptides of the invention may be dependent on knowledge of the amino acid sequence (or corresponding DNA or RNA sequence) of bioactive proteins such as enzymes, toxins, growth factors, cell differentiation factors, receptors, antimetabolites, hormones or various cytokines or lymphokines. Such sequences are reported in the literature and available through computerized data banks. For example, a polypeptide of the invention can be constructed that, e.g., consists of the single-chain Fv fragment of the antibody of the invention, preferably M-DC8 and the extracellular part of the human costimulatory protein CD80 (B7-1) connected by a peptide linker. The CD80 costimulatory protein belongs to the Ig superfamily. It is a heavily glycosylated protein of 262 amino acids. A more detailed description was published by Freeman G.J et.al. J.Immunol.143, (1989) 2714 - 2722. Stable expression can be performed in, e.g., DHFR deficient CHO-cells as described by Kaufmann R.J. (1990) Methods Enzymol. 185, 537-566. The protein can then be purified via its His-tag attached to the C-terminus by using a Ni-NTA-column (Mack et.al., Proc. Natl. Acad. Sci. U.S.A. 92 (1995)7021-7025). To analyse the binding properties different ELISA assay can be performed. For example, binding to the 17-1A-antigen can be analysed using soluble 17-1A-antigen obtained as described (Mack, Proc. Natl. Acad. Sci. U.S.A. 92 (1995) 7021-7025) by stable expression in CHO-cells of the DNA encoding the first 264 amino acids of the 17-1A antigen also known as GA 733-2 (Szala, Proc. Natl. Acad. Sci. U.S.A. 87 (1990) 3542-3546)

In another embodiment, the present invention relates to a method for isolating or identifying DCs as defined above from peripheral blood, comprising the steps of
(a) contacting a sample of peripheral blood with the antibody of the invention as deposited under DSM ACC 2241, DSM ACC 2399 or DSM ACC 2398;
(b) detecting the presence of antibody/DC complexes; and/or
(c) recovering DCs which have bound to said antibody or functional fragment thereof.

DCs as the most efficient antigen presenting cells reveal a considerable phenotypic and functional heterogeneity that is deemed to reflect different stages of their maturation^{1,2,3}. According to a recently proposed model, early DCs are found almost ubiquitously at various sites of the vertebrate organism where they efficiently take up and process exogenous antigens for presentation by MHC molecules on their plasma membrane^{4,5}. Subsequently, when antigen uptake is completed DC undergo further differentiation expressing a different pattern of surface molecules and becoming migratory. At this stage, when expression of MHC and costimulatory molecules is highly increased DC are able not only to prime naive T lymphocytes but also direct their programme of cytokine expression versus the Th1 or Th2 type^{6,7}. Their prominent capacity to prime T lymphocytes makes DC particularly attractive for the development of immunomodulatory strategies. In particular, the use of DC has been proposed for therapeutic ex vivo priming of cytolytic T cells against defined tumor peptides or viral antigens⁸. Similarly, DC may serve as tools for reprogramming T cells in order to combat autoimmune or atopic diseases. A major obstacle for the development of such therapeutic strategies until the present invention was the extremely low frequency of DC in blood. The few DC-specific markers reported so far are not suitable for isolation of blood DC. CD83 is induced only during in vitro culture⁹ and the actin bundling protein p55 is confined to the cytoplasm¹⁰. Current isolation protocols rely on the consecutive depletion of cell populations with the help of lineage-specific mAbs combined with density gradient centrifugation or differential adherence^{2,3,11}. At present, the most widely used yet laborious method of enrichment is the in vitro culture of CD34⁺ cells to induce development and differentiation of DC from such - also rare - progenitor cells over a period of several days ^{12,13,14}.

The provision of the novel antibodies in accordance with the present invention, such as exemplified by the M-DC8 monoclonal antibody now allows for the purification of a novel unique population of human DC with a characteristic surface phenotype and typical functional activities. This population of DC displaying an extraordinary homogeneity stands out for and also promotes differentiation of purified CD8 T cells into alloantigen-specific cytolytic cells.

The isolated DCs also called M-DC8+ cells comprise a substantial fraction of circulating DC and show phenotypic and functional characteristics of immature DC. Upon culture, M-DC8+ cells differentiate into mature DC evidenced by their prominent capacity to stimulate T cells. Thus, the antibodies of the present invention can serve as a suitable tool for detection and direct isolation of human DC. A variety of uses for the isolated M-DC8+ cells is encompassed by the invention described herein, including but not limited to, the use of M-DC8+ cells as APC, the activation and expansion of antigen-specific T cells in vitro for use in adoptive cellular immunotherapy, the in vivo administration of antigen-pulsed DC as vaccines, and the identification of antigenic epitopes for vaccine development Therefore M-DC8+ cells may be ideal candidates for the use in immunotherapy against infectious diseases and cancer as well as for the treatment of autoimmune diseases. In this connection the antibody of the present invention can be used for the in vivo recruitment of target cells to DC by the generation of bispecific antibodies directed to DC cells and antigens on target cells, see supra.

Described herein are also dendritic cells as defined above, recognized by the antibody of the invention and/or containing an antigen or epitope of the invention or obtainable by the method described above. The human dendritic cells isolated according to the method of the present invention can be used, e.g., to stimulate T cells and to present antigens for the induction of antigen-specific T cell-mediated immune responses. The isolated population of human dendritic cells described herein can also be used for a wide range of applications, including but not limited to, activation and expansion of antigen-specific T cells for use in adoptive immunotherapy against cancer and infectious diseases. Furthermore, they can be pulsed with an antigen and thus be used as vaccines and/or immunotherapeutics and identification of new antigenic targets for immunotherapy.

It is also evident to the person skilled in the art, that the DCs described herein can be further modified, for example, so as to express a recombinant nucleic acid molecule. For example, DCs described herein can be transfected with genes for cytokines or signaling molecules to modulate or program immune response in vitro or In vivo. For example, secreted IL-12 when present In the microenvironment of T cell stimulation directs differentiation of T cells into Th1 type T helper cells whereas IL-4 programmes T cells for Th2 type T helper cells Seder & Paul (Ann. Rev. Immunol. 12 (1994), 635-673). Said modification can be performed according to methods known in the art, see also supra. Standard methods for transfecting cells with recombinant DNA are well known to those skilled in the art of molecular biology, see, e.g., WO 94/29469. Furthermore, gene therapy may be carried out by directly administering the recombinant DNA molecule or vector of the invention to a patient or by transfecting cells such as DCs with the polynucleotide or vector ex vivo and infusing the transfected cells into the patient. Furthermore, research pertaining to gene transfer into cells of the germ line is one of the fastest growing fields in reproductive biology. Gene therapy, which is based on Introducing therapeutic genes into cells by ex-vivo or in-vivo techniques is one of the most Important applications of gene transfer. Suitable vectors and methods for in-vitro or in-vivo gene therapy are described in the literature and are known to the person skilled in the art as described herein above. The polynucleotides and vectors may be designed for direct introduction or for introduction via liposomes, or viral vectors (e.g. adenoviral, retroviral) containing said recombinant DNA molecule into the cell. Preferably, said cell is a germ line cell, embryonic cell, stem cell or egg cell or derived therefrom. The pharmaceutical compositions according to the invention can also be used for the treatment of diseases hitherto unknown as being mediated by DCs. An embryonic cell can be for example an embryonic stem cell as described in, e.g., Nagy, Proc. Natl. Acad. Sci. USA 90 (1993) 8424-8428.

It is to be understood that the introduced polynucleotides and vectors express the gene product after introduction into said cell and preferably remain in this status during the lifetime of said cell. For example, cell lines which stably express the polynucleotide under the control of appropriate regulatory sequences may be engineered according to methods well known to those skilled in the art. Rather than using expression vectors which contain viral origins of replication, host cells can be transformed with the polynucleotide or vector of the invention and a selectable marker, either on the same or separate vectors. Following the introduction of foreign DNA, engineered cells may be allowed to grow for 1-2 days in an enriched media, and then are switched to a selective media. The selectable marker in the recombinant plasmid confers resistance to the selection and allows for the selection of cells having stably integrated the plasmid into their chromosomes and grow to form foci which in turn can be cloned and expanded into cell lines. Such engineered cell lines are also particularly useful in screening methods described below.

A number of selection systems may be used, including but not limited to the herpes simplex virus thymidine kinase (Wigler, Cell 11(1977), 223), hypoxanthineguanine phosphoribosyltransferase (Szybaiska, Proc. Natl. Acad. Sci. USA 48 (1962), 2026), and adenine phosphoribosyltransferase (Lowy, Cell 22 (1980), 817) in tk, hgprt or aprt cells, respectively. Also, antimetabolite resistance can be used as the basis of selection for dhfr, which confers resistance to methotrexate (Wigler, Proc. Natl. Acad. Sci. USA 77 (1980), 3567; O'Hare, Proc. Natl. Acad. Sci. USA 78 (1981). 1527), gpt, which confers resistance to mycophenolic acid (Mulligan, Proc. Natl. Acad. Sci. USA 78 (1981), 2072); neo, which confers resistance to the aminoglycoside G-418 (Colberre-Garapin, J. Mol. Biol. 150 (1981), 1); hygro, which confers resistance to hygromycin (Santerre, Gene 30 (1984), 147); or puromycin (pat, puromycin N-acetyl transferase). Additional selectable genes have been described, for example, trpB, which allows cells to utilize indole in place of tryptophan; hisD, which allows cells to utilize histinol in place of histidine (Hartman, Proc. Natl. Acad. Sci. USA 85 (1988). 8047); and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.).

Despite their resemblance to immature DC M-DC8⁺ cells, they are very potent in T cell stimulation. This activity may be reflected by the increased MHC class II expression and the raised exhibition of costimulatory molecules on M-DC8⁺ cells after in vitro culture. Coculturing with allogeneic T cells appeared to be particularly potent to upregulate these molecules on M-DC8⁺ cells. Various methods for using DCs to activate T-cells have been described; see, e.g., WO94/02156.

Thus, also described herein is a method for preparing activated antigen-specific human T-cells in vitro comprising co-culturing T-cells with the dendritic cells of the invention, exposed to an antigen or expressing an antigen to activate the T-cells to proliferate or to become cytotoxic in response to the antigen. There are various methods which can be used to have antigenic molecules presented by DC for stimulation of T cells. These methods are known to those skilled in the art When the allelic composition of MHC class I or class II genes of an individual is known extraneous antigenic peptides can be selected according to known algorithms and directly bound to MHC molecules on the surface of DC by incubating DC with the isolated peptides. Alternatively, DC can be exposed to intact antigenic protein molecules. These are endocytosed, enzymatically cleaved within endosomes, bound into the binding grove of MHC class II molecules and transported as complexes with these molecules to the cell membrane for presentation to CD4⁺ T cells. Part of the endocytosed proteins can reach the cytosolic compartment where they are cleaved by the proteasomal machinery ("cross priming"). The generated peptides are transported via the TAP-molecules into the lumen of the endoplasmatic reticulum where they are bound to nascent MHC class I molecules and consecutively transported to the cell surface to be presented to cytotoxic CD8⁺ T cells or their precursors. Antigenic peptides derived from an external protein can efficiently be presented by MHC class I molecules on DC when the protein is synthesized within DC after transduction of the coding gene in an expression vector; see also supra. Hence, such In vitro educated and/or primed T cells can be used in clinical trials to combat serious viral infections such as described for CMV in immune-compromised patients (Walter et al., New Engl. J. Med. 333 (1995), 1038-1044) or to induce immune rejection of tumor metastases (Nestle et al., Nature Med. 4 (1998), 328-332). Thus, the fast and facile procurement of DCs according to the invention provides for further development of adoptive immunotherapeutic strategies for various indications including cancer.

Furthermore, also described herein is a method for identifying an antigen recognizable by T-cells comprising
(a) co-culturing T-cells with the dendritic cells described above, exposed to said antigen, and
(b) measuring T-cell proliferation, T-cell cytotoxicity or T-cell lymphokine production.

Preferably, said T-cells In the above described methods are CD4⁺ or CD8⁺ cells. For example, for identifying an antigen recognized by CD4⁺ T cells DC are incubated with the antigenic protein in question or with a mixture of different antigenic proteins or with overlapping peptides of 15 to 25 amino acid length synthesized according to the amino acid sequence of the antigenic protein in question or by peptides obtained from a peptide library. The T cell response is determined after either 3, 5 or 7 days by measuring the incorporation of [³H] thymidine or by assaying secreted cytokines such as interferon-gamma by ELISA or by assessing intracellular cytokines by FACS analysis after staining with specific monoclonal antibodies labeled with a fluorescent dye.

For identifying an antigen recognized by CD8⁺ T cells DC are transfected with the gene coding for the antigenic protein or for fragments thereof in an expression vector or incubated with overlapping peptides of 9 to 10 amino acids length or with peptides generated in form of a peptide library. The CD8⁺ T cell response can be determined by measuring secreted cytokines such as tumor necrosis factor-alpha or interferon-gamrna. Cytotoxicity can be evaluated by lysis of ⁵¹Cr-labeled target cells expressing the respective antigenic peptides bound to MHC class I molecules on their surface.

Furthermore, a method is described for identifying T-cell activating or costimulating compounds comprising
(a) culturing the dendritic cells described above and T-cells in the presence of a component capable of providing a detectable signal in response to T-cell activation with a compound to be screened under conditions to permit interaction of the compound with the cells, and
(b) detecting the presence of a signal generated from the activation of the T-cells.

In a further embodiment, the present invention relates to a method for identifying compounds which suppress T-cell activation or stimulation comprising
(a) contacting T-cells and dendritic cells of the invention in the presence of a component capable of providing a detectable signal in response to the activation of said T-cells by a T-cell activator with a compound to be screened under conditions to permit activation of the T-cell, and
(b) detecting the presence or absence of the signal generated from the interaction of the activator with the T-cells.

The detection of the signal generated by the T-cells can be performed according to methods known in the art such as described above or in the appended examples that can be easily adapted to the above described methods.

Preferably, in this method said dendritic cells are exposed to an antigen by incubation in culture media.

The term "compound" in a method of the invention includes a single substance or a plurality of substances which may or may not be identical.

Said compound(s) may be comprised in, for example, samples, e.g., cell extracts from, e.g., plants, animals or microorganisms. Furthermore, said compounds may be known in the art but hitherto not known to be capable of inhibiting T-cell activation or not known to be useful as a T-cell co-stimulatory factor, respectively. The plurality of compounds may be, e.g., added to the culture medium or injected into the cell.

If a sample containing (a) compound(s) is identified in the methods described herein, then it is either possible to isolate the compound from the original sample identified as containing the compound, in question or one can further subdivide the original sample, for example, if it consists of a plurality of different compounds, so as to reduce the number of different substances per sample and repeat the method with the subdivisions of the original sample. It can then be determined whether said sample or compound displays the desired properties, for example, by the methods described herein, the appended examples or in the literature. Depending on the complexity of the samples, the steps described above can be performed several times, preferably until the sample identified according to the method of the invention only comprises a limited number of or only one substance(s). Preferably said sample comprises substances of similar chemical and/or physical properties, and most preferably said substances are identical. The methods of the present invention can be easily performed and designed by the person skilled in the art, for example in accordance with other cell based assays described in the prior art or by using and modifying the methods as described In the appended examples. Furthermore, the person skilled In the art will readily recognize which further compounds and/or cells may be used in order to perform the methods of the invention, for example, interleukins, or enzymes, if necessary, that convert a certain compound into the precursor which in turn stimulates or suppresses T-cell activation. Such adaptation of the method of the invention is well within the skill of the person skilled in the art and can be performed without undue experimentation.

It is envisaged in the method as provided above that said compound or antigen is a tumor antigen, a viral antigen, a microbial antigen, an allergen, an auto-antigen, a virus, a microorganism, a polypeptide, a peptide or a plurality of tumor cells.

Compounds and antigens which can be used in accordance with the description above include peptides, proteins, nucleic acids, antibodies, small organic compounds, ligands, peptidomimetics, PNAs and the like. Said compounds can also be functional derivatives or analogues of known T-cell activators or inhibitors. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described, for example, in the appended examples. Furthermore, peptidomimetics and/or computer aided design of appropriate activators or inhibitors of T-cell activation can be used, for example, according to the methods described herein. Appropriate computer programs can be used for the identification of interactive sites of a putative inhibitor and the antigen of the invention by computer assistant searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known T-cell activators or inhibitors. Appropriate peptidomimetics can also be identified by the synthesis of peptidomimetic combinatorial libraries through successive chemical modification and testing the resulting compounds, e.g., according to the methods described herein and in the appended examples. Methods for the generation and use of peptidomimetic combinatorial libraries are described in the prior art, for example in Ostresh, Methods in Enzymology 267 (1996), 220-234 and Domer, Bioorg. Med. Chem. 4 (1996), 709-715. Furthermore, the three-dimensional and/or crystallographic structure of inhibitors or activators of DC/T-cell interaction can be used for the design of peptidomimetic inhibitors or activators of T-cell activation, e.g., in combination with the antibody or antigen of the invention (Rose, Biochemistry 35 (1996), 12933-12944; Rutenber, Bioorg. Med. Chem. 4 (1996), 1545-1558).

In summary, also described are methods for identifying compounds which are capable of modulating DC and T-cell mediated immune responses.

Compounds found to activate T-cell mediated responses may be used in the treatment of cancer, for example the treatment of epithelial cancers, like carcinomas of the breast, prostate, gastro intestinal tract, kidney, lung, skin and/or other sites and the treatment of related diseases, like cell proliferative disorders. In addition, it may also be possible to specifically inhibit and/or protect from viral diseases, thereby preventing viral infection or viral spread. Compounds identified as suppressors of T-cell activation or stimulation may be used In organ transplantation in order to avoid graft rejection; see also supra.

The compounds identified or obtained according to the method described herein are thus expected to be very useful in diagnostic and in particular for therapeutic applications. Hence, in a further embodiment the invention relates to a method for the production of a pharmaceutical composition comprising formulating the compound identified in step (b) of the above described methods in a pharmaceutically acceptable form.

The therapeutically useful compounds identified according to the method of the invention may be administered to a patient by any appropriate method for the particular compound, e.g., orally, intravenously, parenterally, transdermally, transmucosally, or by surgery or implantation (e.g., with the compound being in the form of a solid or semi-solid biologically compatible and resorbable matrix) at or near the site where the effect of the compound is desired. Therapeutic doses are determined to be appropriate by one skilled in the art, see supra.

Moreover, the present invention relates to kits and compositions comprising the antibody of the invention, the aforementioned polypeptide, the polynucleotide or the vector of the invention. Preferably said composition is a pharmaceutical composition.

The pharmaceutical composition of the present invention may further comprise a pharmaceutical acceptable carrier. Examples of suitable pharmaceutical carriers are well known in the art and Include phosphate buffered saline solutions, water, emulsions, such as oil/water emulsions, various types of wetting agents, sterile solutions etc. Compositions comprising such carriers can be formulated by well known conventional methods. These pharmaceutical compositions can be administered to the subject at a suitable dose. Administration of the suitable compositions may be effected by different ways, e.g., by intravenous, intraperitoneal, subcutaneous, intramuscular, topical or intradermal administration. The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Generally, the regimen as a regular administration of the pharmaceutical composition should be in the range of 1 µg to 10 mg units per day. If the regimen is a continuous infusion, it should also be in the range of 1 µg to 10 mg units per kilogram of body weight per minute, respectively. Progress can be monitored by periodic assessment Dosages will vary but a preferred dosage for intravenous administration of DNA is from approximately 10⁶ to 10¹² copies of the DNA molecule. The compositions of the invention may be administered locally or systemically. Administration will generally be parenterally, e.g., intravenously; DNA may also be administered directly to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringers dextrose, dextrose and sodium chloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

It is envisaged by the present invention that the various polynucleotides and vectors of the invention are administered either alone or in any combination using standard vectors and/or gene delivery systems, and optionally together with an appropriate compound, for example an (auto)antigen specific for tumors, allergies, or autoimmune diseases and/or together with a pharmaceutically acceptable carrier or excipient. Subsequent to administration, said polynucleotides or vectors may be stably integrated into the genome of the subject. On the other hand, viral vectors may be used which are specific for certain cells or tissues, preferably for DCs and persist in said cells. Suitable pharmaceutical carriers and excipients are well known in the art. The pharmaceutical compositions prepared according to the invention can be used for the prevention or treatment or delaying of different kinds of diseases, which are related to immunodeficiencies or viral infection or cancer. Furthermore, said pharmaceutical composition may be a vaccine.

Vaccines may be prepared, inter alia, from one or more antibodies, fragments of said antibodies, derivatives of said antibodies, polynucleotides or antigens of the invention.

For example, polynucleotides of the invention may be used for gene vaccination or as DNA vaccines. Routes for administration of gene/DNA vaccines are well known in the art and DNA vaccination has been successfully used to elicit alloimmune, anti-tumor and antiidiotype immune responses (Tighe M. et al., Immunology Today 19 (1998), 89-97). Moreover, inoculation with nucleic acid molecules/DNA has been found to be protective in different modes of disease (Fynan, Proc. Natl. Acad. Sci. U.S.A. 90 (1993), 11478-11482; Boyer, Nat. Med. 3 (1997), 526-532; Webster, Vaccine 12 (1994), 1495-1498; Montgomery et al., DNA Cell Biol. 12 (1993), 777-783; Barry, Nature 311 (1995), 632-635; Xu and Liew, Immunology 84 (1995), 173-176; Zhoug, Eur. J. Immunol. 26 (1996), 2749-2757; Luke, J. Inf. Dis. 175 (1997), 91-97; Mor, Biochem. Pharmacology 55 (1998), 1151-1153; Donelly, Annu. Rev. Immun. 15 (1997), 617-648; MacGregor, J. Infect. Dis. 178 (1998), 92-100).

The antibodies, fragments or derivatives of said antibodies or polynucleotides of the invention used in a pharmaceutical composition as a vaccine may be formulated e.g. as neutral or salt forms. Pharmaceutically acceptable salts, such as acid addition salts, and others, are known in the art. Vaccines can be, inter alia, used for the treatment and/or the prevention of an infection with pathogens, like viruses, and are administered in dosages compatible with the method of formulation, and in such amounts that will be pharmacologically effective for prophylactic or therapeutic treatments.

Proteins, protein fragments and/or protein derivatives used as vaccines are well known in the art (see, e.g. Cryz, "Immunotherapy and Vaccines", VCH Weinheim (1991); Paul (1989), loc. cit.). Furthermore, it has been shown that even intracellular enzymes of bacterial pathogens can act as antigenic entities which provide immunological protection (Michetti, Gastroenterology 107 (1994), 1002; Radcliff, Infect. Immun. 65 (1997), 4668; Lowrie, Springer Semin. Immunopathol. 19 (1997), 161)

A vaccination protocol can comprise active or passive immunization, whereby active immunization entails the administration of an antigen or antigens using the antibodies (and/or derivatives or fragments thereof) of the present invention to the host/patient In an attempt to elicit a protective immune response. Principles and practice of vaccination and vaccines are known to the skilled artisan, see, for example, in Paul, "Fundamental Immunology" Raven Press, New York (1989) or Morein, "Concepts in Vaccine Development", ed: S.H.E. Kaufmann, Waiter de Gruyter, Berlin, New York (1996), 243-264. Typically, vaccines are prepared as injectables, either as liquid solutions or suspensions; solid forms suitable for solution In or suspension in liquid prior to injection also may be prepared. The preparation may be emulsified or the protein may be encapsulated in liposomes. The active immunogenic ingredients often are mixed with pharmacologically acceptable excipients which are compatible with the active ingredient. Suitable excipients include but are not limited to water, saline, dextrose, glycerol, ethanol and the like; combinations of these excipients in various amounts also may be used. The vaccine also may contain small amounts of auxiliary substances such as wetting or emulsifying reagents, pH buffering agents, and/or adjuvants which enhance the effectiveness of the vaccine. For example, such adjuvants can include aluminum compositions, like aluminumhydroxide, aluminumphosphate or aluminumphosphohydroxide (as used in "Gen H-B-Vax®" or "DPT-Impfstoff Behring"), N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1'2'-dipalmitoyl-sn-glycero-3-hydroxphaosphoryloxy)-ethylamine (CGP 19835A, also referred to as MTP-PE), MF59 and RIBI (MPL + TDM + CWS) in a 2% squalene/Tween-80® emulsion. Further adjuvants may comprise DNA or oligonucleotides, like, inter alia, CpG-containing motifs (CpG-oligonucleotides; Krieg, Nature 374 (1995), 546-549; Pisetsky, An. Internal. Med. 126 (1997), 169-171).

The vaccines usually are administered by intravenous or intramuscular injection. Additional formulations which are suitable for other modes of administration include suppositories and, in some cases, oral formulations. For suppositories, traditional binders and carriers may include but are not limited to polyalkylene glycols or triglycerides. Oral formulation include such normally employed excipients as, for example, pharmaceutical grades of mannitol, lactose, starch, magnesium stearate, sodium saccharine, cellulose, magnesium carbonate and the like. These compositions may take the form of solutions, suspensions, tables, pills, capsules, sustained release formulations or powders and contain about 10% to about 95% of active ingredient, preferably about 25% to about 70%.

Vaccines are administered in a way compatible with the dosage formulation, and in such amounts as will be prophylactically and/or therapeutically effective. The quantity to be adminstered generally is in the range of about 5 micrograms to about 250 micrograms of antigen per dose, and depends upon the subject to be dosed, the capacity of the subject's immune system to synthesize antibodies, and the degree of protection sought. Precise amounts of active ingredient required to be administered also may depend upon the judgment of the practitioner and may be unique to each subject. The vaccine may be given in a single or multiple dose schedule. A multiple dose Is one in which a primary course of vaccination may be with one to ten separate doses, followed by other doses given at subsequent time intervals required to maintain and/or to reinforce the immune response, for example, at one to four months for a second dose, and if required by the individual, a subsequent dose(s) after several months. The dosage regimen also will be determined, at least in part, by the need of the individual, and be dependent upon the practitioner's judgment.

Within the scope of the present invention are also vaccines based on cells or cellular fragments. For example, a dendric cell (DC) based vaccine can be produced by selection of M-DC8⁺ cells and ex vivo-pulsing with antigens which are specific for tumor cells or virus-infected cells. It is envisaged that DCs which can be isolated with the antibodies of the invention are employed as antigen carriers for, inter alia, tumor vaccination or anti-virus vaccinations. The antigen loading of DCs is well known in the art and may range from minimal MHC class I restricted peptides to proteins (Mayordomo, Nature Med. 1 (1995), 1297-1302; Hsu, Nature Med. 2 (1996), 52-58; Paglia, J. Exp. Med. 183 (1996), 317-322; reviewed in Pardoll, Nature Medicine Vaccine Suppl. 4(5) (1998), 525-531). Furthermore, antigen may be presented/loaded by fusion of DCs with whole tumor cells (Gong, Nature Med. (1996), 558-561) or employing replication-defective recombinant viral vectors (Specht, J. Exp. Med. 186 (1997), 1213-1221; Song, J. Exp. Med. 186 (1997), 1247-1256).

Additionally, it is possible to use a pharmaceutical composition of the invention which comprises polynucleotide or vector of the invention in gene therapy. Suitable gene delivery systems may include liposomes, receptor-mediated delivery systems, naked DNA, and viral vectors such as herpes viruses, retroviruses, adenoviruses, and adeno-associated viruses, among others. Delivery of nucleic acids to a specific site in the body for gene therapy may also be accomplished using a biolistic delivery system, such as that described by Williams (Proc. Natl. Acad. Sci. USA 88 (1991), 2726-2729).

Furthermore, transgenic non-human animals that comprise a polynucleotide or vector of the invention are within the scope of the present invention. Such transgenic non-human animals can be generated according to methods well known in the art and may useful in studying the biological activity of the antibody, antigens and polypeptides of the invention and/or corresponding inhibitors and activators of DC mediated immune response.

Also described herein is a method for the production of a transgenic animal, preferably transgenic mouse, comprising introduction of a polynucleotide or vector of the invention into a germ cell, an embryonic cell, stem cell or an egg or a cell derived therefrom. The non-human animal to be used in the method of the invention may be a non-transgenic healthy animal, or may have a viral disease or cancer, or a DC or T-cell mediated autoimmune disease. Production of transgenic embryos and screening of those can be performed, e.g., as described by A. L. Joyner Ed., Gene Targeting, A Practical Approach (1993), Oxford University Press. The DNA of the embryonal membranes of embryos can be analyzed using Southern blots with an appropriate probe.

The transgenic non-human animals may be transgenic mouse, rats, hamsters, dogs, monkeys, rabbits or pigs comprising a polyncleotide or vector of the invention or obtained by the method described above, preferably wherein said polynucleotide or vector is stably integrated into the genome of said non-human animal, preferably such that the presence of said polynucleotide or vector leads to the expression of the antibody of the invention. On the other hand, knock out non-human animals may be generated that are no longer capable of expressing the antigen of the invention.

In another embodiment the present invention relates to a diagnostic composition comprising any one of the above described the antibodies, polypeptides, polynucleotides or vectors of the invention and optionally suitable means for detection.

The antigens described herein are particularly suited for use in immunoassays in which they can be utilized in liquid phase or bound to a solid phase carrier. In addition, the antigens used in these assays can be detectably labeled in various ways. Examples of immunoassays which can utilize the antigen of the invention are competitive and non-competitive immunoassays in either a direct or indirect format. Examples of such immunoassays are the radioimmunoassay (RIA), the sandwich (immunometric assay) and the Western blot assay. Detection of antibodies which bind to the antigen or epitope as described herein can be done utilizing immunoassays which run in either the forward, reverse, or simultaneous modes, including immunohistochemical assays on physiological samples. The antigen and polypeptides described herein can be bound to many different carriers and used to detect the presence of antibody specifically reactive with the DCs as described above. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble.

There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds; see also supra.

The materials for use in the assay of the invention are ideally suited for the preparation of a kit. Such a kit may comprise a carrier means being compartmentalized to receive in close confinement one or more container means such as vials, tubes and the like, each of the container means comprising one of the separate elements to be used in the method. For example, one of the container means may comprise an antigen of the invention bound to a carrier. A second container may comprise soluble, detectably-labeled second antibody, in lyophilized form or in solution. In addition, the carrier means may also contain a plurality of containers each of which comprises different, predetermined amounts of the antigen of the invention. These latter containers can then be used to prepare a standard curve into which can be interpolated the results obtained from the sample containing the unknown amount of antibodies to the antigen of the present invention.

Furthermore, a vaccine is described which comprises the antigen exposed dendritic cells or antigen expressing DCs or the antigen or epitope described above. Isolated DCs can be prepared in vitro to present antigenic peptides for stimulation of either CD4⁺ or CD8⁺ T cells as described below. They are then injected either intradermally, subcutaneously, intramuscularly or, particularly in case of anti-tumor vaccination, into areas of tumor growth or into lymphatic vessels or lymph nodes draining areas of tumor growth. Injections of antigen-presenting DC is repeated several times at various intervals (Nestle, Nature Med. 4 (1998) 328-332).

Also described is an immunopotentiating composition comprising the dendritic cells of the invention and at least one antigen as defined above capable of generating a protective immunological response to a disease in a human or an animal susceptible to such disease. Immunogenicity of DCs used for vaccination can be enhanced and specifically modified for programming either a Th1- or a Th2-directed immune response, e.g., by transducing cDNA coding for secreted cytokines or chemokines or membrane molecules into DCs. For programming a Th1-directed immune response antigen-presenting DCs are transduced with cDNA coding for IL-12, For programming a Th2-directed immune response they are modified to secrete IL-4.

Also described is herein the use of the T-cells obtainable by a method described above for the preparation of a pharmaceutical composition for adoptive immunotherapy and to the use of the dendritic cells of the invention exposed to an antigen for the preparation of a pharmaceutical composition for activating T-cells in a human or an animal. For example, T lymphocytes are cocultured with autologous DCs of the invention prepared to present antigenic peptides to T cells (see above) for several days in vitro to induce antigen-specific activation of T cells. Antigens may be tumor antigens or allergens or autoantigens. Proliferation of activated T cells might be supported by addition of cytokines such as IL-2. Antigen-specific stimulation of the T cells is repeated several times. The T cells are then adoptively transferred to the donor mostly by i.v. injection.

Furthermore disclosed herein is the use of the bispecific antibody described above for the preparation of a pharmaceutical composition for recruiting target cells with said dendritic cells. Preferably, said target cells are tumor cells or virus-Infected cells or cells infected with a microorganism; see also supra.

The dosage ranges for the administration of the antibodies of the invention or antigens and epitopes as described herein are those large enough to produce the desired effect in which the symptoms or cellular destruction of the autoimmune response are ameliorated. The dosage should not be so large as to cause adverse side effects, such as unwanted cross-reactions, anaphylactic reactions, and the like. Generally, the dosage will vary with the age, condition, sex, and extent of the disease In the patient and can be determined by one of skill in the art The dosage can be adjusted by the individual physician in the event of any counterindications.

Furthermore, a method for the modification of dendritic cells is disclosed herein, said method comprising transfection of genes for cytokines or signaling molecules to modulate or program immune response in vitro or in vivo. For example of such cytokines and molecules see supra.

Furthermore, a method for identifying molecules synthesized by DCs having enhancing, modulating or suppressing effect on the antigen-specific activation of T cells is described herein, said method comprising
(a) separating of molecules secreted by DCs described above into the culture supernatant using, e.g., conventional biochemical methods and testing the enriched or isolated molecules for antigen-specific T cell activation in a cell culture system lacking DCs; and/or
(b) comparing gene expression in DCs described above with that in other antigen-presenting cells such as monocytes by subtractive cloning or differential display RT-PCR.

In addition, also described is a method of propagating DC in vitro comprising
(a) culturing DCs described above in a specific cytokine cocktail supporting growth and proliferation of DCs in vitro; and/or
(b) immortalizing said DCs by transduction of transforming genes such as the gene coding for the SV40 large T antigen.

For example, total RNA can be isolated from the DCs of the invention and other antigen-presenting cells using, e.g., the RNAzol B method (Tel-Test, (Inc.) and differential display can be performed as described in the art; see e.g., Kojima, J. Biol. Chem. 271 (1996), 12327-12332.

These and other embodiments are disclosed and encompassed by the description and examples of the present invention. Further literature concerning any one of the methods, uses and compounds to be employed in accordance with the present invention may be retrieved from public libraries and databases, using for example electronic devices. For example the public database "Medline" may be utilized which is available on the Internet, for example under http://www.ncbi.nim.nih.gov/PubMed/medtine.html. Further databases and addresses, such as http://www.ncbi.nlm.nih.gov/, http://www.infobiogen.fr/, http://www.fmi.ch/biotogy/research_toots.htmt, http://www.Dgr.org/, are known to the person skilled in the art and can also be obtained using, e.g., http://www.tycos.com. An overview of patent information in biotechnology and a survey of relevant sources of patent information useful for retrospective searching and for current awareness is given in Berks, TIBTECH 12 (1994), 352-364.

The pharmaceutical compositions, uses and methods of the invention can be used advantageously for the treatment of all kinds of diseases known or hitherto unknown as being related to autoimmune diseases, hypersensitive diseases such as allergic diseases, immunodeficiencies or dependent on viral diseases or cancer or any other infectious disease that involves responses of the immune system. The pharmaceutical compositions, methods and uses of the present invention may be desirably employed in humans, although animal treatment is also encompassed by the methods and uses described herein.

**The figures show:**

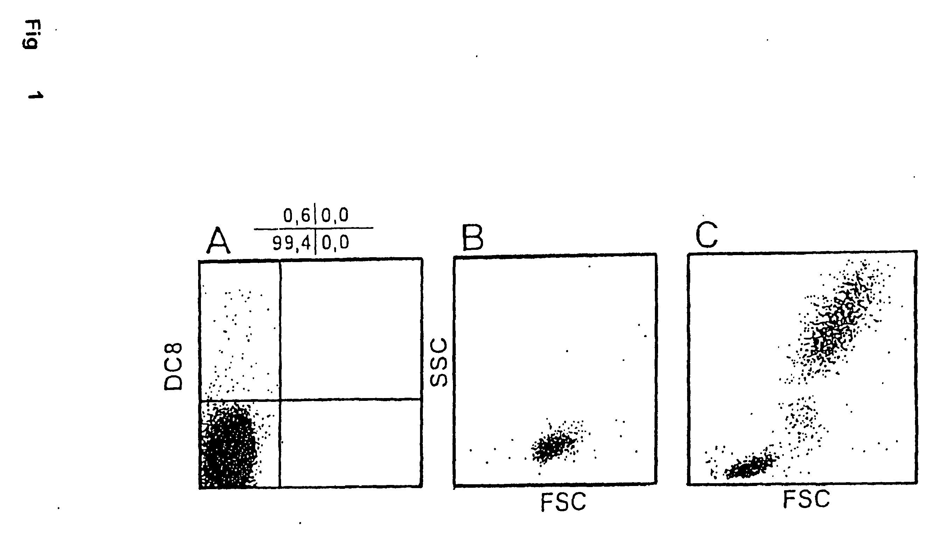

- **Figure 1**: Frequency, size and granularity of M-DC8⁺ cells.
Flow cytometric analysis of human peripheral blood cells after lysis of erythrocytes and staining with the mAb M-DC8 followed by phycoerythrin-conjugated anti-Ig antibodies (A). The numbers indicate the percentage of cells in the respective quadrant. The scatter profile of gated M-DC8⁺ cells is shown in (B). For comparison, (C) gives the scatter profile of ungated leukocytes.

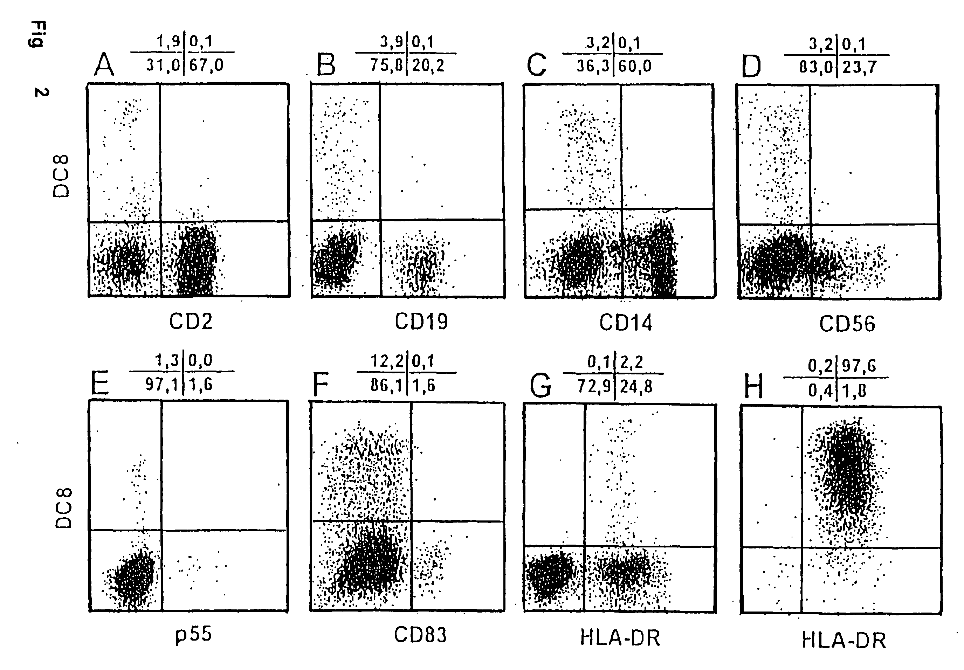

- **Figure 2**: Analysis of expression of lineage-specific and DC-related surface markers on M-DC8⁺ cells and efficiency of immunomagnetic isolation of M-DC8⁺ cells.
Double immunofluorescence staining of M-DC8⁺ cells was performed either on PBMC (A, E and G), on T cell-depleted PBMC (B-D), on PBMC depleted of T and B cells (F) or on purified M-DC8⁺ cells (H). For detection of the intracellular p55 antigen (E) unseparated PBMC were stained for the M-DC8 antigen, fixed with paraformaldehyde, permeabilized with saponin and labeled with a p55-specific mAb. The CD83 antigen was demonstrated on PBMC depleted of T and B cells and cultured for 48h simultaneous staining with M-DC8 and CD83 mAbs (F). The frequency of M-DC8⁺ and HLA-DR⁺ cells before and after isolation of M-DC8⁺ cells by magnetic cell sorting from PBMC is shown in (G) and (H). Results are representative for 20 (A-D, G and H) and 5 (E and F) different donors. The numbers indicate the percentage of cells in the respective quadrant.

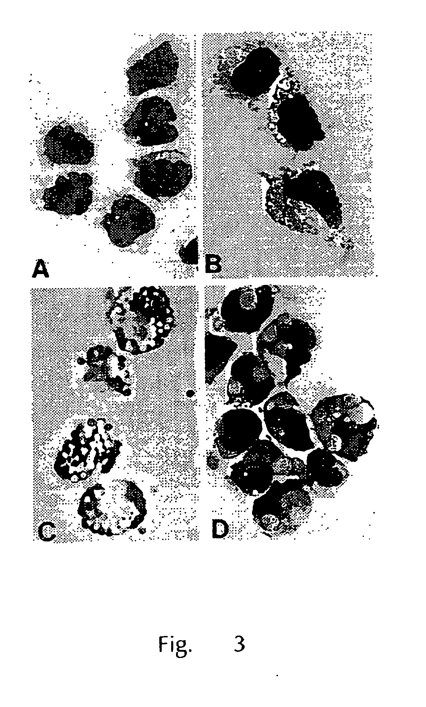

- **Figure 3**: Morphology and phagocytic activity of M-DC8⁺ cells.
In the upper panel cytospins of purified M-DC8⁺ cells immediately after isolation (A) and after in vitro culture for 48h (B) are shown. The lower panel shows M-DC8⁺ cells after phagocytosis of latex beads (C) or of antibody-coated SRBC (D). For light microscopy cells were stained using May-Gruenwald/Giemsa stain (A, B and D, magnification, x100). In (C) cells were subjected to confocal laser scanning microscopy after embedding in gelatine.

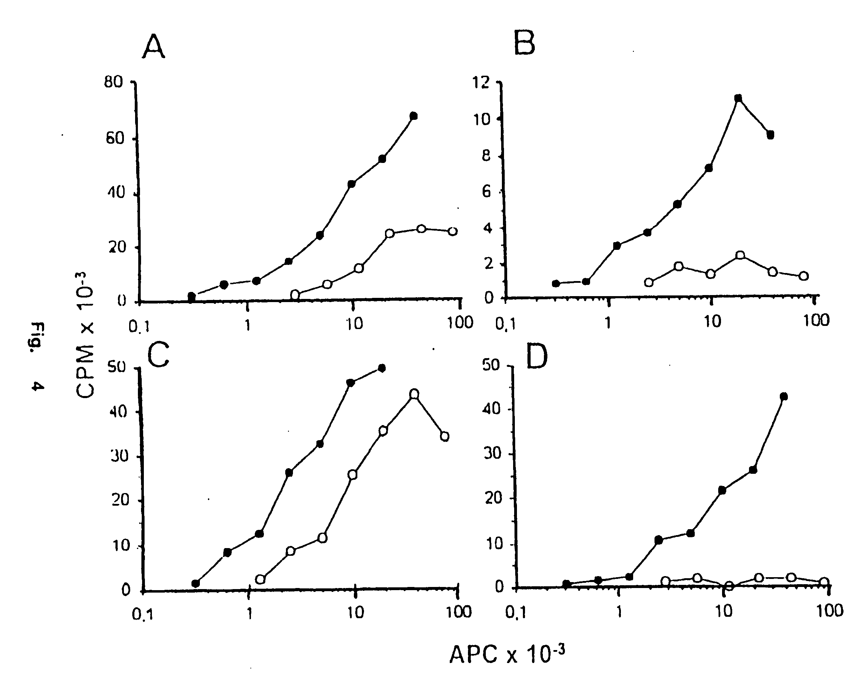

- **Figure 4**: Stimulation of T cells by M-DC8⁺ cells.
Purified T cells (1x10⁵) were cultured with graded numbers of either M-DC8⁺ cells (closed circles) or CD14⁺ monocytes (open circles) in the presence (C, D) or absence (A, B) exogenous soluble antigens. (A), allogeneic APC; (B), autologous APC; (C), autologous APC in the presence of TT (5 µg/ml); (D), autologous APC in the presence of KLH (1 µg/ml). After 5 days (A, C), or 7 days (B, D), proliferation of T cells was determined by ³H-thymidine uptake. In (C) and (D) values of T cell proliferation in the absence of soluble antigen were subtracted from the total cpm. Values shown represent the mean of triplicate samples. SEM was <15%. The results shown in (A), (B), and (C) are representative of 6 independent experiments, (D) is representative of 3 different experiments.

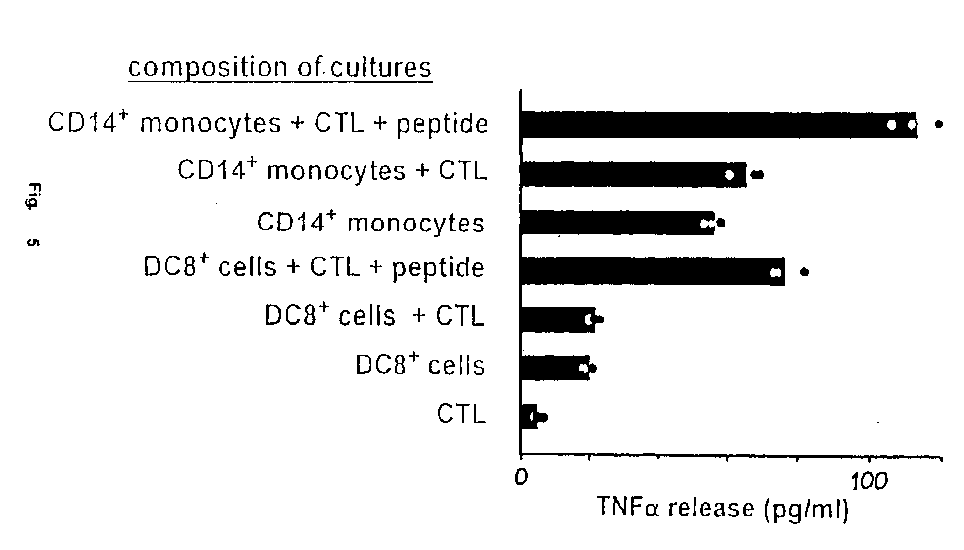

- **Figure 5**: Stimulation of a CTL clone by peptide loaded M-DC8⁺ cells and CD14⁺ monocytes.
M-DC8⁺ cells and CD14⁺ monocytes were pulsed overnight with 100 µg/ml of a tyrosinase-derived peptide and then cocultured with the tyrosinase-specific cloned CTL for further 40h. Activation of T cells was assayed by determining TNF-α secreted in the supernatants of triplicate cultures. Columns represent the mean of three individual values which are indicated in addition.

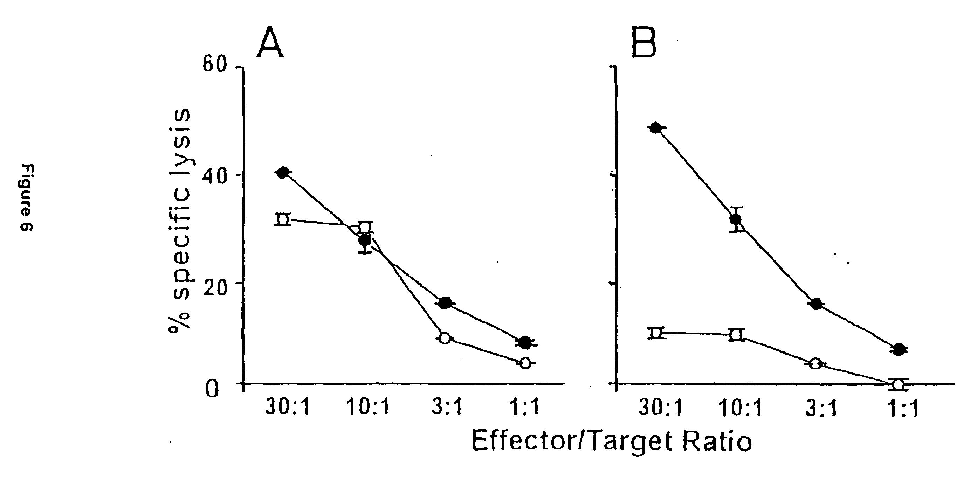

- **Figure 6**: Induction of alloantigen-specific cytotoxic T effector cells.
Unseparated T lymphocytes (A) and purified CD8⁺ T lymphocytes (B) were cocultured with allogeneic M-DC8⁺ cells (closed circles) or CD14⁺ monocytes (open circles) for 7 days. Cytotoxicity of T lymphocytes was tested against ⁵¹Cr-labeled stimulator type PHA blasts in a 4 hour chromium release test.

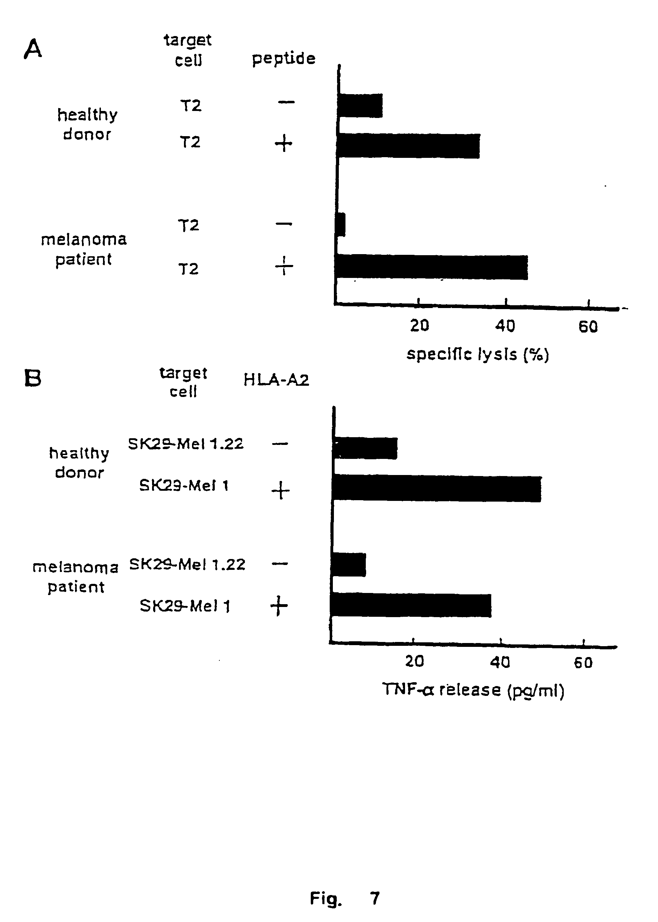

- **Figure 7**: Induction of cytotoxic effector cells specific for a tyrosinase-derived tumor peptide. PBMC of a healthy donor and a melanoma patient were activated by four consecutive rounds of stimulation against a tyrosinase-derived nonamer peptide presented by purified autologous M-DC8⁺ cells, A) Specific lysis of ⁵¹Cr-laboled T2 cells loaded with peptide at an effector to target ratio of 40 : 1. B) TNF-α release by effector cells after contact with melanoma cells lacking or expressing tyrosinase (E/T ratio 1 : 2). Bars represent means of triplicate determinations, SEM was <15%.
- **Figure 8**: Synopsis of different human blood DC subsets characterized on the basis of surface marker expression.

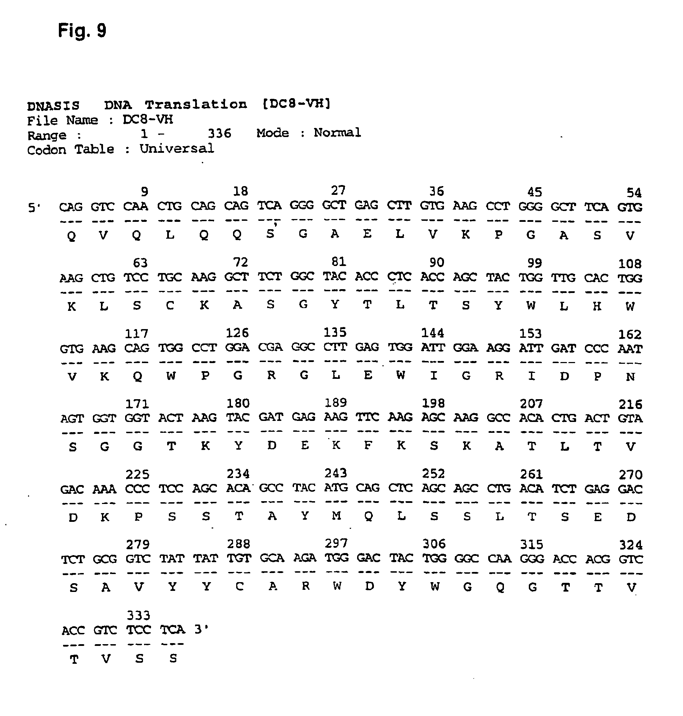

- **Figure 9**: Nucleotide sequences of the cDNAs encoding the heavy chain (VH) of the variable region of monoclonal antibody DC8 (SEQ ID NO: 1) and the deduced amino acid sequences (SEQ ID NO: 2) in one letter code. Numbers refer to the nucleotide sequence positions at the 3'-end of the triplet shown. 5'- end 3'-ends of the sequences are indicated.

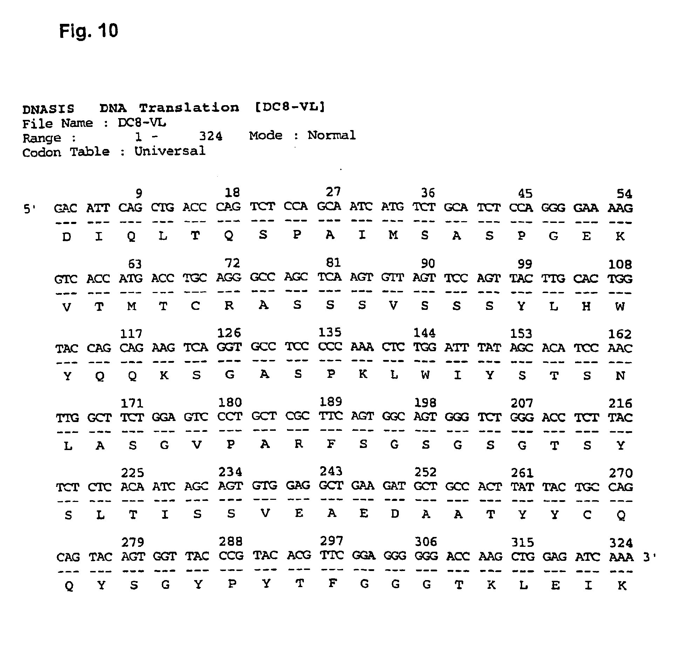

- **Figure 10**: Nucleotide sequences of the cDNAs encoding the light chain (VL) of the variable region of monoclonal antibody DC8 (SEQ ID NO: 3) and the deduced amino acid sequences (SEQ ID NO: 4) in one letter code. Numbers refer to the nucleotide sequence positions at the 3'-end of the triplet shown. 5'- end 3'-ends of the sequences are indicated.

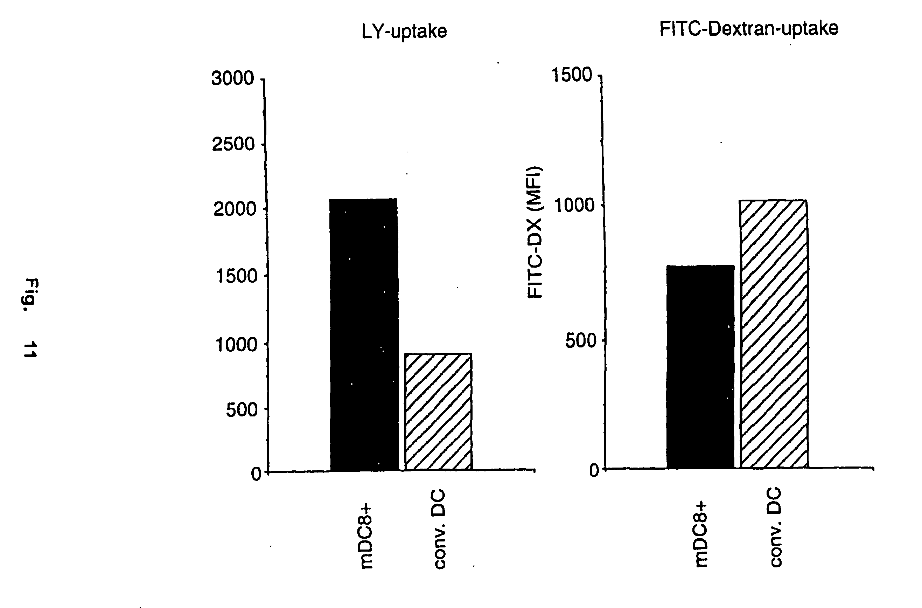

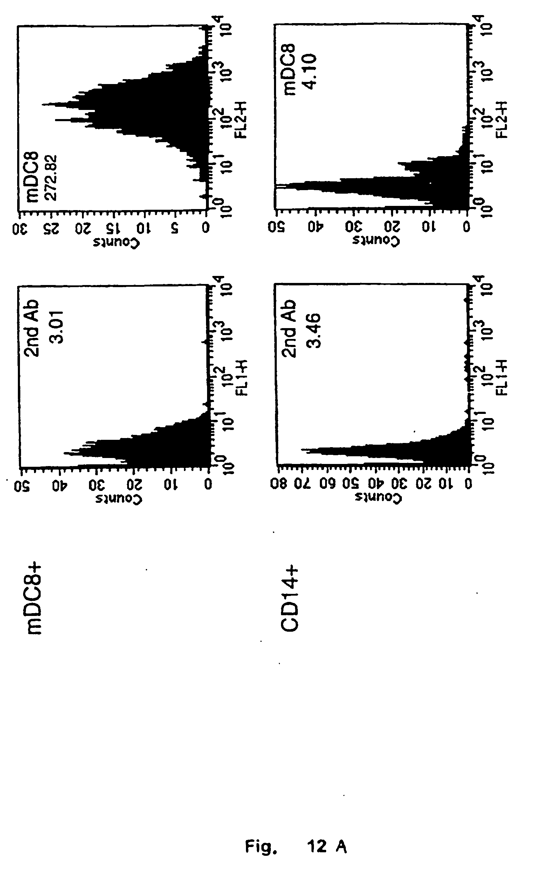

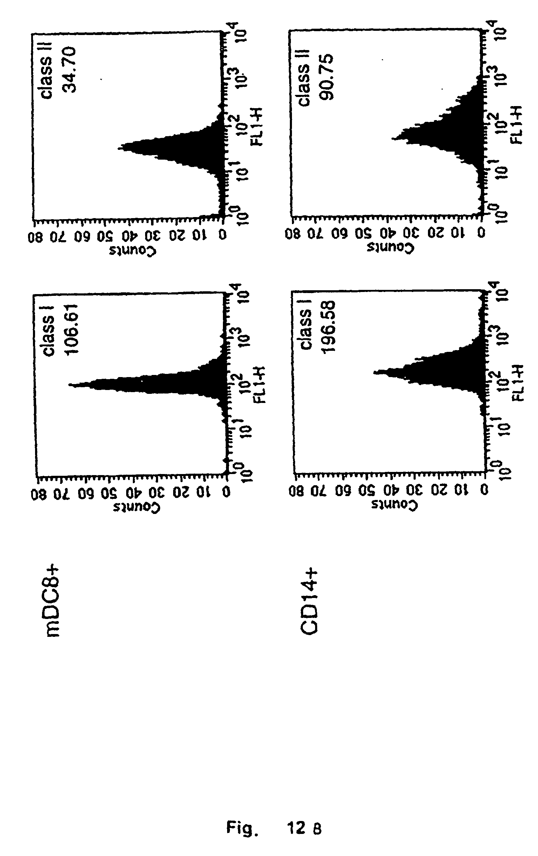

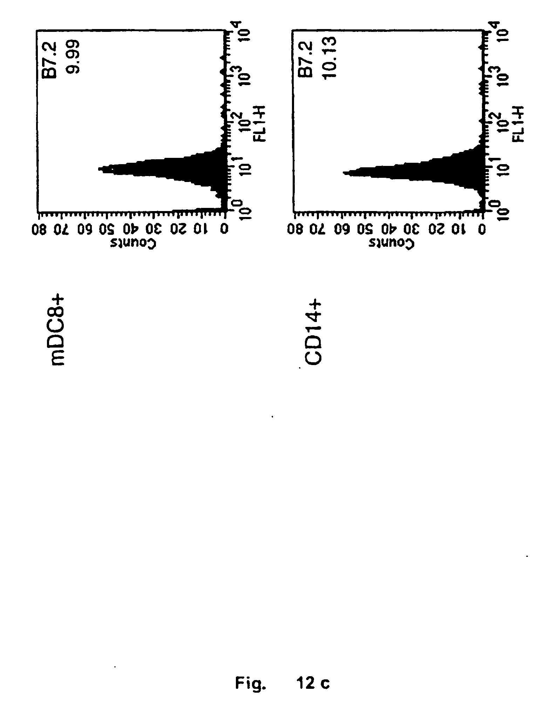

- **Figure 11**: Comparison of endocytotic capacity between mDC8-positive (filled bars) and conventional DCs (shaded bars). Micropinocytosis was measured by FACS using Lucifer yellow (LY uptake; left panel) and mannose receptor-mediated uptake by FITC-conjugated dextran (FITC-DX; right panel). Results from FACS analysis were quantitated and are expressed as mean fluorescence intensities (MFI).

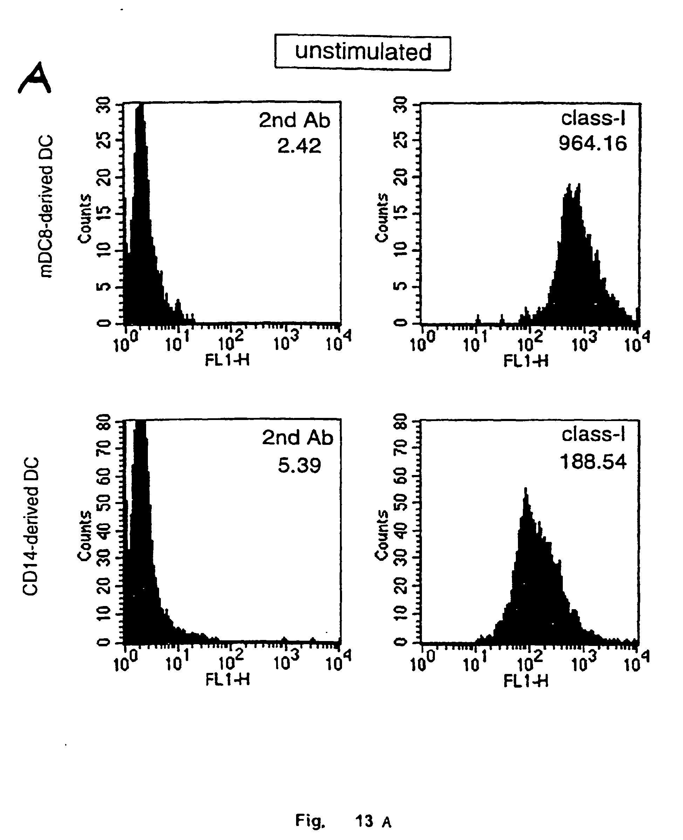

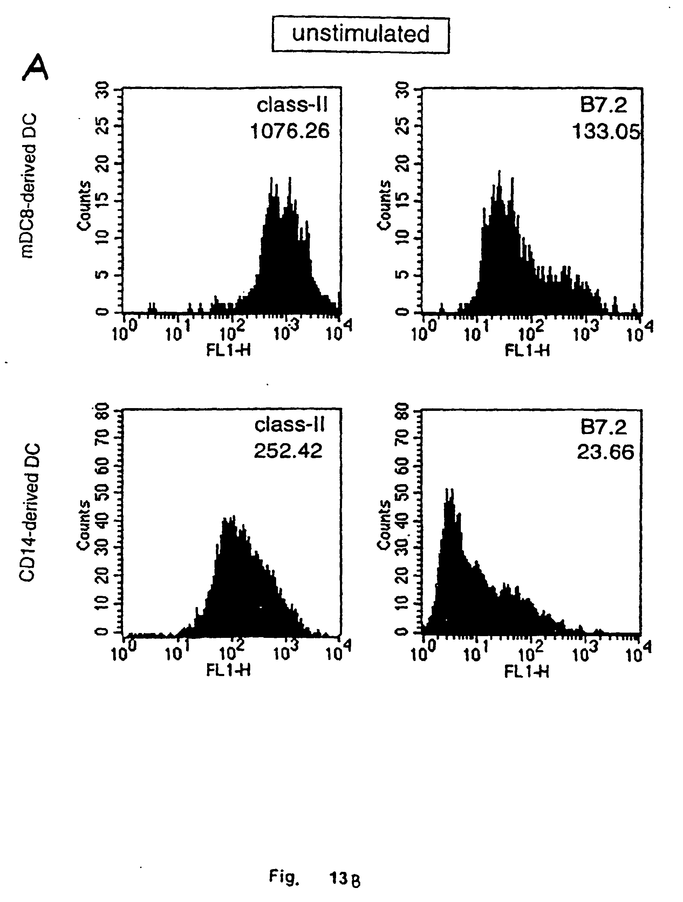

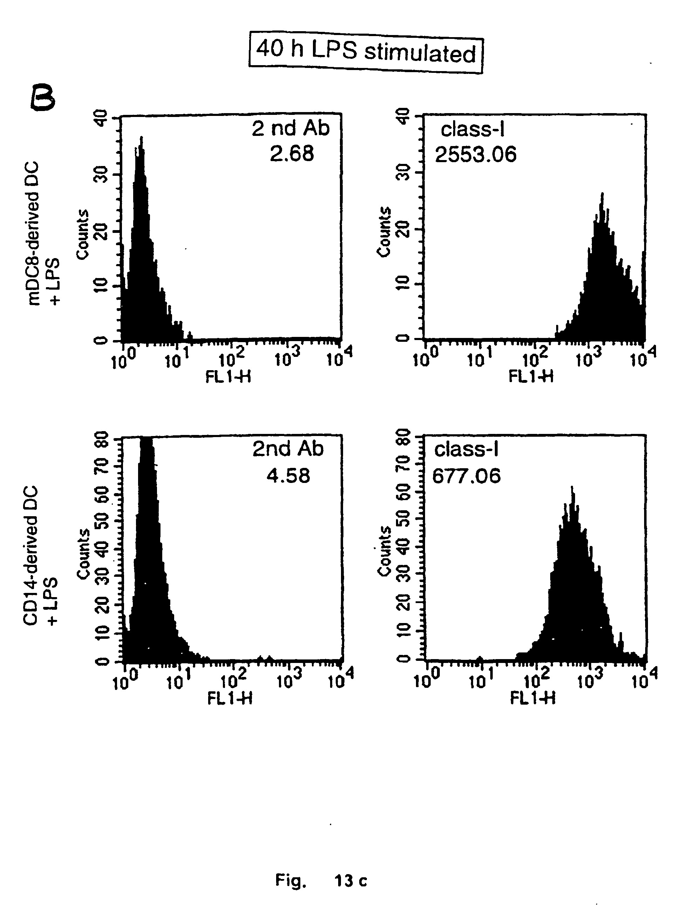

- **Figure 12**: Comparison of cell surface molecule expression capacity between freshly isolated mDC8-positive (upper panel) and precursor cells for conventional DCs (CD14-derived; lower panel). Histogram plots from FACS analyses are shown. The first histograms were obtained with the secondary antibody alone. The y-axis shows the number of cells; the x-axis shows the fluorescence intensity and is a measure for the level of cell surface expression. Numbers below the name of the surface antigen give the calculated mean fluorescence intensity.

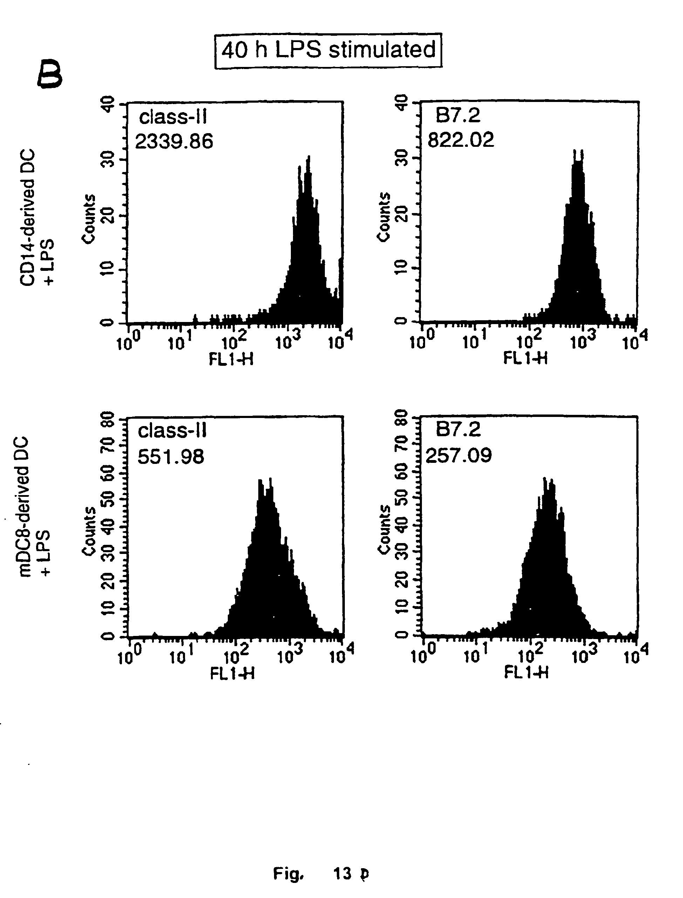

- **Figure 13**: Comparison of cell surface molecule expression capacity between cultured mDC8-positive (upper panel) and precursor cells for conventional DCs (CD14-derived; lower panel). A. Cells cultured for 7 days in the presence of GM-CSF and IL-4 only; B. Cells stimulated at day 5 of the GM-CSF/IL-4 culture for 40 h with 1 µg/ml LPS. Histogram plots from FACS analyses are shown. The first histograms were obtained with the secondary antibody alone. The y-axis shows the number of calls; the x-axis shows the fluorescence intensity and is a measure for the level of cell surface expression. Numbers below the name of the surface antigen give the calculated mean fluorescence intensity.

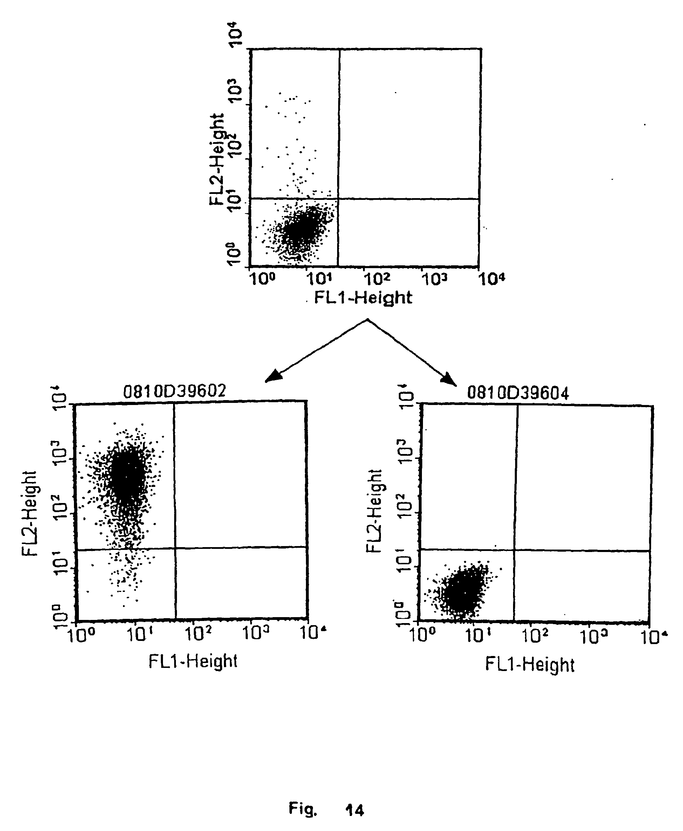

- **Figure 14**: Establishment of Jurkat T lymphoma cell subclones that express or not express the antigen which is recognized by the monoclonal antibody M-DC8. Jurkat T cells were analyzed by FACS after staining with M-DC8 IgM and detection with PE-conjugated anti-IgM antibody (y-axis). Normal Jurkat T cells (upper panel) had very few M-DC8-positive cells (upper left quadrant). These cells were isolated and propagated over many rounds of selection resulting in a subclone highly expressing M-DC8 (lower left panel). A subclone of Jurkat cells showing virtually no M-DC8-positive cells (lower right panel) was isolated after depletion of any M-DC8 positive cells using magnetic beads decorated with M-DC8 antibody.

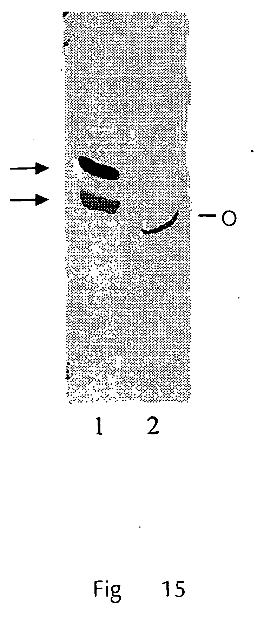

- **Figure 15**: Binding of monoclonal antibody M-DC8 to glycolipids extracted from Jurkat expressing the M-DC8 antigen. Lipids were extracted from Jurkat subclones either highly expressing the M-DC8 antigen (lane 1) or not expressing the antigen (lane 2). Lipids were seperated by thin layer chromatography and the chromatogram probed with M-DC8 IgM and its detection by a secondary anti-IgM antibody. A Western blot with lipids is shown. The position of two lipids immunoreacting with M-DC8 IgM are indicated by arrows. The open circle marks a minor band from M-DC8-negative cells.

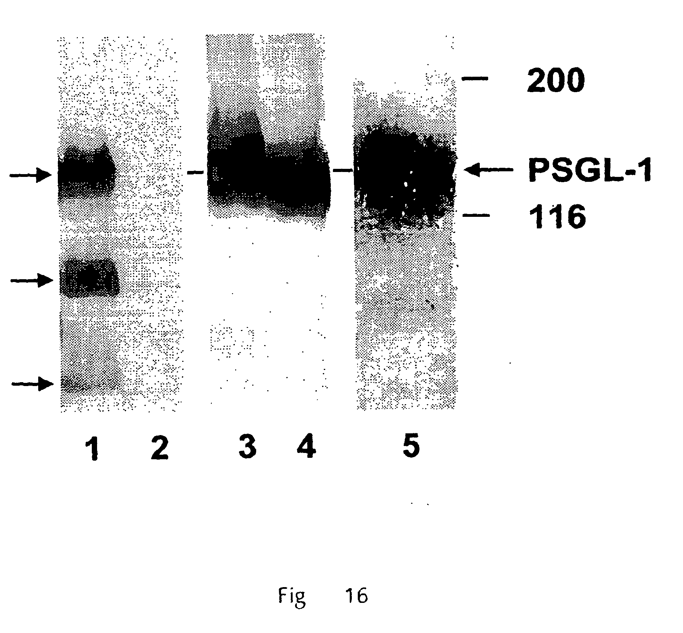

- **Figure 16**: Characterization of membrane proteins immunoreacting with monoclonal antibody M-DC8. Integral membrane proteins were prepared from M-DC8-positive Jurkat cells (lanes 1 and 3), M-DC8-negative Jurkat cells (lanes 2 and 4) and M-DC8-positive dendritic cells isolated from human PBMCs (lane 5). Western blots are shown. For lanes 1, 2 and 5, filters were probed with M-DC8 IgM detected by a secondary anti-IgM antibody. For lanes 3 and 4, an antibody was used which recognizes the glycoprotein PSGL-1. The positions of PSGL-1 (right) and of three M-DC8-positive proteins (left) are indicated by arrows. The position of two molecular size standards of 200 and 116 kDa are indicated on the left.

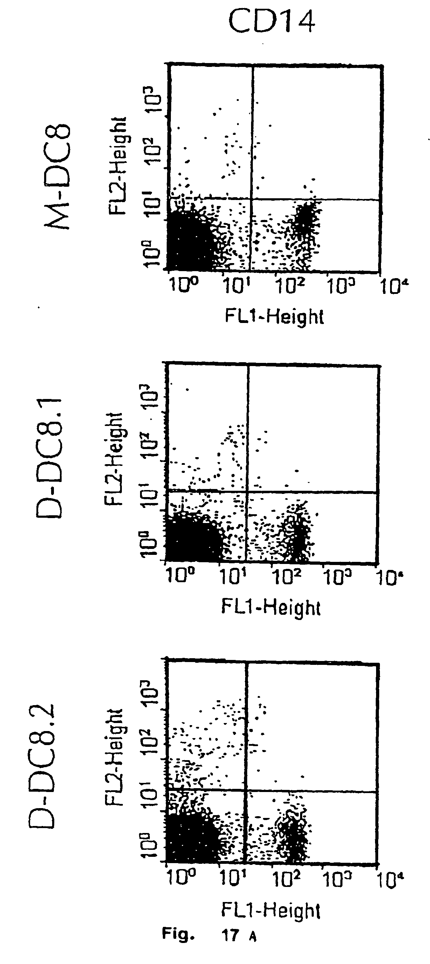

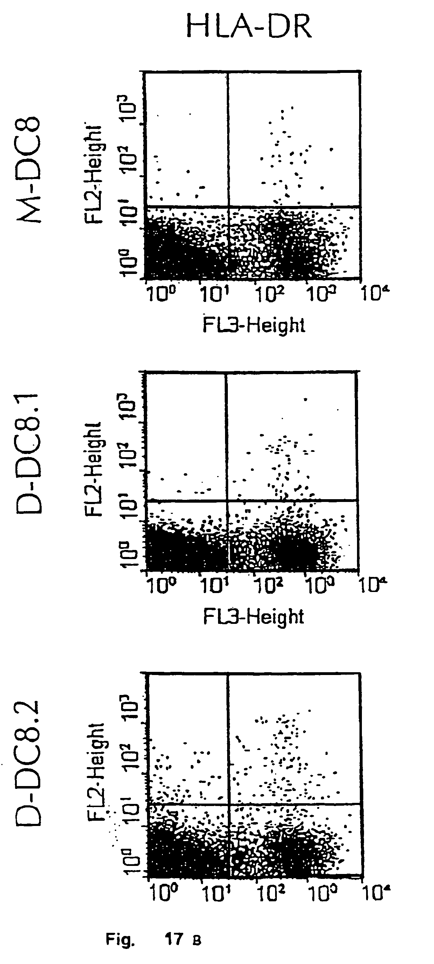

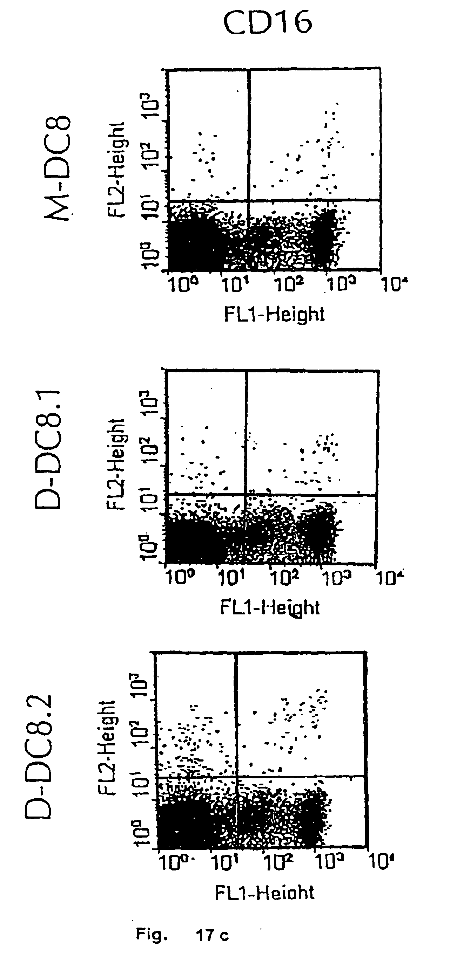

- **Figure 17**: Characterization of PBMCs recognized by the monoclonal antibodies D-DC8.1 and D-DC8.2. Human PBMCs from the same donor were double-labeled with antibodies against CD14, HLA-DR or CD16 and the three monoclonals as indicated, and analyzed by FACS. Double positive cells are seen in the upper right quadrants.

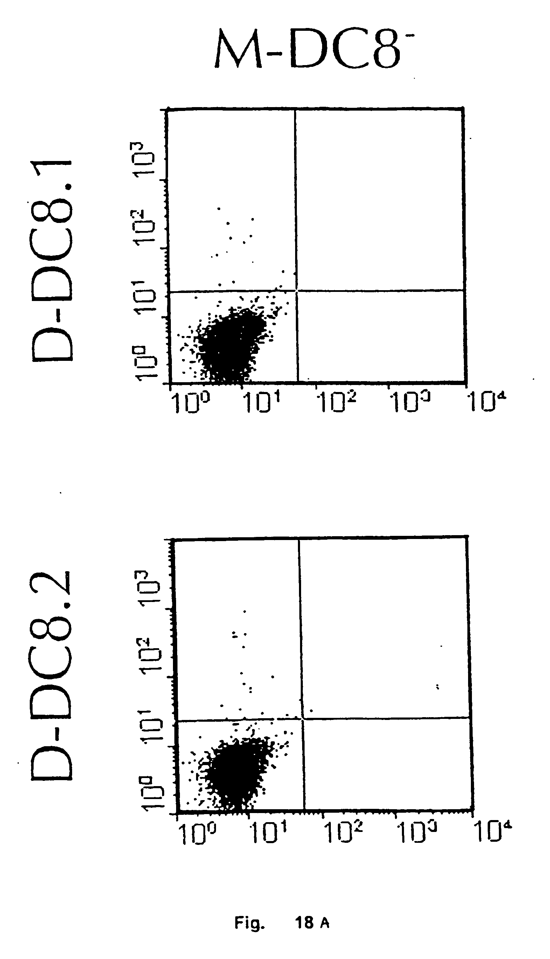

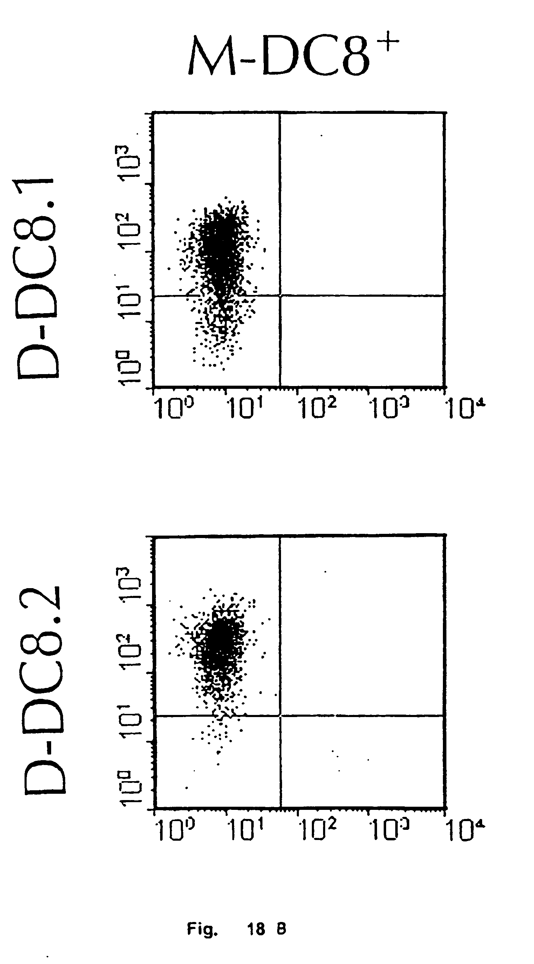

- **Figure 18**: Binding of monoclonal antibodies D-DC8.1 and D-DC8.2 to Jurkat subclones expressing the M-DC8 antigen. Jurkat subclones expressing M-DC8 antigen (M-DC8⁺) and not expressing the antigen (M-DC8⁻) were tested for binding of D-DC8.1 and D-DC8.2 antibodies. FACS analyses are shown. Monoclonal IgM was detected by a PE-conjuagted anti-IgM antibody (Y-axis). Cells binding the monoclonal antibodies are shown in the upper left quadrants.

### The examples illustrate the invention

### Example 1: Isolation and Characterization of M-DC8⁺ leukocytes

Whole heparinized blood and buffy coat preparations were obtained from the Department of Blood Transfusion, Medical Faculty, TU Dresden, with the informed consent of the blood donors. Cells were cultured in RPMI 1640 medium supplemented with 2 mM L-glutamine, 1% nonessential amino acids (Biochrom AG, Berlin, FRG), 100 U/ml penicillin, 100 µg/ml streptomycin (both from Gibco, Eggenstein, FRG) and 10% heat inactivated pooled human serum, referred to as complete medium (CM). For FACS analysis of total leukocytes, red blood cells were lysed with lysing solution (Becton Dickinson & Co., Heidelberg, FRG) according to the manufacturers instructions. Peripheral blood mononuclear cells (PBMC) were prepared by Ficoll-Hypaque (Pharmacia, Freiburg, FRG) density centrifugation. T lymphocytes, B-lymphocytes and monocytes were removed from PBMC by a direct mAb rosetting technique (DART) using bovine red blood cells (BRBC) coated with purified CD2 (M-T910). CD37 (M-B372) or CD14 (M-M42) mAbs (originating from our laboratory and clustered at the 'IVth Int. Workshop on Human Leukocyte Differentiation Antigens'²⁸) using the CrCl₃ method essentially as described²⁷. A greater than 99% depletion was obtained by a second round of rosetting with BRBC coated with the respective mAbs. T lymphocytes of >98 % purity were obtained from PBMC by rosetting with sheep erythrocytes treated with aminoethyl isothiuronium bromide (AET, Sigma, Deisenhofen, FRG) followed by Ficoll density centrifugation as described²⁷. From this population CD8⁺ T cells of >98% purity were prepared by depletion of CD4⁺ cells as rosettes with BRBC coated with the CD₄ mAb M-T310²⁸.

M-DC8 was selected out of more than 6 x 10³ hybridoma supernatants by screening on HLA-DR⁺ PBMC which had been depleted of T and B cells and monocytes. After lysis of erythrocytes M-DC8 mAb labeled 0.5-1% of the total leukocyte population (Fig.1A). M-DC8⁺ cells were obtained by incubating PBMC for 15 min at 4°C with undiluted supernatant of the M-DC8 hybridoma containing 10 µg/ml antibody. After washing with PBS 1x10⁸ cells were labeled with 10 µl of rat anti-mouse IgM coupled to paramagnetic microbeads (Miltenyi Biotec, Bergisch-Gladbach, FRG) for another 15 minutes at 4°C. After washing, cells were thoroughly resuspended to avoid cell aggregation and sorted on a VS+ separation column (Miftenyl). If necessary, the highly enriched cells were further purified by a second round of magnetic cell sorting using a RS+ separation column (Mlltenyi). Degased, ice cold PBS containing 1% human serum was used as running and elution buffer. Pure monocytes were prepared from the non magnetic cell fraction obtained after the initial magnetic cell sorting by incubation with anti CD14-coated paramagnetic microbeads (Miltenyl). After magnetic cell sorting using a VS+ separation column a >95% pure population of CD14⁺ cells was obtained. With regard to size and granularity M-DC8⁺ cells represented a distinct population which In light scatter display was located between lymphocytes and monocytes (Fig.1B, C). By two-color immunofluorescence analysis using the M-DC8 mAb together with each of the following lineage-specific mAbs CD2, CD19, CD14 and CD56, M-DC8⁺ cells could be excluded from T cell, B cell, monocyte or NK cell populations, respectively (Fig. 2 A-D). For two-color fluorescence analysis cells were incubated with undiluted M-DC8 hybridoma supernatant, followed by PE- or FITC-conjugated goat F(ab')₂ anti-mouse IgM (Coulter-Immunotec, Hamburg, FRG). After saturation of uncomplexed binding sites of the secondary antibody with normal mouse serum diluted 1/10 the following fluorescent dye-conjugated mAbs were applied at concentrations recommended by the suppliers: CD2 (T9-10, IgG1, FITC), CD4 (13B8.2, IgG1. PE), CD11b (ZLPM19C, IgG1, PE), CD14 (T†K4, IgG2a, FITC), CD18 (MHM23, IgG1, FITC), all purchased from Dako, Hamburg, FRG; CD1a (M-T102, IgG2b, PE), CD11a (G43-25B, IgG1, FITC), CD11c (B-LY6, IgG1, PE), CD32 (FLI8.26, IgG2b, FITC), CD33 (WM53, IgG1, PE), CD40 (5C3, IgG1, FITC), CD54 (HA58, IgG1, PE), CD56 (B159, IgG1, PE-Cy5), CD80 (BB1, IgM, FITC), CD86 (IT2.2, IgG2b, FITC) (FUN1, IgG1, FITC), CD106 (51-10C9, IgG1, FITC), HLA-DP (HI43, IgG1, FITC), HLA-DQ (T†169, IgG2a, FITC), HLA-A, B, C (G46-2.6, IgG1, FITC) all obtained from Pharmingen, Hamburg, FRG; CD3 (UCHT1, IgG1, PE-Cy5), CD13 (WM15, IgG1, FITC), CD16 (3G8, IgG1, FITC), CD19 (J4.119, IgG1, PE), CD34 (QBENP10, IgG1, FITC), CD45RO (UCHL1, IgG2a, PE), CD45RA (ALB11, IgG1, FITC), CD50 (HP2/19, IgG2a, PE), CD58 (AICD58, IgG2a, PE), CD64 (22, IgG1, FITC), anti-HLA-DR (B8.12.2, IgG2b, PE) all bought from Coulter-Immunotech, Hamburg, FRG. Two-color immunofluorescent staining with the two unlabeled mAbs M-DC8 and CD83 (HB-15a and HB-15b, obtained in the course of the Vth Workshop on Human Leucocyte Differentiation Antigens) was performed as follows: cells were first incubated with the CD83 mAb, followed by FITC-conjugated goat F(ab')₂ anti-mouse Ig (Dako, Hamburg, FRG). After quenching with 1/10 diluted normal mouse serum cells were stained with M-DC8 mAb and PE-oonjugated goat F(ab')₂ anti mouse IgM (Coulter-Immunotec) as secondary antibody. For two-color staining using mAb M-DC8 and p55 (kindly provided by Dr. E. Langhoff) PBMC were first surface stained for M-DC8 as described above, followed by fixation with ice-cold 4% paraformaldehyde (Merck, Darmstadt, FRG), permeabilisation with 0.1% saponin (Sigma, Deisenhofen, FRG), staining with the mAb p55, followed by FITC-conjugated rat-anti mouse IgG1. After each incubation cells were washed two times with cell-wash (Becton Dickinson & Co., Heidelberg, FRG). Stained cells were analyzed on a FACScan (Becton Dickinson) using the Lysis II program. As two markers of human blood DC, CD83 and p55, define an identical DC subset^{9,10}, it was of interest to know whether M-DC8 characterized an overlapping cell population. When unseparated PBMC were simultaneously stained for p55 and M-DC8 two different cell populations were defined by these two markers (Fig. 2E). For induction of CD83 expression PBMC were depleted of T and B cells and cultured for 48h. Staining of these cells with the CD83 and M-DC8 mAb revealed two different cell subsets (Fig. 2F). Thus M-DC8 defines a unique leukocyte population different from the one identified by known lineage and DC markers. Table 1 shows the results of an extensive surface marker analysis comparing freshly isolated and cultured M-DC8⁺ cells as well as CD14⁺ monocytes. Fresh M-DC8⁺ cells displayed HLA class II antigens (HLA-DR, -DP, -DQ), adhesion molecules such as the β₂-integrins CD11a,b,c/CD18 and the β₁-integrin chain CD29, cellular adhesion molecules like ICAM-1 (CD54) and ICAM-3 (CD50), LFA-3 (CD58) and the Fcγ receptors CD32 (FcγRII) and CD16 (FcγRIII). In addition, they were positive for the myeloid markers CD13 and CD33 and expressed CD4 at moderate density. CD1a, a marker for epidermal DC¹⁵ as well as CD106 (VCAM-1), expressed by thymic DC¹⁶, was not detected on fresh M-DC8⁺ cells.

M-DC8⁺ cells expressed the classical hallmarks of antigen presenting cells such as B-7.1 (CD80), B-7.2 (CD86) and CD40 in addition to MHC class II and adhesion molecules^{1,9}. While HLA-DR, CD40 and CD86 were expressed at lower levels on freshly isolated M-DC8⁺ cells these molecules were substantially upregulated after 36h of In vitro culture. At this time, also the costimulatory molecule B-7.1 (CD80) became detectable, although at low density only.

Surface markers of freshly isolated and cultured M-DC8⁺ cells are compiled in Table 1; see below. For comparison, the phenotype of freshly prepared CD14⁺ monocytes is also shown.

**Table 1: Surface phenotype of freshly isolated and cultured M-DC8⁺ cells compared with CD14⁺ monocytes.**

| | **M-DC8⁺ cells** | | **CD14⁺ monocytes** |
|---|---|---|---|
| **Antigen** | **freshly isolated** | **cultured for 36h** | **freshly isolated** |
| CD1a | - | | - |
| CD4 | + | | + |
| CD11a | ++ | | +/++ |
| CD11b | +/++ | | ++ |
| CD11c | ++/+++ | +++ | ++ |
| CD13 | + | | + |
| CD16 | ++ | - | -/+ |
| CD18 | ++ | | ++ |
| CD29 | + | | + |
| CD32 | + | | + |
| CD33 | + | | ++ |
| CD34 | - | | - |
| CD40 | -/+ | ++ | -/+ |
| CD45RA | ++ | | + |
| CD45R0 | -/+ | | ++ |
| CD50 | ++/+++ | +++ | ++l+++ |
| CD54 | ++ | +++ | ++ |
| CD58 | ++ | +++ | ++ |
| CD64 | - | | +/++ |
| CD80 | - | + | - |
| CD86 | + | ++ | + |
| CD106 | - | | - |
| HLA-A, B, C | ++ | ++++ | ++ |
| HLA-DR | +/++ | +++ | ++ |
| HLA-DP | + | | + |
| HLA-DQ | + | | + |

M-DC8⁺ cells and CD14⁺ monocytes were isolated from PBMC by an immunomagnetic procedure. Immediately after isolation or after culture for 36h in CM cells were subjected to immunofluorescence staining and FACS analysis. Levels of mean fluorescence intensity (MFI) are given as -, indicating MFI in the first decade on a four log scale, corresponding to isotype control levels; +, ++, and +++ indicate MFI in the second, third, and fourth decades, respectively. No symbol means not determined. The data are representative of at least 5 individual experiments.

The most prominent features distinguishing M-DC8⁺ cells from CD14⁺ monocytes were the high density of CD16 (FcγRIII) and the lack of CD64 (FcγRI) expression on M-DC8⁺ cells. Interestingly, CD16 disappeared from the surface of M-DC8⁺ cells during in vitro culture for 36h. In addition, M-DC8⁺ cells consistently expressed CD45RO antigen at lower levels when compared with monocytes, whereas the surface density of CD11a and CD11c and the RA isoform of the CD45 molecule was considerably higher on M-DC8⁺ cells.

### Example 2: Morphological and functional characterization of M-DC8⁺ cells

The M-DC8 antibody proved to be particularly efficient to isolate DC from PBMC by a one step procedure employing magnetic cell sorting. With this method viable M-DC8⁺ cells could be reproducibly isolated with a yield of 60 to 90% and a purity of >97% (Fig. 2G, H). These cells appeared as round, medium-sized leukocytes on cytospins (Fig. 3A) that during culture increased In size and showed dendritic cytoplasmatic protrusions (Fig 3B). The freshly isolated cells eagerly phagocytosed 1µ latex beads as well as antibody-coated SRBC (Fig. 3C and D).

Freshly prepared monocytes and M-DC8⁺ cells were incubated with sheep red blood cells (SRBC) opsonized with anti-SRBC serum 1:100 (Sigma, Deisenhofen, FRG) or latex beads of 1 or 5 µ diameter (Sigma) at 37°C and 5% CO₂. After 30 minutes non internalized SRBC were lysed by hypotonic lysis (distilled water for 5 seconds followed by addition of an equal volume of 1.8% NaCl). Internalization of SRBC was assessed on cytospins after May Grünwald Giernsa staining. Internalized latex beads were visualized by confocal laser scanning microscopy (Leica TCS 4D, Leica, Heidelberg, FGR).

### Example 3: Allogeneic and autologous MLR

Various T cell stimulatory assays were performed to evaluate the ability of M-DC8⁺ cells to induce antigen-specific T cell activation. For comparison, monocytes were always assayed in parallel. 1 x 10⁵ purified T lymphocytes were cocultured with graded numbers of APC in 0.2 ml CM In 98-well round-bottomed microtiter plates (Coming, New York, NY, USA). Tetanus toxoid (TT, Behringwerke, Marburg, FRG) or keyhole limpet hemocyanin (KLH, Boehringer-Mannheim, Mannheim, FRG) were added as antigens at a concentration of 5 µg/ml and 1 µg/ml, respectively. Proliferation of T cells was determined after 5 days by uptake of ³H-thymidine (specific activity 79.1 Ci/mmol, concentration 1.0 mCi/ml, DuPont, Bad Homburg, FRG) which was added at a concentration of 2 µCi/well 12-15h before harvesting. In experiments where cloned melanoma specific cytotoxic T cells were activated by APC external loaded with antigenic peptides, the APC preparations from a HLA-A0201 donor were incubated overnight with 100 µg/ml of the tyrosinase peptide YMDGTMSQV at 37°C in RPMI 1640 medium supplemented with 1% human serum. After extensive washing 104 peptide-pulsed APC were cocultured with 10⁴ cells of the T cell clone IVSB (kindly provided by Dr. T. Wolfel, University of Mainz, FRG) in 200 µl CM in round bottomed microtiter plates (Coming) in the presence of 25 U/ml rhIL-2 (Genzyme, Munich, FRG). The cytotoxic CD8⁺ T cell clone specifically recognizes the above mentioned tyrosinase peptide complexed with HLA-A0201 molecules (19). After 40h released TNF-α was determined in the supernatants by ELISA (R&D Systems, Minneapolis, USA). As can be seen from Figure 4A, purified cells efficiently induced an allogeneic MLR when 1x10⁵ T cells were cultured with various numbers of allogeneic APC. M-DC8⁺ cells proved to be at least five times more efficient than monocytes tested simultaneously. As induction of an autologous MLR is considered a characteristic property of DC¹ isolated M-DC8⁺ cells were cocultured with autologous T cells. As Fig. 4B demonstrates autologous T cells were stimulated in a dose dependent fashion. The absolute values, however, were much lower in the autologous than in the allogeneic MLR. When assayed under the same conditions, CD14⁺ monocytes induced only a marginal T cell reactivity.

### Example 4: Activation of T cells against recall and neoantigens

Fig. 4C shows that M-DC8⁺ cells very efficiently presented tetanus toxoid (TT) to T cells obtained from TT-vaccinated donors. Already 600 M-DC8⁺ cells in 1x10⁵ T cells were able to induce a tetanus-specific T cell activation. When simultaneously tested, about 2500 monocytes were required to obtain the same level of T cell proliferation. In contrast to CD14⁺ monocytes, M-DC8⁺ cells induced a marked primary T cell response against keyhole limpet hemocyanin (KLH) in vitro (Fig. 4D).

### Example 5: Activation of a cytotoxic T cell clone

In addition to their capacity to activate CD4⁺ T lymphocytes DC are pivotal to stimulate CD8⁺ T lymphocytes by class I-presented endogenous peptides^{17,18} The ability of M-DC8⁺ cells to present MHC class I-bound peptides to CD8⁺ T cells was first demonstrated with a cytotoxic T cell clone (IVSB) which was derived from a melanoma patient and which recognizes a tyrosinase-derived peptide on HLA-A0201 molecules¹⁹. For stimulation M-DC8⁺ cells and monocytes from a HLA-A0201 positive blood donor were preincubated with the peptide over night before adding the cloned T cells. After 48h the response of the cytotoxic clone was determined by measuring secreted TNF-α. As shown in Fig. 5 M-DC8⁺ cells presented the peptide to the cloned T lymphocytes as efficiently as peptide pulsed monocytes.

### Example 6: Induction of alloantigen-specific cytotoxic CD8⁺ T cells

In order to determine the capacity of M-DC8⁺ cells to prime CD8⁺ cytotoxic T cells in vitro they were cocultured either with unseparated allogeneic T cells or with purified allogeneic CD8⁺ T lymphocytes. Antigen-specific priming of CD8⁺ T lymphocytes was assessed by lysis of ⁵¹Cr-labeled allogeneic PHA blasts. Purified T cells or isolated CD8⁺ T cells (1 x 10⁶) were cocultured with 5 x 10⁴ allogeneic M-DC8⁺ cells or monocytes as stimulator cells in a total volume of 2 ml CM in 24 well Costar plates (Costar, Heidelberg, FRG). After 7 days, cells were harvested, washed and tested for cytotoxic activity against ⁵¹Cr-labeled PHA blasts of stimulator and, as a control, of responder origin. During the final 24h of culture rhIL-2 (Genzyme) was added at a concentration of 25 U/ml. To generate PHA blasts 1x10⁶ PBMC were stimulated with 1 µg/ml PHA (Gibco) in 2 ml CM for 3 days. The cells were then cultivated for one additional day in the presence of 26 U/ml hrlL-2 (Genzyme). After washing, 1 x 10⁶ cells were resuspended in 100 µl FCS and 100 µCi of ⁵¹Cr (sodium chromate, specific activity 440 mCi/mg, DuPont) was added at 37°C for 1h. Cytotoxic activity of alloantigen-stimulated T lymphocytes was determined by incubating various numbers of effector cells with 5x10^{3 51}Cr-labeled PHA blasts in 200 µl of CM in microtiter plates at 37°C for 4h. The cells were spun down and 100 µl of the supernatant were removed for determination of radioactivity in a gamma scintillation counter (Packard, Dreieich, FRG). Maximal release from labeled cells was determined after three cycles of freezing and thawing. The specific cytotoxicity was calculated according to the formula:

Percent specific lysis = 100 x [(cpm test release - cpm spontaneous release) / (cpm maximal release - cpm spontaneous release)]. Fig. 6A shows that M-DC8⁺ cells and monocytes induced alloantigen-specific cytotoxic T cells with the same efficiency when cocultured with unseparated T lymphocytes. M-DC8⁺ cells, however, were by far more effective than monocytes in activating allospecific cytotoxic effector cells when cocultured with purified allogeneic CD8⁺ T lymphocytes (Fig. 6B).

### Example 7: Induction of tumor-specific cytotoxic T-cells

To test the capacity of M-DC8+ cells to prime cytotoxic T cells against tumor-derived peptides PBMC of healthy donors and melanoma patients expressing the HLA-A*0201 antigen were activated by four consecutive rounds of stimulation with autologous M-DC8⁺ cells loaded with the tyrosinase-derived peptide YMDGTMSQV known to bind to this HLA class I molecule. Specificity of the induced T cells was determined both by lysis of peptide-loaded T2 cells that express the HLA-A*0201 antigen and release of TNF-α after contact with tyrosinase expressing melanoma cells. Freshly isolated M-DC8⁺ cells were loaded with 50 µg/ml of the tyrosinase-derived peptide YMDGTMSQV and 3 µg/ml of human β2-microglobulin (Sigma) in serum-free medium for 4 h at 37°C. After washing, 2 x 10⁵ peptide-loaded M-DC8⁺ cells were cocultured with 2 x 10⁶ autologous PBMC in 2 ml CM per well of a 24 well tissue culture plate. After 3 days the medium was supplemented with 25 U/ml rhlL-2 (Genzyme, Munich, FRG). After one week responder cells were collected, washed, distributed at 1 x10⁶ cells / well and restimulated with 1 x 10⁵ of freshly prepared M-DCB⁺ cells loaded with the tyrosinase peptide. Restimulation was repeated after two and three weeks. One week later cytotoxic activity was tested either by lysis of ⁵¹Cr-labeled T2 target cells loaded with peptide or by determining the TNF-α release after contact with tyrosinase expressing melanoma cells. T2 cells (obtained from ATCC, Rockville, MD) were incubated overnight with 100 µg/ml of the tyrosinase-derived peptide, washed and labeled at 1 x 10⁶ cells with 100 µCi of 51 Cr for 1h at 37°C. After washing cells were seeded at 5 x 10³ per well of a round-bottomed microtiter plate and incubated with various concentrations of cytotoxic effector cells for 4 h. Specific lysis was determined as described above. For the TNF-α release test 1 x 10⁴ peptide stimulated cells were incubated with 2 x 10⁴ cultured melanoma cells in 200 µl CM at 37°C in the presence of 25 U/ml rhIL-2. After 24 h released TNF-α was determined in the supernatants by ELISA (R&D Systems, Wiesbaden, FRG). Figure 7 shows the data of a representative experiment obtained with PBMC of a healthy donor and of a melanoma patient. PBMC of both donors could be induced to peptide-directed cytotoxicity. The specificity of peptide recognition was derived from control experiments where neither T2 target cells without addition of the tyrosinase peptide nor the mutant melanoma cell line SK29-Mel 1.22 lacking the restricting HLA-A*0201 allele were able to activate the generated cytotoxic effector cells. That the cytotoxic activity was caused by bona fide CD8⁺ T cells was demonstrated by selective immunomagnetic removal of CD8⁺ T cells which completely ablated the induced cytotoxicity.

### Example 8: Cloning of the variable regions (V_{L} and V_{H}) of the antibody M-DC8.

The variable regions V_{L} and V_{H} of the monoclonal antibody M-DC8 were cloned from the total RNA of the corresponding hybridoma cell line (DSM ACC224) as described by Orlandi et at. (Proc. Natl. Acad. Sci. USA 86 (1989), 3833-3837). Nucleotide and amino acid sequences of V_{L} and V_{H} are depicted in Figures 9 and 10.

### Example 9: Comparison of M-DC8 positive dendritic cells (M-DC8⁺ cells) with conventional, CD14 positive dendritic cells (DCs)

Employing an immunoisolation approach, DCs isolated with the monoclonal antibody M-DC8 (as deposited under the accession No: DSM ACC 2241) were compared to monocyte-derived DCs isolated with an anti-CD14 monoclonal antibody with respect to their endocytotic capacity and expression of surface markers characteristic for antigen-presenting cells.

M-DC8-positive cells were isolated from human peripheral blood mononuclear cells (PBMC) using 30 µl of supernatant from the hybridoma cell line M-DC8 (DSM ACC 2241) per buffy coat (5-8 x 10⁸ PBMC). For all experiments, samples obtained from the same healthy donor were used. Cells were collected by magnetic beads coated with rat-anti-mouse IgM (both Miltenyi I. Bergisch Gladbach, Germany) [Werden die "beads" mit diesen IgM beladen oder werden sie so gekauft? Wurde ein sog. "sandwich bead" hergestelit?]. Freshly isolated M-DC8-positive cells were cultured at 3x10⁵/ml in RPMI medium supplemented with 2 mM L-glutamine, 1% non essential amino acids; 1% pyruvate, 50 µg/ml kanamycin (all Gibco), 50 µM 2-mercaptoethanol (Merck), 10% fetal calf serum (FCS; HyClone Laboratories), 50 ng/ml GM-CSF (Leucomax; Novartis) and 1000 U/ml human recombinant IL-4 (Basel Institute for Immunology) for 7 days. Conventional DCs were isolated from human peripheral blood mononuclear cells essentially as described (Sallusto, J. Exp. Med. 179 (1994), 1109-1118) employing an anti-CD14 antibody conjugated to magnetic microbeads (Miltenyi I. Bergisch Gladbach, Germany). CD14⁺ cells (~99% pure) were cultured as described above for M-DC8-positive cells. DC maturation was induced by addition of 1 µg/ml lipopolysaccharide (LPS) from *Salmonella abortus equi* (Sigma) for 40 h.

DCs have two specialized mechanisms for antigen capture: the mannose receptor system and macropinocytosis (Sallusto et al., J. Exp. Med 182 (1995), 389-400). These systems allow DCs to take up and concentrate macromolecules in the MHC class II compartment. In contrast to macrophages, DCs show a very high, and constitutive level of macropinocytosis. While in all other cell types surface molecules are internalized via coated pits that act as selective molecular filters, in DCs, all surface molecules are primarily internalized via macropinocytosis, which is a non-selective process.

The endocytotic capacity of cultured M-DC8+ cells and conventional DCs was studied using two classical markers: Lucifer yellow CH (LY; pottasium salt; Molecular Probes Inc., Eugene, OR) and lysine-fixable FITC-dextran (FITC-DX; Molecular Probes Inc.). Both markers allow quantitation of uptake at the single cell level by FACS analysis. Cells were resuspended in 10% FCS medium buffered with 25 mM HEPES at 37°C. FITC-DX or LY were added at a final concentration of 1 mg/ml for 30 minutes. The cells were washed four times with cold PBS containing 1% FCS and 0.01% NaN₃, and were analyzed in a FACScan (Becton Dickinson) using propidium iodide to exclude dead cells. The background (cells pulsed at 0°C) was substracted. M-DC8+ cells showed double the activity of fluid phase uptake than conventional DCs (Figure 11, left panel) while the uptake of FITC-DX via the mannose receptor was at a comparable level (Fig. 11, right panel).

The expression of cell surface molecules characteristic for antigen-presenting cells on M-DC8-positive and conventional DCs were analysed by FACS. Cell surface staining was performed using mouse monoclonal antibodies followed by addition of FITC- or PE-conjugated affinity purified, isotype-specific goat-anti-mouse antibodies (Southern Biotechnology Associates, Birmingham, AL). The following monoclonal antibodies were used: W6/32 (IgG2a, anti-HLA class I; obtained from the Basel Inst. of Immunology); HB55 (IgG2a, anti-HLA class II; obtained from the Basel Inst. of Immunology); IT2.2 (IgG2b, anti-B7.2; obtained from Pharmingen) and supernatant from the hybridoma line producing M-DC8 IgM monoclonal antibody.

As shown in Fig. 12, freshly isolated M-DC8-positive cells showed a level of expression of HLA class I and class II and the co-stimulatory molecule B7.2 which was comparable to that of freshly isolated, conventional, CD14-positive DC precursor calls. The only difference was the absence of the M-DC8-antigen from conventional DC precursor cells. After 7 days of culture in the presence of GM-CSF and IL-4, M-DC8-positive cells showed significantly elevated surface expression of HLA class I and II molecules as well as 87.2 (Fig. 13, A, upper panel). Conventional DCs also showed increased expression of these four molecules but did not reach the level observed with M-DC8-positive cells (Fig.. 13, A, lower panel).

Upon stimulation with LPS for 40 h, a further increase in HLA class I and II expression was seen with both M-DC8-positive and conventional DCs (Fig. 13, B). Again, DC8-positive cells reached a higher level of HLA expression. Most dramatic was the enhancement of 87.2 surface expression in response to LPS.

Taken together, these data show that M-DC8-positive DCs have an endocytotic capacity via the mannose receptor comparable to that of conventional monocyte-derived DCs and a capacity for micropinocytosis superior to that of conventional DCs. Likewise, M-DC8-positive DCs have a level of surface expression of antigen-presenting and T cell co-stimulatory molecules exceeding that of conventional DCs under both unstimulated and LPS-induced conditions. The data show that the monoclonal antibody mDC8 is capable of isolating a cell population from human blood with a very high potential for uptake and presentation of antigens for induction of an immune response.

### Example 10: Establishment of a Jurkat T cell lymphoma subclone expressing the M-DC8 antigen

Jurkat is a cell line originally derived from a human T cell lymphoma. As shown in Figure 14, these cells do not normally express the antigen recognized by M-DC8 (upper panel, upper left quadrant). However very few cells (<1%) were seen to react with M-DC8 (see two M-DC8 positive cells in the upper left quadrant). These cells were isolated through multiple rounds of cell sorting with M-DC8 monoclonal antibody and subsequent propagation. Ultimately, a subclone of Jurkat T lymphoma cells could be established that stably expressed the M-DC8 antigen at high levels (Fig. 14, lower left panel). Likewise, a Jurkat subclone was established by removal of M-DC8-positive cells that essentially lacked cells expressing M-DC8 (Fig. 14, lower right panel). These two Jurkat subclones were used to further characterize the M-DC8 antigen as well as other monoclonal antibodies recognizing the M-DC8 antigen (see Example 12, supra).

### Example 11: Characteristics of the antigen recognized by monoclonal antibody M-DC8

The antigen recognized by the M-DC8 monoclonal antibody is either a polypeptide structure of a membrane protein or a carbohydrate moiety. Carbohydrate structures , can be linked either to the extracellular domains of membrane proteins, to glycolipids that are integral components of the lipid bilayer, or to both. In order to investigate this question, lipids were analyzed for binding of M-DC8 IgM. There lipids were extracted from cell membranes which were isolated from the above described Jurkat subclones which are either M-DC8-negative or M-DC8-positive. Jurkat cells (3-5 x 10⁹)were collected by centrifugation, washed three times in PBS and sonicated in 15 ml 1:2 chloroform/methanol for 5 min. After centrifugation at 2000xg for 10 min the supernatant was collected and the cellpellet was twice re-extracted with 15 ml chloroform/methanol. Pooled extracts were vacuum dried; was resuspended in 50 ml KCI 0.88%. The suspension was loaded on a 2.8x10 cm C18 column (Bakerbond). After washing with 50 ml KCl solution, and de-sulfing with 200 ml distilled water, the lipids were eluted with 250 ml methanol. The vacuum-dried eluate was dissolved in 5 ml chloroform/methanol. After addition of 1 ml water and vortexing, the lower phase was re-extracted twice with 1 ml water and the combined upper phases (containing membrane lipids) were reduced under vacuum and lyophilized. An 1% aliquot of lipid extracts was loaded on a 2.5x10 cm HPTLC plate (Merck Si 60) and developed for 35 min using as solvent chloroform/methanol/water at 120:70:17 (vole) with 0.02% calcium chloride. Analogous to Western blots, chromatograms were reacted with M-DC8 antibody as described (Bethke et al., J. Immunol. Methods 89: 111-116, 1986). Bound M-DC8 IgM was detected by alkaline phosphatase (AP)-conjugated goat-anti-mouse IgM (µ-chain) (Caltag). M-DC8 reacted with two distinct lipid bands that were only obtained with the M-DC8-positive Jurkat subclone (Fig. 15, first lane). These two bands were absent from lipids extracted from M-DC8-negative Jurkat T lymphoma cells. In these cells only a very minor, distinctly migrating band was detected (Fig. 15, second lane). Since the fatty acid moiety of lipids are usually not immunogenic, the immunoreactivity of M-DC8 with these two lipid species was most likely due to their carbohydrate moiety.

In order to investigate whether the carbohydrate moiety recognized by M-DC8 IgM was also present on polypeptides, integral membrane proteins were prepared from M-DC8-positive and -negative Jurkat cells as described (Pasqual et al., Electrophoresis 18: 2573-2581, 1997). The prepared membrane proteins were sujected to SDS-PAGE followed by Western blotting using mAb M-DC8. Detection of the bound lgM was carried out by AP-conjugated goat-anti-mouse anti-µ antibody (Biosource). M-DC8 recognized a number of polypeptide bands in a Western blot (Figure 16, lane 1). This shows that the M-DC8 antigen can also be present on various integral membrane proteins of M-DC8 positive Jurkat cells. Such signals were not obtained with M-DC8-negative Jurkat T cells (Fig. 16, lane 2). One of the bands that were immunoreactive with M-DC8 was identified by Western blotting as P-selectin glycoprotein ligand-1 (PSGL-1) using anti-PSGL-1 antibodies (Pharmingen). PSGL-1 is a polypeptide found on many lymphocytic cell types (Yang et al., Thromb. Haemost 81: 1-7, 1999) and was present in similar amounts on M-DC8-positive and -negative Jurkat cells (Figure 16, lanes 3 and 4). A signal identical to that of M-DC8-positive PSGL-1 was obtained when membrane proteins from dendritic cells that were isolated by M-DC8 from human PBMCs were analyzed by Western blotting (Figure 16, lane 5). The fuzzy appearance of the PSGL-1 band upon SDS-PAGE is due to its known high carbohydrate content.

Taken together these data indicate that the M-DC8 antigen was a carbohydrate structure that was present on glycolipids as well as on integral membrane proteins of the M-DC8-positive Jurkat subclone and of human M-DC8-posibve dendritic cells. One major carrier protein for the M-DC8 carbohydrate antigen on dendritic cells and M-DC8-psoitive Jurkat cells was identified as PSGL-1.

### Example 12: Generation of new dendritic cell-specific monoclonal antibodies

New monoclonal antibodies, referred to as D-DC8.1 and D-DC8.2, were generated by immunization of Balb/c x C57BI F1 mice with either membrane preparations from the M-DC8-positive Jurkat T lymphoma subclone (for D-DC8.1) or using the complete M-DC8-positive Jurkat cells (D-DC8.2). Plasma membranes were prepared by hypotonic lysis of cells, followed by mechanical disruption of cells, sequential centrifugation and washing of membranes and removal of peripheral membrane proteins by treatment with sodium carbonate at pH 11 using a standard procedures (Pasqual et al., Electrophoresis 18: 2573-2581, 1997). Mice were injected with the equivalent of 150 µg of membrane protein (for D-DC8.1) or 1-2x10⁷ cells (for D-DC8.2) in five i.p. injections at 3-4 week intervals. Three days following the last injection, spleen cells were prepared and used for fusion with X63Ag8.653 myeloma cells, as described for M-DC8. Both antibodies obtained were of the IgM class.

In order to investigate whether the two monoclonal antibodies D-DC8.1 and D.DC8.2 recognize the same population of dendritic cells as M-DC8, human PBMCs were analyzed by FACS after double immunofluorescence staining of human PBMCs. The same healthy donor was used for all the experiments shown. For Figure 17, human PBMCs were double-stained with the monoclonal antibodies M-DC8 (upper row), D-DC8.1 (middle row) or D-DC8.2 (lower row) together with antibodies recognizing the cell surface markers CD14 (left panels), HLA-DR (middle panels) or CD16 (right panels) (all obtained from Pharmingen). IgM monoclonal antibodies were detected by a goat-anti-mouse phycoerythrin (PE)-labeled anti-µ secondary antibody (Immunotech). As is seen in the upper right quadrants of the FACS scans in Figure 17, both D-DC8.1 and D-DC8.2 recognized an amount of PBMCs very similar to that recognized by the monoclonal antibody M-DC8. These cells had in common that they were high in the expression of HLA-DR (Fig. 17, middle panels) and CD16 (right panels) but low in the expression of CD14 (left panels). This shows that both D-DC8.1 and D-DC8.2 recognized a discrete subset of PBMCs with characteristics very similar to that recognized by M-DC8 with respect to frequency and cell surface expression of HLA-DR, CD16 and CD14. All three antibodies appear to recognize a class of DCs referred to here as M-DC8 dendritic cells.

To substantiate that the monoclonal antibodies D-DC8.1 and D-DC8.2 both recognized the M-DC8 antigen, they were tested for binding to M-DC8-negative and M-DC8-positive Jurkat subclones. Both monoclonal antibodies did not significantly bind M-DC8-negative Jurkat cells (Fig. 18, left panels, upper left quadrants) but both strongly reacted with M-DC8-positive Jurkat cells (right panels).

In summary, these results show that the M-DC8 antigen, which is characteristic for a new class of dendritic cells, can be recognized by at least two other monoclonal antibodies, D-DC8.1 and D-DC8.2.

### References

1. Steinmann, R.M. The dendritic cell system and its role in immunogenicity. *Annu. Rev. Immunol.* **9,** 271-296 (1991).
2. O'Doherty, U. *et al.* Human blood contains two subsets of dendritic cells, one immunologically mature and the other immature. *Immunology* **82,** 487-493 (1994).
3. Thomas, R., Davis L. & Lipsky P.E. Isolation and characterization of human peripheral blood dendritic cells. *J. Immunol.* **150,** 821-834 (1993).
4. Sallusto, F., Cella, M., Danieli, C. & Lanzavecchia, A. Dendritic cells use macropinocytosis and the mannose receptor to concentrate macromolecules in the major histocompatibility complex class II compartment: downregulation by cytokines and bacterial products. *J. Exp. Med.* **182**, 389-400 (1995).
5. Germain, R. & Margulies, D.H. The biochemistry and cell biology of antigen processing and presentation. *Annu. Rev. Immunol.* **11,** 403-450 (1993).
6. Macatonia, S.E. *et al.* Dendritic cells produce IL-12 and direct the development of Th1 cells from naive CD4+ T cells. J. *Immunol.* **154**, 5071-5079 (1995).
7. Kalinski, P., Hilkens, C.M.U., Snijders, A., Snijdewint, F.G.M. & Kapsenberg, M.L. IL-12-deficient dendritic cells, generated in the presence of prostaglandin E₂, promote type 2 cytokine production in maturing human naive T helper cells. *J. Immunol.* **159,** 28-35 (1997).
8. Young, J.W. & Inaba, K. Dendritic cells as adjuvants for class I major histocompatibility complex-restricted antitumor immunity. *J. Exp. Med.* **183**, 7-11 (1996).
9. Zhou, L.J. & Tedder T.F. Human blood dendritic cells selectively express CD83, a member of the imunoglobulin superfamily. *J. Immunol.* **154,** 3821-3835 (1995).
10. Mosialos, G. *et al.* Circulating human dendritic cells differentially express high levels of a 55-kd actin-bundling protein. *Am. J. Pathol.* **148**, 593-600 (1996).
11. O'Doherty, U. *et al.* Dendritic cells freshly isolated from human blood express CD4 and mature into typical immunostimulatory dendritic cells after culture in monocyte-conditioned medium. J. *Exp. Med.* **178,** 1067-1078 (1993).
12. Caux, C., Dezutter-Dambuyant, C., Schmitt, D. & Banchereau J. GM-CSF and TNF cooperate in the generation of dendritic Langerhans cells. *Nature* **360,** 258-261 (1992).
13. Sallusto, F. & Lanzavecchia, A. Efficient presentation of soluble antigen by cultured human dendritic cells is maintained by granulocyte/macrophage colony-stimulating factor plus interleukin 4 and downregulated by tumor necrosis factor. *J. Exp. Med.* **179**, 1109-1118 (1994).
14. Romani, N. *et al.* Proliferating dendritic cell progenitors in human blood. *J. Exp. Med.* **180**, 83-93 (1994).
15. Fithian, E. Reactivity of Langerhans cells with hybridoma antibody. *Proc. Natl. Acad. Sci.* USA **78,** 2541-2544 (1988).
16. Salomon, D.R. *et al.* Vascular cell adhesion molecule-1 is expressed by cortical thymic epithelial cells and mediates thymocyte adhesion. Implications for the function of alpha 4 beta 1 (VLA4) integrin in T-cell development. Blood **89**, 2461-2471 (1997).
17. Inaba, K., Young, J.W. & Steinman R.M. Direct activation of CD8+ cytotoxic T lymphocytes by dendritic cells. *J. Exp. Med.* **166,** 182-194 (1987).
18. Young, J.W. & Steinmann R.M. Dendritic cells stimulate primary human cytolytic lymphocyte responses in the absence of CD4+ Helper T cells. *J*. *Exp. Med.* **171**, 1315-1332 (1990).
19. Wölfel, T. *et al.* Isolation of naturally processed peptides recognized by cytolytic T lymphocytes (CTL) on human melanoma cells in association with HLA-A2.1. *Int. J. Cancer* **57**, 413-418 (1994).
20. Fanger, N.A., Wardwell, K., Shen, L., Tedder, T.F. & Guyre, M.P. Type I (CD64) and Type II (CD32) Fc? receptor-mediated phagocytosis by human blood dendritic cells. *J. Immunol.* **157,** 541-548 (1997).
21. Mehta-Damani, A., Markowicz, S. & Engleman, E.G. Generation of antigen-specific CD4+ T cell lines from naive precursors. *Eur. J. Immunol.* **25,** 1206-1211, (1995).
22. Croft, M., Duncan, D.D. & Swain, S.L. Response of naive antigen-specific CD4+ T cells in vitro: Characteristics and antigen-presenting cell requirements. *J. Exp. Med.* **176,** 1431-1437 (1992).
23. Esposito-Farese, M.E. et al. Membrane and soluble Fc gamma RII/III modulate the antigen-presenting capacity of murine dendritic epidermal Langerhans cells for IgG-complexed antigens. *J. Immunol.* **155,** 1725-1736, (1995).
24. Schmidt, R.M. & Perussia, B. Cluster report: CD16. *Leukocyte typing IV.* Knapp, W. et al. eds. Oxford University Press, Oxford, 574-578.(1989).
25. Alexander, E.L., Titus J.A. & Segal, D.M. Quantitation of Fc receptors and surface immunoglobulin is affected by cell Isolation procedures using plasmagel and ficoll-hypaque. *J. Immunol. Methods* **22,** 263-272 (1978).
26. Knapp, W. *et al.* eds. *Leukocyte typing IV.* Oxford University Press, Oxford, (1989).
27. Wilhelm, W. *et al.* Direct monoclonal antibody rosetting: An effective method for weak antigen detection and large scale separation of human mononuclear cells. *J. Immunol. Methods* **90,** 89-96 (1988).
28. Haynes, B.F. Chapter 1. *Leukocyte typing II.* Reinherz, E.L., Haynes, B.F., Nadler, L.M. & Bernstein, I.D. eds. Springer Verlag, New York, 3-30 (1986)

## Claims

1. An antibody which
(i) reacts with an epitope on dendritic cells, DCs, displaying features of immature and/or mature DCs from peripheral blood mononuclear cells, PBMCs, and wherein said DCs are CD64⁻, CD33⁺, CD45RA⁺, CD11c⁺ and p55⁻ and mostly CD16⁺, but
(ii) does not react with other PBMCs,
wherein said antibody is produced by hybridoma cell line DSM ACC 2241, by hybridoma cell line DSM ACC 2399 or by hybridoma cell line DSM ACC 2398.

2. The antibody of claim 1, wherein said DCs represent a DC population of a maturational stage between immature and mature DCs.

3. The antibody of claim 1 or 2, wherein said DCs are HLA-DR⁺.

4. The antibody of any one of claims 1 to 3, wherein said DCs are of restricted size and granularity in light scatter display located between lymphocytes and monocytes.

5. A continuous, stable antibody-producing cell line which is capable of producing an antibody of any one of claims 1 to 4, wherein said cell line is the hybridoma cell line having the deposit number DSM ACC 2241, DSM ACC 2398 or DSM ACC 2399.

6. A polynucleotide encoding at least a variable region of an immunoglobulin chain of the antibody produced by hybridoma cell line DSM ACC 2241, by hybridoma cell line DSM ACC 2399 or by hybridoma cell line DSM ACC 2398.

7. A vector comprising the polynucleotide of claim 6, optionally in combination with a polynucleotide that encodes the variable region of the other immunoglobulin chain of said antibody.

8. A host cell comprising a polynucleotide of claim 6 or a vector of claim 7.

9. A method for preparing an antibody or a functional fragment thereof, wherein said antibody or said functional fragment thereof is capable of recognizing dendritic cells, DCs from peripheral blood mononuclear cells, PBMCs, comprising
(a) culturing the cell of claim 5 or 8; and
(b) isolating said antibody, functional fragment or immunoglobulin chain(s) thereof from the culture.

10. An antibody or immunoglobulin chain encoded by a polynucleotide of claim 6 or obtainable by the method of claim 9, wherein said antibody or immunoglobulin chain is capable of recognizing dendritic cell, DCs, from peripheral blood mononuclear cells, PBMCs.

11. The antibody of claim 10, wherein said antibody is a monoclonal antibody, chimeric antibody, humanized antibody, bispecific antibody, synthetic antibody, antibody fragment, or a chemically modified derivative of any of these.

12. The bispecific antibody of claim 11 which recognizes an epitope specific for a tumor cell, a virus-infected cell, a T cell, a tumor-associated protein or a microbial protein.

13. A polypeptide being capable of recognizing dendritic cells, DCs, from peripheral blood mononuclear cells, PBMCs, comprising
(a) at least a variable region of the antibody produced by the hybridoma cell lines DSM ACC2241, DSM ACC 2399 or DSM ACC 2398; and
(b) at least one further domain.

14. The polypeptide of claim 13, wherein said domains are linked by covalent or non-covalent bonds.

15. The polypeptide of claim 13 or 14, wherein said at least one further domain comprises an effector molecule having a conformation suitable for biological activity, capable of sequestering an ion or selective binding to a solid support or to a preselected determinant.

16. The polypeptide of claim 15, wherein said effector molecule is an enzyme, toxin, antigen, receptor, binding site, biosynthetic antibody binding site, growth factor, cell-differentiation factor, lymphokine, cytokine, hormone, a remotely detectable moiety, or anti-metabolite.

17. The polypeptide of claim 15, wherein said molecule capable of sequestering an ion is calmodulin, methallothionein, a fragment thereof, or an amino acid sequence rich in at least one of glutamic acid, aspartic acid, lysine, and arginine.

18. The polypeptide of claim 15, wherein said molecule capable of selective binding to a solid support is a positively or negatively charged amino acid sequence, a cysteine-containing amino acid sequence, streptavidin, a fragment of Staphylococcus protein A, GST, a His-tag or LexA.

19. The polypeptide of claim 16, wherein said receptor is a co-stimulatory surface molecule important for T-cell activation or comprises an epitope binding site or a hormone binding site.

20. The polypeptide of claim 19, wherein said co-stimulatory surface molecule is CD80 (B7-1) or CD86 (B7-2).

21. A method for isolating or identifying DCs as defined in any one of claims 1 to 3 from peripheral blood, comprising the steps of
(a) contacting a sample of peripheral blood with the antibody of any one of claims 1 to 3; and
(b) detecting the presence of antibody/DC complexes; and/or
(c) recovering dendritic cells which have bound to said antibody.

22. A kit comprising the antibody of any one of claims 1 to 4, 10 and 11, the polypeptide of claims 13 to 20, the polynucleotide of claim 6 or the vector of claim 7.

23. A composition comprising the antibody of any one of claims 1 to 4, 10 and 11, the polypeptide of claims 13 to 20, the polynucleotide of claim 6 or the vector of claim 7.

24. The composition of claim 23 which is a pharmaceutical composition optionally further comprising a pharmaceutical acceptable carrier.

25. A non-human transgenic animal comprising the polynucleotide of claim 6 or the vector of claim 7.

26. Use of the bispecific antibody of claim 11 or 12 for the preparation of a pharmaceutical composition for recruiting target cells with said dendritic cells.

27. The use of claim 26, wherein the target cells are tumor cells or virus-infected cells or cells infected with a microorganism.

## Patentansprüche

1. Antikörper, welcher
(i) mit einem Epitop auf dendritischen Zellen, DCs, reagiert, die Merkmale von unreifen und/oder reifen DCs von mononucleären Zellen des peripheren Bluts, PBMCs, aufweisen, und wobei die DCs CD64⁻, CD33⁺, CD45RA⁺, CD11c⁺ und p55⁻ und größtenteils CD16⁺ sind, jedoch
(ii) nicht mit anderen PBMCs reagiert,
wobei der Antikörper von der Hybridomzelllinie DSM ACC 2241, von der Hybridomzelllinie DSM ACC 2399 oder von der Hybridomzelllinie DSM ACC 2398 produziert wird.

2. Antikörper nach Anspruch 1, wobei die DCs eine DC-Population eines Reifestadiums zwischen unreifen und reifen DCs ausmachen.

3. Antikörper nach Anspruch 1 oder 2, wobei die DCs HLA-DR⁺ sind.

4. Antikörper nach einem der Ansprüche 1 bis 3, wobei die DCs von einer begrenzten Größe und Granularität in der Lichtstreuungsanzeige sind, wobei sie zwischen Lymphocyten und Monocyten liegen.

5. Kontinuierliche, stabile Antikörper-produzierende Zelllinie, welche in der Lage ist, einen Antikörper nach einem der Ansprüche 1 bis 4 zu produzieren, wobei die Zelllinie die Hybridomzelllinie mit der Hinterlegungsnummer DSM ACC 2241, DSM ACC 2398 oder DSM ACC 2399 ist.

6. Polynucleotid, das zumindest eine variable Region einer Immunglobulinkette des Antikörpers codiert, welcher von Hybridomzelllinie DSM ACC 2241, von Hybridomzelllinie DSM ACC 2399 oder von Hybridomzelllinie DSM ACC 2398 produziert wird.

7. Vektor, umfassend das Polynucleotid nach Anspruch 6, gegebenenfalls in Kombination mit einem Polynucleotid, das die variable Region der anderen Immunglobulinkette des Antikörpers codiert.

8. Wirtszelle, umfassend ein Polynucleotid nach Anspruch 6 oder einen Vektor nach Anspruch 7.

9. Verfahren zur Herstellung eines Antikörpers oder eines funktionellen Fragments davon, wobei der Antikörper oder das funktionelle Fragment davon in der Lage ist, dendritische Zellen, DCs, von mononucleären Zellen des peripheren Bluts, PBMCs, zu erkennen, umfassend
(a) das Züchten der Zelle nach Anspruch 5 oder 8; und
(b) das Isolieren des Antikörpers, des funktionellen Fragments oder von Immunglobulinkette(n) davon aus der Kultur.

10. Antikörper oder Immunglobulinkette, codiert von einem Polynucleotid nach Anspruch 6 oder erhältlich durch das Verfahren nach Anspruch 9, wobei der Antikörper oder die Immunglobulinkette in der Lage ist, dendritische Zellen, DCs, von mononucleären Zellen des peripheren Bluts, PBMCs, zu erkennen.

11. Antikörper nach Anspruch 10, wobei der Antikörper ein monoclonaler Antikörper, chimärer Antikörper, humanisierter Antikörper, bi-spezifischer Antikörper, synthetischer Antikörper, ein Antikörperfragment, oder ein chemisch modifiziertes Derivat davon ist.

12. Bi-spezifischer Antikörper nach Anspruch 11, welcher ein Epitop erkennt, das spezifisch für eine Tumorzelle, eine virusinfizierte Zelle, eine T-Zelle, ein tumorassoziiertes Protein oder ein mikrobielles Protein ist.

13. Polypeptid, das in der Lage ist, dendritische Zellen, DCs, von mononucleären Zellen des peripheren Bluts, PBMCs, zu erkennen, umfassend
(a) zumindest eine variable Region des Antikörpers, produziert von den Hybridomzelllinien DSM ACC 2241, DSM ACC 2399 oder DSM ACC 2398; und
(b) zumindest eine weitere Domäne.

14. Polypeptid nach Anspruch 13, wobei die Domänen durch kovalente oder nichtkovalente Bindungen verbunden sind.

15. Polypeptid nach Anspruch 13 oder 14, wobei die zumindest eine weitere Domäne ein Effektormolekül umfaßt mit einer Konformation, die für biologische Aktivität geeignet ist, welches in der Lage ist, ein Ion zu sequestrieren oder an einen festen Träger oder an eine vorgewählte Determinante zu binden.

16. Polypeptid nach Anspruch 15, wobei das Effektormolekül ein Enzym, Toxin, Antigen, Rezeptor, eine Bindestelle, biosynthetische Antikörper-Bindestelle, ein Wachstumsfaktor, Zelldifferenzierungsfaktor, Lymphokin, Cytokin, Hormon, ein entfernt nachweisbarer Rest, oder Anti-Metabolit ist.

17. Polypeptid nach Anspruch 15, wobei das Molekül, das in der Lage ist, ein Ion zu sequestrieren, Calmodulin, Methallothionein, ein Fragment davon oder eine Aminosäuresequenz ist, die reich ist an zumindest einer der Aminosäuren Glutaminsäure, Asparginsäure, Lysin und Arginin.

18. Polypeptid nach Anspruch 15, wobei das Molekül, das in der Lage ist, selektiv an einen festen Träger zu binden, eine positiv oder negativ geladene Aminosäuresequenz, eine Cystein enthaltende Aminosäuresequenz, Streptavidin, ein Fragment von Staphylococcus-Protein A, GST, eine His-Markierung oder LexA ist.

19. Polypeptid nach Anspruch 16, wobei der Rezeptor ein co-stimulatorisches Oberkllichemnolekül ist das wichtig für T-Zellaktivierung ist, oder das eine Epitop-Bindestelle oder eine Hormon-Bindestelle umfaßt.

20. Polypeptid nach Anspruch 19, wobei das co-stimulatorische Oberflächenmolekül CD80 (B7-1) oder CD86 (B7-2) ist.

21. Verfahren zur Isolierung oder Identifizierung von DCs wie definiert in einem der Ansprüche 1 bis 3 aus dem peripheren Blut, umfassend die Schritte des
(a) Kontaktierens einer Probe des peripheren Bluts mit dem Antikörper nach einem der Ansprüche 1 bis 3; und
(b) Nachweisens der Gegenwart der Antikörper/DC-Komplexe; und/oder
(c) Gewinnens der dendritischen Zellen, welche an den Antikörper gebunden haben.

22. Kit, umfassend den Antikörper nach einem der Ansprüche 1 bis 4, 10 und 11, das Polypeptid nach Ansprüchen 13 bis 20, das Polynucleotid nach Anspruch 6 oder den Vektor nach Anspruch 7.

23. Zusammensetzung umfassend den Antikörper nach einem der Ansprüche 1 bis 4, 10 und 11, das Polypeptid nach Ansprüchen 13 bis 20, das Polynucleotid nach Anspruch 6 oder den Vektor nach Anspruch 7.

24. Zusammensetzung nach Anspruch 23, welche ein Arzneimittel ist, das gegebenenfalls ferner einen pharmazeutisch verträglichen Träger umfasst.

25. Nicht-menschliches transgenes Tier, umfassend das Polynucleotid nach Anspruch 6 oder den Vektor nach Anspruch 7.

26. Verwendung des bispezifischen Antikörpers nach Anspruch 11 oder 12 für die Herstellung eines Arzneimittels zur Rekrutierung von Zielzellen mit den dendritischen Zellen.

27. Verwendung nach Anspruch 26, wobei die Zielzellen Tumorzellen oder virusinfizierte Zellen oder Zellen infiziert mit einem Mikroorganismus sind.

## Revendications

1. Un anticorps qui
(i) réagit avec un épitope sur des cellules dendritiques, (DCs), présentant des caractéristiques de DC immatures et matures de cellules mono-nucléées du sang périphérique, PBMCs, lesdites cellules dendritiques étant CD64⁻, CD33⁺, CD45RA⁺, CD11c⁺, et p55⁻ et en grande partie CD16⁺, mais
(ii) ne réagit pas avec les autres PBMCs,
dans lequel ledit anticorps est produit par une lignée cellulaire d'hybridome DSM ACC 2241, par une lignée cellulaire d'hybridome DSM ACC 2399 ou par une lignée cellulaire d'hybridome DSM ACC 2398.

2. Anticorps selon la revendication 1, dans lequel lesdites DCs représentent une population de DCs d'un état de maturation entre DCs immatures et matures.

3. Anticorps selon la revendication 1 ou 2, dans lequel lesdites DCs sont HLA-DR⁺.

4. Anticorps selon l'une quelconque des revendications 1 à 3, dans lequel lesdites DCs sont de taille et de granulosité restreintes en affichage de diffusion de la lumière localisées entre les lymphocytes et les monocytes.

5. Lignée cellulaire continue, produisant de façon stable des anticorps qui est capable de produire un anticorps de l'une quelconque des revendications 1 à 4, laquelle dite lignée cellulaire est une lignée cellulaire, d'hybridome ayant le numéro de dépôt DSM ACC 2241, DSM ACC 2398 ou DSM ACC 2399.

6. Polynucléotide codant au moins une région variable d'une chaîne d'immunoglobuline de l'anticorps produit par la lignée cellulaire d'hybridome DSM ACC 2241, par la lignée cellulaire d'hybridome DSM ACC 2399 ou par la lignée cellulaire d'hybridome DSM ACC 2398.

7. Vecteur comprenant le polynucléotide selon la revendication 6, en combinaison de manière optionnelle avec un polynucléotide codant la région variable de l'autre chaîne d'immunoglobuline dudit anticorps.

8. Cellule hôte comprenant un polynucléotide selon la revendication 6 ou un vecteur selon la revendication 7.

9. Procédé de préparation d'un anticorps ou d'un fragment fonctionnel de celui-ci, dans lequel ledit anticorps ou ledit fragment fonctionnel est capable de reconnaître des cellules dendritiques, DCs de cellules mononucléées du sang périphérique, PBMCs, comprenant
(a) culture de la cellule selon la revendication 5 ou 8 ; et
(b) isolation de la culture dudit anticorps, fragment fonctionnel ou chaîne(s) d'immunoglobuline de celui-ci.

10. Anticorps ou chaîne d'immunoglobuline codé par un polynucléotide selon la revendication 6 ou pouvant être obtenu selon le procédé selon la revendication 9, dans lequel ledit anticorps ou chaîne d'immunoglobuline est capable de reconnaître des cellules dendritiques, DCs, de cellules mononucléées du sang périphérique, PBMCs.

11. Anticorps selon la revendication 10, dans lequel ledit anticorps est un anticorps monoclonal, un anticorps chimère, un anticorps humanisé, un anticorps bi-spécifique, un anticorps synthétique, un fragment d'anticorps, ou un dérivé chimiquement modifié de l'un quelconque de ceux-ci.

12. Anticorps bispécifique selon la revendication 11 qui reconnaît un épitope spécifique d'une cellule tumorale, d'une cellule infectée par un virus, d'une cellule T, d'une protéine associée à une tumeur, ou d'une protéine microbienne.

13. Polypeptide étant capable de reconnaître des cellules dendritiques, DCs, de cellules mononucléées du sang périphérique, PBMCs, comprenant
(a) au moins une région variable de l'anticorps produit par les lignées cellulaires d'hybridome DSM ACC 2241, DSM ACC 2399 ou DSM ACC 2398 ; et
(b) au moins un domaine supplémentaire.

14. Polypeptide selon la revendication 13, dans lequel lesdits domaines sont liés par des liaisons covalentes ou non covalentes.

15. Polypeptide selon la revendication 13 ou 14, dans lequel ledit au moins un domaine supplémentaire comprend une molécule effectrice ayant une conformation appropriée pour une activité biologique, capable de séquestrer un ion ou de liaison sélective à un support solide ou à un déterminant présélectionné.

16. Polypeptide selon la revendication 15, dans lequel ladite molécule effectrice est une enzyme, toxine, antigène, récepteur, site de liaison, site de liaison d'anticorps biosynthétique, facteur de croissance, facteur de différentiation cellulaire, lymphokine, cytokine, hormone, un groupe détectable de façon discernable, ou un anti-métabolite.

17. Polypeptide selon la revendication 15, dans lequel ladite molécule capable de séquestrer un ion est une calmoduline, méthallothionéine, un fragment de celle-ci, ou une séquence d'acides aminés riche en au moins un acide glutamique, acide asparrique, lysine et arginine.

18. Polypeptide selon la revendication 15, dans lequel ladite molécule capable de liaison sélective à un support solide est une séquence d'acides aminés chargée positivement ou négativement, séquence d'acides aminés contenant une cystéine, streptavidine, un fragment de la protéine A de *Staphylococcus,* GST, His-tag ou LexA.

19. Polypeptide selon la revendication 16, dans lequel ledit récepteur est une molécule de surface co-stimulatrice importante pour la stimulation des cellules T ou comprend un site de liaison à un épitope ou un site de liaison à une hormone.

20. Polypeptide selon la revendication 19, dans lequel ladite molécule de surface co-stimulatrice est CD80 (B7-1) ou CD86 (B7-2).

21. Procédé d'isolation ou d'identification de DCs comme définies selon l'une quelconque des revendications 1 à 3 du sang périphérique comprenant les étapes de
(a) mise en contact d'un échantillon de sang périphérique avec l'anticorps selon l'une quelconque des revendications 1 à 3 ; et
(b) détection de la présence de complexes anticorps/DCs ; et/ou
(c) récupération de cellules dendritiques qui ont lié ledit anticorps.

22. Kit comprenant l'anticorps selon l'une quelconque des revendications 1 à 4, 10 et 11, le polypeptide selon les revendications 13 à 20, le polynucléotide selon la revendication 6, ou le vecteur selon la revendication 7.

23. Composition comprenant l'anticorps selon l'une quelconque des revendications 1 à 4, 10 et 11, le polypeptide selon les revendications 13 à 20, le polynucléotide selon la revendication 6, ou le vecteur selon la revendication 7.

24. Composition selon la revendication 23 qui est une composition pharmaceutique comprenant en outre de manière optionnelle, un véhicule acceptable sur le plan pharmaceutique.

25. Animal transgénique non-humain comprenant le polynucléotide selon la revendication 6 ou le vecteur selon la revendication 7.

26. Utilisation de l'anticorps bi-spécifique selon la revendication 11 ou 12 pour la préparation d'une composition pharmaceutique pour le recrutement de cellules cibles avec lesdites cellules dendritiques.

27. Utilisation selon la revendication 26, dans laquelle les cellules cibles sont des cellules tumorales ou des cellules infectées par un virus ou des cellules infectées par un microorganisme.
